# FASCICULE DE BREVET EUROPEEN

(11) **EP 1 519 908 B1**
(45) Date de publication et mention de la délivrance du brevet: **13.06.2007**
(21) Numéro de dépôt: 03762750.2
(22) Date de dépôt: 08.07.2003
(51) Int. Cl.: C07C 51/00, C07C 67/00, C07C 59/90, C07C 59/88, C07C 59/84, C07C 69/712, C07C 69/67, C07C 251/48, C07C 323/63, C07C 281/00, C07C 323/09, C07D 311/30, A61K 31/192, A61K 31/216, A61P 25/16, A61P 25/28, A61P 35/00, A61P 37/08, A61P 9/10, A61P 3/04, A61P 3/08

(54) **COMPOSITION A BASE DE DERIVES DE 1,3-DIPHENYLPROP-2-EN-1-ONE SUBSTITUES, PREPARATIONS ET UTILISATIONS**
ZUSAMMENSETZUNG AUF GRUNDLAGE SUBSTITUIERTER 1,3-DIPHENYLPROP-2-EN-1-ON-DERIVATEN, DEREN HERSTELLUNG UND VERWENDUNGEN
COMPOSITION BASED ON SUBSTITUTED 1,3-DIPHENYLPROP-2-EN-1-ONE DERIVATIVES, PREPARATION AND USES THEREOF

(30) Priorité: 08.07.2002 FR 0208570
(43) Date de publication de la demande: 06.04.2005
(73) Titulaire: Genfit, 59120 Loos (FR)
(72) Inventeur: NAJIB, Jamila, M-40000 MARRAKECH (MA); CAUMONT-BERTRAND, Karine, F-59236 Frelinghien (FR)
(74) Mandataire: Tezier Herman, Béatrice
(86) Numéro de dépôt international: PCT/FR2003/002128
(87) Numéro de publication internationale: WO 2004/005243

(56) Documents cités:
- EP-A- 0 947 511
- WO-A-00/23073
- WO-A-01/98291
- DE-A- 4 327 365
- FR-A- 2 383 157
- FR-A- 2 841 900
- US-A- 3 558 612
- US-A- 3 994 955
- US-A- 4 656 305
- US-A- 5 276 058
- US-A- 5 523 302
- SHIBATA S: "ANTI-TUMORIGENIC CHALCONES" STEM CELLS, ALPHAMED PRESS, DAYTON, OH, US, vol. 12, 1994, pages 44-52, XP002949260 ISSN: 1066-5099
- DIMMOCK J R ET AL: "BIOACTIVITIES OF CHALCONES" CURRENT MEDICINAL CHEMISTRY, BENTHAM SCIENCE PUBLISHERS BV, BE, vol. 6, no. 12, 1999, pages 1125-1149, XP000917367 ISSN: 0929-8673
- LEBEAU J ET AL: "ANTIOXIDANT PROPERTIES OF DI-TERT-BUTYLHYDROXYLATED FLAVONOIDS" FREE RADICAL BIOLOGY AND MEDICINE, ELSEVIER SCIENCE, XX, vol. 29, no. 9, 1 novembre 2000 (2000-11-01), pages 900-912, XP001149406 ISSN: 0891-5849
- MUKHERJEE S ET AL: "SYNTHETIC AND BIOLOGICAL ACTIVITY EVALUATION STUDIES ON NOVEL 1,3-DIARYLPROPENONES" BIOORGANIC & MEDICINAL CHEMISTRY, ELSEVIER SCIENCE LTD, GB, vol. 9, no. 2, 2001, pages 337-345, XP001149402 ISSN: 0968-0896
- RAJAKUMAR D V ET AL: "ANTIOXIDANT PROPERTIES OF PHENYL STYRYL KETONES" FREE RADICAL RESEARCH,, YVERDON, CH, vol. 22, no. 4, 1995, pages 309-317, XP008014946 ISSN: 1071-5762
- ARTY I S ET AL: "Synthesis of benzylideneacetophenones and their inhibition of lipid peroxidation" EUROPEAN JOURNAL OF MEDICINAL CHEMISTRY 2000 FRANCE, vol. 35, no. 4, 2000, pages 449-457, XP004330428 ISSN: 0223-5234
- HALLIWELL B: "Oxidants and the central nervous system: some fundamental questions. Is oxidant damage relevant to Parkinson's disease, Alzheimer's disease, traumatic injury or stroke?" ACTA NEUROLOGICA SCANDINAVICA. SUPPLEMENTUM. DENMARK 1989, vol. 126, 1989, pages 23-33, XP008015255 ISSN: 0065-1427
- SOGAWA S ET AL: "3,4-DIHYDROXYCHALCONES AS POTENT 5-LIPOXYGENASE AND CYCLOOXYGENASE INHIBITORS" JOURNAL OF MEDICINAL CHEMISTRY, AMERICAN CHEMICAL SOCIETY. WASHINGTON, US, vol. 36, no. 24, 1993, pages 3904-3909, XP002951489 ISSN: 0022-2623
- NAKAMURA C ET AL: "SYNTHESIS AND BIOLOGICAL ACTIVITIES OF FLUORINATED CHALCONE DERIVATIVES" BIOORGANIC & MEDICINAL CHEMISTRY, ELSEVIER SCIENCE LTD, GB, vol. 10, no. 3, mars 2002 (2002-03), pages 699-706, XP001149403 ISSN: 0968-0896
- CALLISTE C-A ET AL: "CHALCONES: STRUCTURAL REQUIREMENTS FOR ANTIOXIDANT, ESTROGENIC AND ANTIPROLIFERATIVE ACTIVITIES" ANTICANCER RESEARCH, HELENIC ANTICANCER INSTITUTE, ATHENS,, GR, vol. 21, no. 6A, 2001, pages 3949-3956, XP008014947 ISSN: 0250-7005
- FURMAN C ET AL: "DI-TERT-BUTYLHYDROXYLATED FLAVONOIDS PROTECT ENDOTHELIAL CELLS AGAINST OXIDIZED LDL-INDUCED CYTOTOXICTY" JOURNAL OF BIOCHEMICAL AND MOLECULAR TOXICOLOGY, WILEY, NEW YORK, NY, US, vol. 15, no. 5, 2001, pages 270-278, XP008014948 ISSN: 1099-0461
- SOON SUNG LIM ET AL: "SYNTHESIS OF FLAVONOIDS AND THEIR EFFECTS ON ALDOSE REDUCTASE AND SORBITOL ACCUMULATION IN STREPTOZOTOCIN-INDUCED DIABETIC RAT TISSUES" JOURNAL OF PHARMACY AND PHARMACOLOGY, LONDON, GB, vol. 53, no. 5, mai 2001 (2001-05), pages 653-668, XP008014945 ISSN: 0022-3573
- PATENT ABSTRACTS OF JAPAN vol. 018, no. 415 (C-1233), 4 août 1994 (1994-08-04) & JP 06 122623 A (MINOFUAAGEN SEIYAKU HONPO:GOUSHI), 6 mai 1994 (1994-05-06)
- PATENT ABSTRACTS OF JAPAN vol. 012, no. 209 (C-504), 15 juin 1988 (1988-06-15) & JP 63 010720 A (NIPPON KAYAKU CO LTD), 18 janvier 1988 (1988-01-18) -& DATABASE CAPLUS [en ligne] CHEMICAL ABSTRACTS SERVICE, COLUMBUS, OHIO, US; retrieved from STN Database accession no. 110:88620 XP002236039
- HARAGUCHI H ET AL: "ANTIOXIDATIVE AND SUPEROXIDE SCAVENGING ACTIVITIES OF RETROCHALCONES IN GLYCYRRHIZA INFLATA" BIOORGANIC & MEDICINAL CHEMISTRY, ELSEVIER SCIENCE LTD, GB, vol. 6, no. 3, mars 1998 (1998-03), pages 339-347, XP001149404 ISSN: 0968-0896
- DATABASE CAPLUS [en ligne] CHEMICAL ABSTRACTS SERVICE, COLUMBUS, OHIO, US; retrieved from STTN Database accession no. 105:126832 XP002236041 & OGANESYAN ET AL.: "Study od structure-activity (SA) interrelations in the flavonoid series. I. Synthesis of chalcone derivatives and quantitative SA analysis" KHIMIKO-FARMATSEVTICHESKII ZHURNAL, vol. 20, no. 6, 1986, pages 696-702,
- HSU K-K ET AL: "STRUCTURE-ACTIVITY RELATIONSHIPS OF SUBSTITUTED FLAVONOIDS. (I)" TAIWAN KEXUE - FORMOSAN SCIENCE, TAIPEI, TW, vol. 27, no. 1/2, 1973, pages 23-26, XP002037232 ISSN: 0015-7791
- SZAJDA M ET AL: "New alkoxycarbonylalkyloxychalcones and their alpha, beta-dibromo derivatives of potential antimicrobial activity." DIE PHARMAZIE. GERMANY, EAST MAR 1989, vol. 44, no. 3, mars 1989 (1989-03), pages 190-191, XP002271328 ISSN: 0031-7144
- STOLL R ET AL: "Chalcone derivatives antagonize interactions between the human oncoprotein MDM2 and p53" BIOCHEMISTRY, AMERICAN CHEMICAL SOCIETY. EASTON, PA, US, vol. 40, no. 2, 16 janvier 2001 (2001-01-16), pages 336-344, XP002262903 ISSN: 0006-2960
- DATABASE CAPLUS [en ligne] CHEMICAL ABSTRACTS SERVICE, COLUMBUS, OHIO, US; retrieved from STN Database accession no. 86:55125 XP002271329 & SHI ET AL.: "Synthesis of ethyl flavone (or chalcone) oxyisobutyrate and its derivatives as antilipemic agents" TAIWAN YAOXUE ZAZHI, vol. 27, no. 1-2, 1975, pages 12-16,
- DATABASE CAPLUS [en ligne] CHEMICAL ABSTRACTS SERVICE, COLUMBUS, OHIO, US; retrieved from STN Database accession no. 69:26935 XP002271330 & PALANOWSKI ET AL.: "Synthesis of potential vasoactive compounds. I. phenylacrylophenone derivatives" ACTA POLONIAE PHARMACEUTICA (ENGLISH TRANSLATION), vol. 24, no. 6, 1967, pages 567-574,
- DATABASE CAPLUS [en ligne] CHEMICAL ABSTRACTS SERVICE, COLUMBUS, OHIO, US; retrieved from STN Database accession no. 91:20114 XP002271331 & JP 54 019947 A (TAISHO PHARMACEUTICAL CO.) 15 février 1979 (1979-02-15)
- DATABASE CAPLUS [en ligne] CHEMICAL ABSTRACTS SERVICE, COLUMBUS, OHIO, US; retrieved from STN Database accession no. 100:51185 XP002271332 & SAFAK ET AL.: "Chalcones. II. Synthesis of some chalcone derivatives and their antifungal activity against Candida albicans" FABAD FARMASOTIK BILIMLER DERGISI, vol. 8, no. 2, 1983, pages 80-88,
- DATABASE CAPLUS [en ligne] CHEMICAL ABSTRACTS SERVICE, COLUMBUS, OHIO, US; retrieved from STN Database accession no. 120:77943 XP002271333 & JP 05 255655 A (KANEBO LTD) 5 octobre 1993 (1993-10-05)
- DATABASE CAPLUS [en ligne] CHEMICAL ABSTRACTS SERVICE, COLUMBUS, OHIO, US; retrieved from STN Database accession no. 111:7099 XP002271334 & SUN, JINQIN ET AL.: "Stuides on flavonoids. VIII. Synthesis of 7-substituted flavones and 2',4-dihydroxy-3-methoxy -4'-substituted chalcones" GAODENG XUEXIAO HUAXUE XUEBAO, vol. 9, no. 8, 1988, pages 853-855,
- DATABASE CAPLUS [en ligne] CHEMICAL ABSTRACTS SERVICE, COLUMBUS, OHIO, US; retrieved from STN Database accession no. 111:173482 XP002271335 & SZAJDA ET AL.: "Carbon-13 NMR study of o-, m- and p-[(alkoxycarbonyl)alkoxy] chalcones and their alpha, beta-dibromo derivatives" MAGNETIC RESONANCE IN CHEMISTRY, vol. 27, no. 4, 1989, pages 399-402,

## Description

La présente invention concerne des compositions comprenant des dérivés de 1,3-diphénylprop-2-èn-1-one substitués et leurs utilisations, notamment dans les domaines de la santé humaine et animale. Les composés de l'invention possèdent des propriétés pharmacologiques anti-oxydantes ainsi que des propriétés d'activation de PPARα et PPARγ avantageuses. L'invention décrit plus précisément les différentes utilisations de ces composés et des compositions pharmaceutiques et cosmétiques les comprenant. Les composés de l'invention sont utilisables notamment pour prévenir ou traiter les maladies cardiovasculaires, le syndrome X, la resténose, le diabète, l'obésité, l'hypertension, les maladies inflammatoires, les cancers ou néoplasmes (tumeurs bénignes ou malignes), les maladies neurodégénératives, dermatologiques et les désordres liés au stress oxydatif, pour prévenir ou traiter les effets du vieillissement en général et par exemple le vieillissement cutané, notamment dans le domaine cosmétique (l'apparition de rides, etc.).

Les dérivés et/ou compositions de la présente invention peuvent être utilisés notamment pour traiter des maladies mettant en cause des tissus qui expriment PPARα et PPARγ. Plus précisément, ils permettent avantageusement de traiter des pathologies ou maladies inflammatoires, prolifératives, dégénératives affectant différents organes et tissus, notamment des maladies impliquant une angiogenèse pathologique ou une néovascularisation ainsi que toute pathologie ou désordre (par exemple lié à l'âge) impliquant un stress oxydatif. Les composés de la présente invention peuvent avantageusement être utilisés dans le cadre du traitement de maladies ou de désordres affectant des tissus et/ou organes, indifféremment de la composante étiologique.

Les PPARs, pour « peroxisome proliferator activated receptors », sont des récepteurs nucléaires membres de la super famille des facteurs de transcription activés par les ligands suivants : stéroïdes/thyroïdeslrétinoïdes. Trois isotypes de PPARs ont été, jusqu'à présent, clonés chez la souris et l'humain : PPARα, PPARβ/δ et PPARγ. Chez l'humain si l'expression de PPARβ/δ semble ubiquitaire, il existe une distribution tissulaire différentielle entre PPARα et γ (Braissant and Wahli 1998). PPARα est exprimé dans des cellules dont l'activité catabolique des acides gras est élevée ainsi que dans celles avec une forte activé des peroxysomes (hepatocytes, cardiomyocytes, tubules proximaux de rein, muqueuse intestinale). PPARβ/δ est exprimé de façon ubiquitaire et abondante dans la plupart des tissus. L'expression de PPARγ est quant à elle principalement limitée au tissu adipeux, à certaines cellules du système immunitaire, à la rétine et n'apparaît, dans d'autres organes, qu'à l'état de traces (Braissant and Wahli 1998).

Les PPARs possèdent plusieurs domaines dont les propriétés diffèrent. Un domaine de liaison à l'ADN (DBD) qui reconnaît des séquences spécifiques, également appelées éléments de réponse, localisées dans les régions de régulation de leurs gènes cibles. Comme les autres récepteurs nucléaires, les PPARs possèdent également un domaine de liaison avec un ligand, l'activation des PPARs par leur ligand modulant l'expression des gènes qui contiennent les éléments de réponse spécifiques des PPARs (PPRE) dans la région du promoteur. Pour activer la transcription de leurs gènes cibles, les PPARs activés doivent former un hétérodimère avec un autre récepteur nucléaire RXR (Retinoïd-X-Receptor). Si l'on prend l'exemple de PPARα, son action est médiée par une classe de composés comme les fibrates qui ont un effet hypolipémiant. Des ligands naturels ont également été identifiés comme par exemple, les acides gras, les eicosanoïdes (leukotriène B₄) et l'acide 8(S)-hydroxyeicosatetraenoïque (Kliewer, Sundseth et al. 1997).

Les PPARs ont été principalement associés au métabolisme des lipides et du glucose. Les activateurs de PPARs, les fibrates par exemple, permettent de réguler le cholestérol plasmatique ainsi que la concentration de triglycérides via l'activation de PPARα (Hourton, Delerive et al. 2001). Le traitement avec des fibrates entraîne une augmentation de l'oxydation des acides gras au niveau hépatique. Ils réduisent également la synthèse et l'expression des triglycérides (Staels and Auwerx 1998). Les activateurs de PPARα sont également capables de corriger une hyperglycémie ainsi que la concentration d'insuline. Les fibrates diminuent par ailleurs la masse du tissu adipeux grâce à un mécanisme indépendant de la prise alimentaire et de l'expression du gène codant pour la leptine (Guerre-Millo, Gervois et al. 2000).

L'intérêt thérapeutique des agonistes PPARγ a été largement étudié dans le traitement du diabète de type Il (Spiegelman 1998). Il a été montré que les agonistes PPARγ permettent la restauration de la sensibilité à l'insuline des tissus cibles ainsi que la réduction des taux plasmatiques de glucose, de lipide et d'insuline aussi chez des modèles animaux de diabète de type Il et chez l'homme (Ram VJ 2003)

L'activation des PPARs par les ligands intervient également dans la régulation de l'expression de gènes participant à des processus comme l'inflammation, l'angiogenèse, la prolifération et la différenciation cellulaire, l'apoptose et les activités de iNOS, de la MMPase et des TIMPs. L'activation de PPARα dans des kératinocytes entraîne un arrêt de leur prolifération et l'expression de gènes impliqués dans la différenciation (Komuves, Hanley et al. 2000). Les PPARs ont des propriétés anti-inflammatoires car ils interfèrent négativement dans des mécanismes de transcription impliquant d'autres facteurs de transcription tels que NF-kB ou les activateurs de la transcription (STAT) et AP-1 (Desvergne and Wahli 1999). Ces propriétés anti-inflammatoires et anti-prolifératives font des PPARs (et notamment de PPARα) des cibles thérapeutiques d'intérêt pour le traitement de maladies comme les maladies occlusives vasculaires (athérosclérose, etc.), l'hypertension, les maladies liées à une néo-vascularisation (rétinopathies diabétiques, etc.), les maladies inflammatoires (maladie de Bowel, psoriasis, etc.) et les maladies néoplasiques (carcinogenèse, etc.).

Les radicaux libres interviennent dans un spectre très large de pathologies comme les allergies, l'initiation et la promotion cancéreuse, les pathologies cardiovasculaires (athérosclérose, ischémie, etc.), les désordres génétiques et métaboliques (diabètes, etc.), les maladies infectieuses et dégénératives (Alzheimer, Parkinson, Prion, etc.), les problèmes ophtalmiques et le vieillissement (Mates, Perez-Gomez et al. 1999).

Les espèces réactives oxygénées (ROS) sont produites pendant le fonctionnement normal de la cellule. Les ROS sont constituées de radicaux hydroxyle (OH), de l'anion superoxyde (O₂°₋), du peroxyde d'hydrogène (H₂O₂) et de l'oxyde nitrique (NO). Ces espèces sont très labiles et du fait de leur grande réactivité chimique, elles constituent un danger pour les fonctions biologiques des cellules. Elles provoquent des réactions de peroxydation lipidique, l'oxydation de certains enzymes et des oxydations très importantes des protéines qui mènent à leur dégradation. La protection contre la peroxydation lipidique est un processus essentiel chez les organismes aérobies, car les produits de peroxydation peuvent causer des dommages à l'ADN. Ainsi un dérèglement ou une modification de l'équilibre entre la production, la prise en charge et l'élimination des espèces radicalaires par les défenses antioxydantes naturelles conduisent à la mise en place de processus délétères pour la cellule ou l'organisme.

La prise en charge des ROS se fait via un système antioxydant qui comprend une composante enzymatique et une non enzymatique.

Le système enzymatique se compose de plusieurs enzymes dont les caractéristiques sont les suivantes :
- La superoxyde dismutase (SOD) détruit le radical superoxyde en le convertissant en peroxyde. Ce dernier est lui même pris en charge par un autre système enzymatique. Un faible niveau de SOD est constamment généré par la respiration aérobie. Trois classes de SOD ont été identifiées chez l'homme, elles contiennent chacune du Cu, Zn, Fe, Mn, ou Ni comme cofacteur. Les trois formes de SOD humaines sont réparties de la manière suivante : les Cu-Zn SOD qui sont cytosoliques, une Mn-SOD mitochondriale et une SOD extracellulaire.
- La catalase est très efficace pour convertir le peroxyde d'hydrogène (H₂O₂) en eau et en O₂. Le peroxyde d'hydrogène est catabolisé de manière enzymatique dans les organismes aérobies. La catalase catalyse également la réduction d'une variété d'hydroperoxydes (ROOH).
- La glutathion peroxydase contient du sélénium comme cofacteur et catalyse la réduction d'hydroperoxydes (ROOH et H₂O₂) en utilisant du glutathion. Elle protège ainsi les cellules contre les dommages oxydatifs.

Les défenses cellulaires antioxydantes non enzymatiques sont constituées par des molécules qui sont synthétisées ou apportées par l'alimentation.

II existe des molécules antioxydantes présentes dans différents compartiments cellulaires. Les enzymes détoxifiantes sont par exemple des molécules chargées d'éliminer les radicaux libres et sont indispensables à la vie de la cellule. Les trois types de composés antioxydants les plus importants sont les caroténoïdes, la vitamine C et la vitamine E (Gilgun-Sherki, Melamed et al. 2001).

Le brevet US4656305, la demande de brevet FR2383157, l'article de Shibata S. et al, « Anti-tumorigenic chalcones », Stem Cells, Alphamed Press, Dayton, OH, US, vol. 12, 1994, p. 44-52, l'article de Shi et al., « Synthesis of ethyl flavone (or chalcone) oxyisobutyrate and its derivatives as potential antilipidemic agents », Taiwan Yaoxue Zazhi., vol. 27, n°1-2, 1975, p. 12-16, et le document JP54019947, décrivent des dérivés de chalcone.

La demande de brevet WO0198291 décrit des composés 1,3-bis-phenyl-2-propen-1-one substitués par un groupement aryl, un hétéroaromatique ou un hétérocycle.

Le document DE4327365 décrit des composés dérivés du phénol.

Le brevet US3558612 décrit des dérivés de l'acide 4-carbonylvinylphenoxyacétique.

La demande de brevet EP0947511 décrit des dérivés de l'acide phénoxyacétique et du phénoxyméthyltétrazole.

Le brevet US3994955 décrit des acides phénoxydialkylacétiques substitués et les esters correspondants.

L'article de Hsu et al., « Structure-activity relationships of substituted flavonoids. (I) », Taiwan Kexue - Formosan Science, Taipei, TW, vol. 27, n°1/2, 1973, p. 23-26, décrit le screening de 300 composés de type chalcones, flavanones, flavones, flavonols et acetophénones.

L'article de Szajda et al., « New alkoxycarbonylalkyloxychalcones and their alpha, beta-dibromo derivatives of potential antimicrobial activity », Die Pharmazie. Germany, East Mar 1989, vol.44, n°3, mars 1989, p. 190-191, décrit des composés d'alkoxycarbonylalkyloxychalcones et leurs dérivés substitués par un brome en alpha et beta.

L'article Palanowski et al., « Synthesis of potential vasoactive compounds. I. phenylacrylophenone derivatives », Acta Poloniae Pharmaceutica, vol. 24, n°6, 1967, p. 567-574 décrit des dérivés de phenylacrylophénone ayant des propriétés vasodilatatrices et diurétiques.

L'article de Safak et al., « Chalcones. II. Synthesis of some chalcone derivatives and their antifungal activity against Candida albicans », Fabad Farmasotik Bilimler Dergisi, vol. 8, n°2, 1983, p. 80-88, et le document JP05255655 décrivent la synthèse de dérivés de chalcones.

Les inventeurs ont mis en évidence que, de manière surprenante, les composés selon l'invention possèdent une activité PPARα agoniste et des propriétés antioxydantes. Les composés selon l'invention sont donc capables d'interférer avec au moins deux voies de signalisation qui sont activées en particulier pendant l'inflammation : la production de cytokines ainsi que la production de radicaux libres. En agissant de manière synergique les composés selon l'invention représentent un moyen thérapeutique avantageux pour le traitement de pathologies liées à l'inflammation (athérosclérose, allergies, asthme, eczéma, démangeaisons, etc.), aux neurodégénérescences (Alzheimer, Parkinson, etc.), aux dérèglements du métabolisme lipidique et/ou glucidique (diabètes, athérosclérose, obésité, etc.), à la prolifération/différenciation cellulaire (carcinogenèse, etc.) et aux désordres liés au vieillissement (cutané ou du système nerveux central, etc.).

Les inventeurs ont mis en évidence que les composés selon l'invention ont à la fois des propriétés d'activateurs PPAR, d'antioxydants et d'anti-inflammatoires.

La présente invention concerne ainsi, des compositions pharmaceutiques comprenant au moins un dérivé de 1,3-diphénylprop-2-èn-1-one substitués pour le traitement de pathologies liées à l'inflammation, à la neurodégénérescence, aux dérèglements du métabolisme lipidique et/ou glucidique, à la prolifération et/ou à la différentiation cellulaire et/ou au vieillissement cutané ou du système nerveux central.

La présente invention a donc notamment pour objet , selon la revendication 1, une composition comprenant, dans un support acceptable sur le plan pharmaceutique, au moins un dérivé de 1,3-diphénylprop-2-èn-1-one substitué de formule (1) suivante : 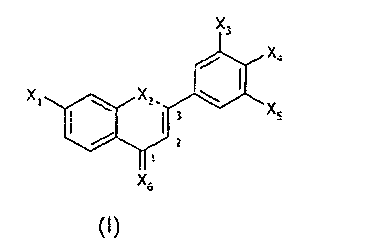 dans laquelle :
X1 représente un halogène ou un groupement -R1 ou un groupement répondant à la formule suivante : -G1-R1,
X2 représente un atome d'hydrogène ou un groupement thionitroso ou un groupement hydroxy ou un groupement alkyloxy non substitué ou un groupement alkylcarbonyloxy ou un groupement thiol ou un groupement alkylthio ou un groupement alkylcarbonylthio, X2 peut également représenter un atome d'oxygène ou de soufre lié au carbone 3 de la chaîne propène, pour former un dérivé de type 2-phényl-4H-1-benzopyran-4-one ou de type 2-phenyl-4H-1-benzothiopyran-4-one (cette possibilité est représentée dans la formule (1) par les pointillés),
X3 représente un groupement -R3 ou un groupement répondant à la formule suivante : -G3-R3,
X4 représente un halogène ou un groupement thionitroso ou un groupement -R4 ou un groupement répondant à la formule suivante : -G4-R4,
X5 représente un groupement -R5 ou un groupement répondant à la formule suivante : -G5-R5,
X6 est un atome d'oxygéne,
R1, R3, R4, R5, identiques ou différents, représentent un atome d'hydrogène ou un groupement alkyle substitué ou non par au moins un groupement faisant partie du groupe 1 ou du groupe 2 définis ci-dessous,
G1, G3, G4, G5, identiques ou différents, représentent un atome d' oxygène ou de soufre,
avec au moins un des groupements X1, X3, X4 ou X5 répondant à la formule -G-R dans laquelle G représente un atome de soufre, et
avec au moins un des groupements R1, R3, R4 ou R5 présent sous la forme d'un radical alkyle portant au moins un substituant du groupe 1 ou 2, ledit radical alkyle étant lié directement au cycle ou étant associé à un groupement G selon la formule -GR,
les substituants du groupe 1 sont choisis parmi les groupements carboxy de formule : -COOR₆ et les groupements carbamoyle de formule : -CONR₆R₇,
les substituants du groupe 2 sont choisis parmi l'acide sulfonique (-SO₃H) et les groupements sulfonamide de formule : -SO₂NR₆R₇
avec R₆ et R₇, identiques ou différents, représentant un atome d'hydrogène ou un radical alkyle éventuellement substitué par au moins un groupe de type 1 ou 2,
leurs isomères optiques et géométriques, leurs racémates, leurs tautomères, leurs sels, leurs hydrates et leurs mélanges,
à l'exclusion des composés de formule (1) dans laquelle :
   - X₂ représente un atome d'hydrogène et X₁ représente -G1R1 où G1 représente un atome d'oxygène et R1 représente CH2COOH.

La présente invention a également pour objet une composition comprenant dans un support acceptable sur le plan pharmaceutique, au moins un dérivé de 1,3-diphénylprop-2-èn-1-one substitué de formule telle que définie dans la revendication 2.

Cette composition peut être utilisée notamment pour le traitement ou la prophylaxie d'une pathologie liée à l'inflammation, à la neurodégénérescence, aux dérèglements du métabolisme lipidique et/ou glucidique, à la prolifération et/ou à la différentiation cellulaire et/ou au vieillissement cutané ou du système nerveux central.

La présente invention inclut également une composition comprenant des prodrogues des composés de formule (1), qui, après administration chez un sujet, vont se transformer en composés de formule (I), et/ou les métabolites des composés de formule (I) qui présentent des activités thérapeutiques comparables aux composés de formule (I), éventuellement en association avec un autre actif thérapeutique, pour le traitement ou la prophylaxie d'une pathologie liée à l'inflammation, à la neurodégénérescence, aux dérèglements du métabolisme lipidique et/ou glucidique, à la prolifération et/ou à la différentiation cellulaire et/ou au vieillissement cutané ou du système nerveux central.

Dans le cadre de la présente invention, les dérivés de formule (1) tels que décrits ci-dessus peuvent adopter la conformation cis ou trans. Une composition selon l'invention peut ainsi comprendre des dérivés correspondant à la conformation cis, trans ou leur mélange.

De manière avantageuse, aucun des groupements X3, X4 et X5 ne représente un atome d'hydrogène. Les composés de la formule (I) répondant à la précédente définition constituent les composés de la formule générale (II).

De manière avantageuse, un ou deux des groupements X3, X4 et X5 représente un atome d'hydrogène et X1 est un groupement alkyle non substitué. Les composés de la formule (1) répondant à la précédente définition constituent les composés de la formule générale (III).

De manière avantageuse, un ou deux des groupements X3, X4 et X5 représente un atome d'hydrogène et X2 est groupement thionitroso ou un groupement alkylcarbonyloxy ou un groupement thiol ou un groupement alkylthio ou un groupement alkylcarbonylthio, X2 peut également représenter un atome d'oxygène ou de soufre lié au carbone 3 de la chaîne propène, pour former un dérivé de type 2-phényl-4H-1-benzopyran-4-one (cette possibilité est représentée dans la formule (I) par les pointillés). Les composés de la formule (I) répondant à la précédente définition constituent les composés de la formule générale (IV).

De manière avantageuse, un ou deux des groupements X3, X4 et X5 représente un atome d'hydrogène et au moins un des groupements X1, X2, X3, X4 ou X5 est de la forme GR dans laquelle G est un atome de soufre. Les composés de la formule (1) répondant à la précédente définition constituent les composés de la formule générale (V).

De manière avantageuse, un ou deux des groupements X3, X4 et X5 représente un atome d'hydrogène et au moins un des groupements X1, X3, X4 ou X5 est de la forme -G-R dans laquelle G est un atome d'oxygène et R est un groupement alkyle substitué par un substituant du groupe 1 où R6 n'est pas un atome d'hydrogène. Les composés de la formule (1) répondant à la précédente définition constituent les composés de la formule générale (VI).

De manière avantageuse, un ou deux des groupements X3, X4 et X5 représente un atome d'hydrogène et au moins un des groupements X1, X3, X4 ou X5 est de la forme GR dans laquelle G est un atome d'oxygène et R est un groupement alkyle substitué par une sulfonamide telle que définie ci-dessus. Les composés de la formule (1) répondant à la précédente définition constituent les composés de la formule générale (VII).

De manière avantageuse, X4 est un groupement thionitroso ou un groupement -R4 ou un groupement répondant à la formule -G4-R4. Les dérivés de la formule (1) dans lesquels X4 répond à la précédente définition représentent les dérivés de formule générale (VIII) dans laquelle G4 et R4 sont tels que définis précédemment.

De manière avantageuse, X2 est un groupement thionitroso ou un groupement hydroxy ou un groupement alkyloxy ou un groupement thiol ou un groupement alkylthio. Les dérivés de la formule (1) dans lesquels X2 répond à la précédente définition représentent les dérivés de formule générale (IX).

D'autres dérivés avantageux de la formule (I) de l'invention ont une formule générale (X) telle que X4 est un groupement thionitroso ou un groupement -R4 ou un groupement répondant à la formule -G4-R4 et X2 est un groupement thionitroso ou un groupement hydroxy ou un groupement alkyloxy ou un groupement thiol ou un groupement alkylthio, G4 et R4 étant tels que définis précédemment.

D'autres dérivés avantageux de la formule (1) de l'invention ont une formule générale (XI) telle que X1 représente un groupement -R1 ou un groupement répondant à la formule -G1-R1, avec R1 étant un groupement alkyle substitué par un groupement faisant partie du groupe 1 et G1 et le substituant du groupe 1 étant tels que définis précédemment.

Plus préférentiellement, un autre objet de l'invention concerne les dérivés de la formule (XI) tels que décrits précédemment, caractérisés en ce que X1 est un groupement -G1-R1 .

Encore plus préférentiellement, un autre objet de l'invention concerne les dérivés de la formule (XI) tels que décrits précédemment, caractérisés en ce que X1 est un groupement -G1-R1 dans lequel G1 est un atome d'oxygène.

D'autres dérivés avantageux de la formule (I) de l'invention ont une formule générale (XII) telle que X1 représente un groupement -R1 ou un groupement répondant à la formule -G1-R1, avec R1 étant un groupement alkyle substitué par un groupement faisant partie du groupe 2 et G1 et le substituant du groupe 2 étant tels que définis précédemment.

D'autres dérivés avantageux de la formule (I) de l'invention ont une formule générale (XIII) telle que X3 représente un groupement -R3 ou un groupement répondant à la formule -G3-R3, avec R3 étant un groupement alkyle substitué par un groupement faisant partie du groupe 1 et G3 et le substituant du groupe 1 étant tels que définis précédemment.

D'autres dérivés avantageux de la formule (1) de l'invention ont une formule générale (XIV) telle que X3 représente un groupement -R3 ou un groupement répondant à la formule -G3-R3, avec R3 étant un groupement alkyle substitué par un groupement faisant partie du groupe 2 et G3 et le substituant du groupe 2 étant tels que définis précédemment.

D'autres dérivés avantageux de la formule (1) de l'invention ont une formule générale (XV) telle que X4 représente un groupement -R4 ou un groupement répondant à la formule -G4-R4 avec R4 étant un groupement alkyle substitué par un groupement faisant partie du groupe 1 et G4 et le substituant du groupe 1 étant tels que définis précédemment.

Plus préférentiellement, un autre objet de l'invention concerne des dérivés de la formule (XV) tels que décrits précédemment, caractérisés en ce que X4 est un groupement -G4-R4 .

Encore plus préférentiellement, un autre objet de l'invention concerne des dérivés de la formule (XV) tels que décrits précédemment, caractérisés en ce que X4 est un groupement -G4-R4 dans lequel G4 est un atome d'oxygène.

Encore plus préférentiellement, un autre objet de l'invention concerne des dérivés de la formule (XV) tels que décrits précédemment, caractérisés en ce que X4 est un groupement -G4-R4 dans lequel G4 est un atome d'oxygène, et X3 ou X5 représente respectivement R3 ou G3R3, d'une part, et R5 ou G5R5, d'autre part, avec R3 ou R5 étant des un groupements alkyles portant un substituant du groupe 1, ledit substituant du groupe 1 étant tel que défini précédemment.

D'autres dérivés avantageux de la formule (I) de l'invention ont une formule générale (XVI) telle que X4 représente un groupement -R4 ou un groupement répondant à la formule -G4-R4 avec R4 étant un groupement alkyle substitué par un groupement faisant partie du groupe 2 et G4 le substituant du groupe 2 étant tels que définis précédemment.

D'autres dérivés avantageux de la formule (I) de l'invention ont une formule générale (XVII) telle que X1 représente un halogène.

D'autres dérivés avantageux de la formule (I) de l'invention ont une formule générale (XVIII) telle que X1 représente un groupement -R1 avec R1 étant un groupement alkyle de C1 à C4 substitué ou non par au moins un groupement faisant partie du groupe 1 ou du groupe 2 définis ci-dessus.

D'autres dérivés avantageux de la formule (1) de l'invention ont une formule générale (XIX) telle que X1 représente un groupement -G1R1 avec R1 étant un groupement alkyle de C1 à C3 substitué ou non par au moins un groupement faisant partie du groupe 1 ou du groupe 2 définis ci-dessus.

D'autres dérivés avantageux de la formule (1) de l'invention ont une formule générale (XX) telle que X1 représente un groupement -R1 avec R1 étant un groupement alkyle de C5 à C24 substitué ou non par au moins un groupement faisant partie du groupe 1 ou du groupe 2 définis ci-dessus

D'autres dérivés avantageux de la formule (I) de l'invention ont une formule générale (XXI) telle que X1 représente un groupement -G1R1 avec R1 étant un groupement alkyle de C4 à C24 substitué ou non par au moins un groupement faisant partie du groupe 1 ou du groupe 2 définis ci-dessus.

Un autre objet de l'invention concerne les dérivés de formule (I) dans laquelle X1, X3, X4 ou X5 est de la forme OC(CH3)2COOR6 avec R6 étant tel que défini précédemment.

Un autre objet de l'invention concerne les dérivés de formule (I) dans laquelle X1, X3, X4 ou X5 est de la forme SC(CH3)2COOR6 avec R6 étant tel que défini précédemment.

Selon la présente invention, le terme "alkyle" désigne un radical hydrocarboné saturé, linéaire, ramifié ou cyclique ayant de plus particulièrement 1 à 24, de préférence 1 à 10, atomes de carbone tels que méthyle, éthyle, propyle, isopropyle, butyle, isobutyle, *tert*-butyle, pentyle, néopentyle, n-hexyle. Les groupes comportant un ou deux atome de carbone ou comportant de deux à sept atomes de carbone sont particulièrement préférés. Les groupes méthyle et éthyle sont tout particulièrement préférés.

Le terme thionitroso fait référence à un groupement nitroso lié au cycle aromatique par l'intermédiaire d'un atome de soufre.

Le terme halogène représente un atome de chlore ou un atome de brome ou un atome d'iode ou un atome de fluor.

Le terme alkyloxy fait référence à une chaîne alkyle liée au cycle par l'intermédiaire d'un atome d'oxygène. La chaîne alkyle répond à la définition précédemment énoncée.

Le terme alkylthio fait référence à une chaîne alkyle liée au cycle aromatique par l'intermédiaire d'un atome de soufre (liaison thicéther). La chaîne alkyle répond à la définition précédemment énoncée.

Selon un mode particulier de l'invention, les dérivés préférés sont indiqués ci-dessous avec les formules qui leur sont associées :
le 1-[2-hydroxy-4-carboxydiméthylméthyloxyphényl]-3-[3,5-di*tert*butyl-4-hydroxyphényl]prop-2-èn-1-one, le 1-[2-hydroxy-4-isopropyloxycarbonyl diméthylméthyloxyphényl]-3-[3,5-di*tert*butyl-4-hydroxyphényl]prop-2-èn-1-one et le 1-[2-hydroxy-4-éthoxycarbonyldiméthylméthyloxyphényl]-3-[3,5-ditertiobutyl-4-hydroxyphényl]prop-2-èn-1-one: 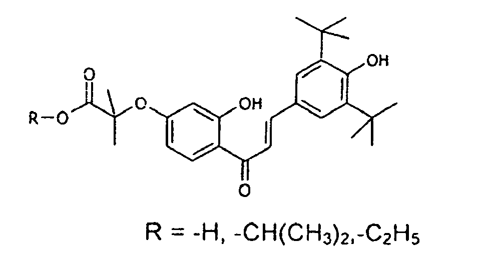
le 1-[2-hydroxyphényl]-3-[3-carboxydiméthylméthyloxy-4-hydroxy-5-*tert*butylphényl]prop-2-èn-1-one et le 1-[2-hydroxyphényl]-3-[3-*iso*propyloxycarbonyldiméthylméthyloxy-4-hydroxy-5-*tert*butylphényl]prop-2-én-1-one: 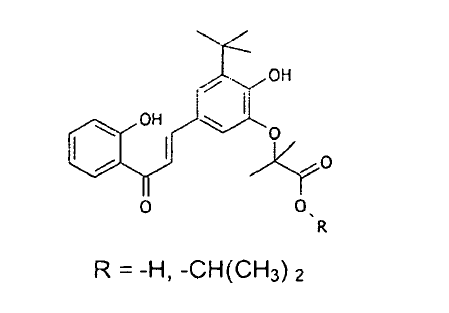
le 1-[2-hydroxy-4-chlorophényl]-3-[3-carboxydiméthylméthyloxy-4-hydroxy-5-*ter*tbutylphényl]prop-2-èn-1-one et le 1-[2-hydroxy-4-chlorophényl]-3-[3-*i*s*o*propyloxycarbonyldiméthylméthyloxy-4-hydroxy-5-*tert*butylphényl]prop-2-èn-1-one: 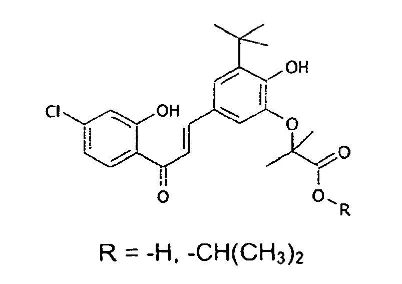
le 1-[2-hydroxyphényl]-3-[3-carboxydiméthylméthyl-4-hydroxy-5-*ter*tbutylphényl]prop-2-èn-1-one et le 1-[2-hydroxyphényl]-3-[3-*iso*propyloxycarbonyldiméthylméthyl-4-hydroxy-5-*tert*butylphényl]prop-2-èn-1-one (: 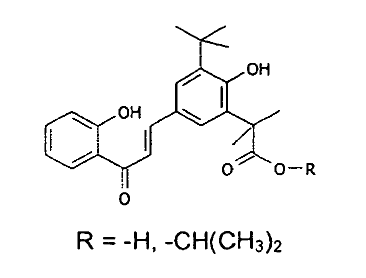
le 1-[2-hydroxy-4-chlorophényl]-3-[3-carboxydiméthylméthyl-4-hydroxy-5-*tert*butylphényl]prop-2-èn-1-one et le 1-[2-hydroxy-4-chlorophényl]-3-[3-*iso*propyloxycarbonyldiméthylméthyl-4-hydroxy-5-*tert*butylphényl]prop-2-èn-1-one: 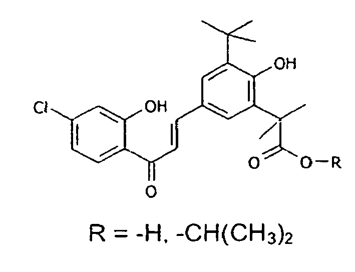
le 1-[2-hydroxy-4-chlorophényl]-3-[3,5-diméthoxy-4-carboxydiméthylméthyloxyphényl]prop-2-èn-1-one et le 1-[2-hydroxy-4-chlorophényl]-3-[3,5-diméthoxy-4-*iso*propyloxycarbonyldiméthylméthyloxy phényl]prop-2-èn-1-one: 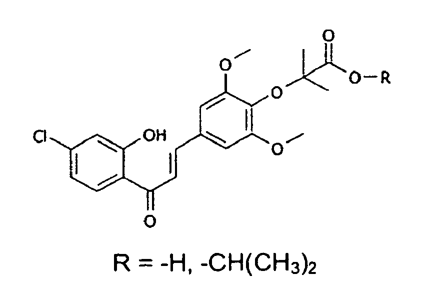
le 1-[2-hydroxyphényl]-3-[3,5-diméthoxy-4-carboxydiméthylméthyloxyphényl]prop-2-èn-1-one et le 1-[2-hydroxyphényl]-3-[3, 5-diméthoxy-4-*iso*propyloxycarbonyldiméthylméthyloxyphényl]prop-2-èn-1-one: 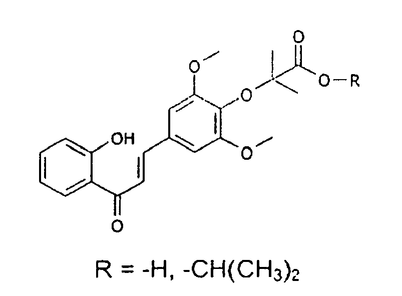
le 1-[2-hydroxy-4-carboxydiméthylméthyloxyphényl]-3-[3,5-diméthoxy-4-hydroxyphényl]prop-2-èn-1-one et le 1-[2-hydroxy-4-*iso*propyloxycarbonyldiméthylméthyloxyphényl]-3-[3,5-di-méthoxy-4-hydroxyphényl]prop-2-èn-1-one 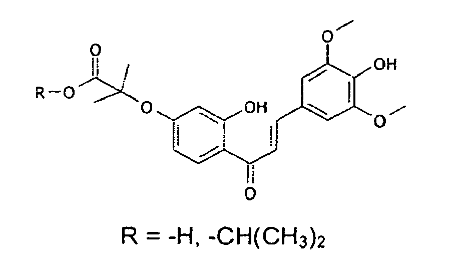
le 1-[2-hydroxy-4-chlorophényl]- 3-[3,4-dihydroxy-5-carboxydiméthylméthyloxyphényl]prop-2-èn-1-one et le 1-[2-hydroxy-4-chlorophényl]- 3-[3,4-dihydroxy-5-*iso*propyloxycarbonyldiméthylméthyloxyphényl]- 2-propen-1-one: 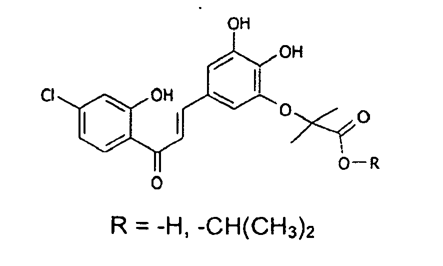
le 1-[2-hydroxy-4-carboxydiméthylméthyloxyphényl]- 3-[3,5-diméthyl-4-hydroxyphényl]prop-2-èn-1-one et le 1-[2-hydroxy-4-*iso*propyloxycarbonyl diméthylméthyloxyphényl]- 3-[3,5-diméthyl-4-hydroxyphényl]prop-2-èn-1-one: 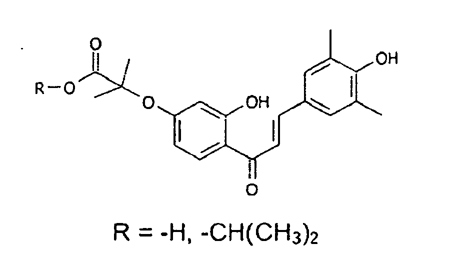
le 1-[2-hydroxy-4-chlorophényl]-3-[3,5-diméthyl-4-carboxydiméthylméthyl oxyphényl]prop-2-èn-1-one et le 1-[2-hydroxy-4-chlorophényl]-3-[3,5-diméthyl-4-*iso*propyloxycarbonyldiméthylméthyloxy phényl]prop-2-èn-1-one: 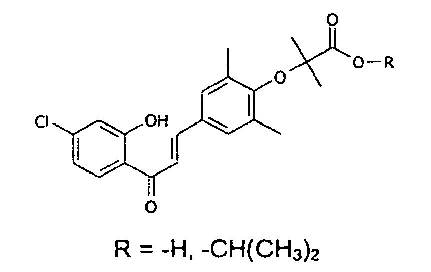
et le 1-[2-hydroxyphényl]-3-[3,5-diméthyl-4-carboxydiméthylméthyloxyphényl]prop-2-èn-1-one et le 1-[2-hydroxyphényl]-3-[3,5-diméthyl-4-*i*sopropyloxycarbonyl diméthylméthyioxyphényl]prop-2-èn-1-one: 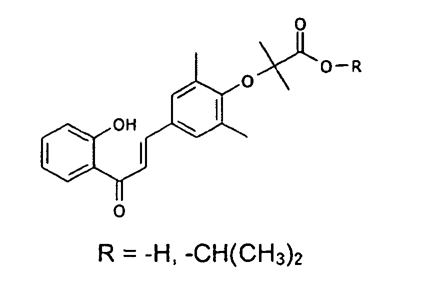
le 1-[2-hydroxyphényl]-3-[4-carboxydiméthylméthylthiophényl]prop-2-èn-1-one et le 1-[2-hydroxyphényl]-3-[4-*iso*propyloxycarbonyldiméthylméthylthiophényl]prop-2-èn-1-one: 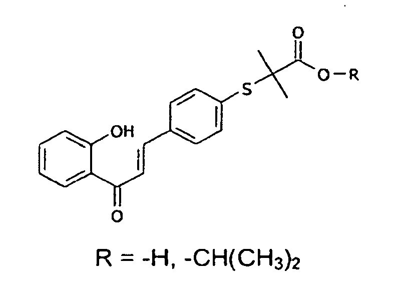
le 1-[2-hydroxy-4-carboxydiméthylméthyloxyphényl]-3-[4-méthylthiophényl]prop-2-èn-1-one,
le 1-[4-chlorophényl]-3-[3,5-diméthyl-4-tertiobutyloxycarbonyldiméthylméthyloxyphényl]prop-2-èn-1-one
le1-[4-chlorophényl]-3-[3,5-diméthyl-4-isopropyloxycarbonyldiméthylméthyloxyphényl]prop-2-èn-1-one
le 1-[4-chlorophényl]-3-[3,5-diméthyl-4-carboxydiméthylméthyloxyphényl]prop-2-èn-1-one
le 1-[2-hydroxyphényl]-3-[4-carboxydiméthylméthylthiophényl]prop-2-èn-1-one
le 1-[4-chloro-2-hydroxyphényl]-3-[4-carboxydiméthylméthylthiophényl]prop-2-èn-1-one
le 1-[4-carboxydiméthytméthyloxyphényl]-3-[3,5-diméthyl4-hydroxyphényl]prop-2-èn-1-one
le 1-[4-méthylthiophényl]-3-[4-carboxydiméthylméthyloxyphényl]prop-2-èn-1-one
le 1-[4-carboxydiméthylméthylthiophényl]-3-[4-méthylthiophényl]prop-2-èn-1-one le 1-[2-hydroxy-4-bromophényl]-3-[3,5-diméthyl-4-carboxydiméthylméthyloxyphényl]prop-2-èn-1-one
le 1-[4-carboxydiméthylméthyloxyphényl]-3-[4-méthylthiophényl]prop-2-èn-1-one le 1-[4-méthylthiophényl]-3-[3,5-diméthyl-4-tertiobutyloxycarbonyldiméthylméthyloxyphényl]prop-2-èn-1-one
le 1-[4-méthylthiophényl]-3-[3,5-diméthyl-4-isopropyloxycarbonyldiméthylméthyloxyphényl]prop-2-èn-1-one
le 1-[4-méthylthiophényl]-3-[3,5-diméthyl-4-carboxydiméthylméthyloxyphényl]prop-2-èn-1-one
le 1-[2-méthoxyphényl]-3-[3,5-diméthyl-4-tertiobutyloxycarbonyldiméthylméthyloxyphényl]prop-2-èn-1-one
le 1-[2-méthoxyphényl]-3-[3,5-diméthyl-4-carboxydiméthylrnéthyloxyphényl]prop-2-èn-1-one
le 1-[4-hexyloxyphényl]-3-[3,5-diméthyl-4-tertiobutyloxycarbonyldiméthylméthyloxyphényl]prop-2-èn-1-one
le 1-[4-hexyloxyphényl]-3-[3,5-diméthyl-4-carboxydiméthylméthyloxyphényl]prop-2-èn-1-one
le 1-[2-méthyloxy-4-chlorophényl]-3-[3,5-diméthyl-4-tertiobutyloxycarbonyldiméthylméthyloxyphényl]prop-2-èn-1-one
le 1-[2-méthyloxy-4-chlorophényl]-3-[3,5-diméthyl-4-carboxydiméthylméthyloxyphényl]prop-2-èn-1-one
le 1-[4-heptylphényl]-3-[3,5-diméthyl-4-tertiobutyloxycarbonyldiméthylméthyloxyphényl]prop-2-èn-1-one
le 1-[4-heptylphényl]-3-[3,5-diméthyl-4-carboxydiméthylméthyloxyphényl]prop-2-èn-1-one
le 1-[4-bromophényl]-3-[3,5-diméthyl-4-tertiobutyloxycarbonyldiméthylméthyloxyphényl]prop-2-èn-1-one
le 1-[4-bromophényl]-3-[3,5-diméthyl-4-carboxy diméthylméthyloxyphényl]prop-2-èn-1-one

L'invention concerne ainsi l'utilisation d'au moins un composé de formule (I) tel que défini ci-dessus et en particulier l'un des composés décrit de manière avantageuse ou préférée pour la préparation d'une composition pharmaceutique destinée à traiter de manière préventive ou de préférence curative une pathologie liée à l'inflammation, à la neurodégénérescence, aux dérèglements du métabolisme lipidique et/ou glucidique, à la prolifération et/ou à la différentiation cellulaire et/ou au vieillissement cutané ou du système nerveux central, telles que les allergies, l'asthme, l'eczéma, le psoriasis, les démangeaisons, la maladie d'Alzheimer, la maladie de Parkinson, le diabète, l'athérosclérose, l'obésité, la carcinogenèse, etc.

La présente invention fournit également un procédé de préparation de composés ou dérivés de formule (I).

Le procédé de la présente invention comprend une mise en contact en milieu basique ou en milieu acide d'au moins un composé de formule (A) avec au moins un composé de formule (B), les formules (A) et (B) étant : 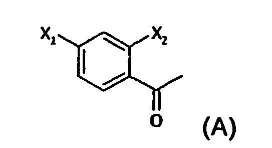 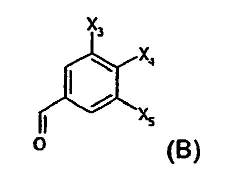 formules dans lesquelles X1, X2, X3, X4 et X5 ont les définitions données précédemment.

Les conditions de mise en oeuvre de cette réaction en milieu acide ou basique sont à la portée de l'homme du métier et peuvent varier dans une large mesure.

La mise en contact de ces deux composés est avantageusement réalisée de manière stoechiométrique. Elle est réalisée de préférence à une température ambiante (entre environ 18°C et 25°C) et à pression atmosphérique.

En milieu basique, la réaction est de préférence réalisée en présence d'une base forte, tel qu'un hydroxyde de métal alcalin, comme l'hydroxyde de sodium ou un alcoolate de métal alcalin comme l'éthylate de sodium.

En milieu acide, la réaction est de préférence réalisée en présence d'un acide fort, tel que l'acide chlorhydrique.

Le schéma réactionnel peut être représenté comme suit :

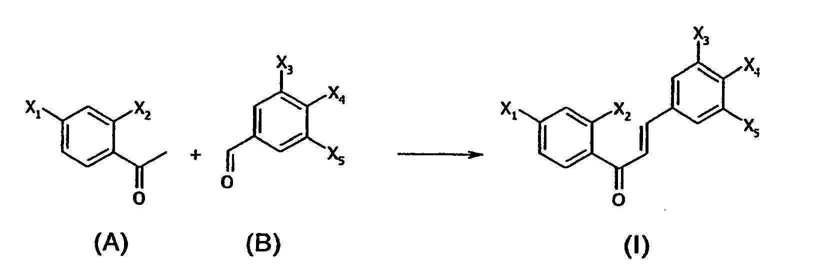

La synthèse en milieu basique peut être réalisée de la façon suivante :
La cétone (composé (A) à 1 équivalent-molaire et l'aldéhyde (composé (B)) à 1 équivalent-molaire sont solubilisés dans une solution hydroalcoolique d'hydroxyde de sodium à 20 équivalents-molaire. L'ensemble est agité pendant environ 18 heures à température ambiante (entre 18 et 25°C). Le milieu est ensuite acidifié (pour atteindre en particulier un pH d'environ 2) notamment avec de l'acide chlorhydrique.
La 1,3-diphénylprop-2-èn-1-one substituée attendue peut être obtenue par précipitation ou extraction solide/liquide après évaporation du milieu réactionnel. Elle peut être ensuite purifiée par chromatographie sur gel de silice ou par recristallisation.

La synthèse en milieu acide peut être réalisée de la façon suivante :

La cétone (composé (A)) à 1 équivalent-molaire et l'aldéhyde (composé B) à 1 équivalent-molaire sont solubilisés dans une solution d'éthanol saturée d'acide chlorhydrique gazeux. L'ensemble est agité à température ambiante pendant environ 6 heures, le solvant est éliminé, notamment par évaporation sous pression réduite. La 1,3-diphénylprop-2-èn-1-one substituée est purifiée, notamment par chromatographie sur gel de silice.

L'invention concerne ainsi l'utilisation d'un composé ou dérivé tel que défini ci-avant pour la préparation d'une composition pharmaceutique destinée à la mise en oeuvre d'une méthode de traitement ou de prophylaxie du corps humain ou animal.

Les compositions pharmaceutiques ou les composés de formule (I) selon l'invention sont avantageusement utilisées pour le traitement ou la prophylaxie des pathologies liées à l'inflammation, à la neurodégénérescence, aux dérèglements du métabolisme lipidique et/ou glucidique, à la prolifération et/ou à la différentiation cellulaire et/ou au vieillissement cutané ou du système nerveux central et plus particulièrement d'une ou plusieurs allergies, de l'asthme, de l'eczéma, du psoriasis, des démangeaisons, de la maladie d'Alzheimer, de la maladie de Parkinson, du diabète, de l'athérosclérose, de l'obésité, de la carcinogenèse, etc.. II a en effet été trouvé de manière surprenante que les composés de formule (I) possèdent des propriétés pharmacologiques anti-oxydantes ainsi que des propriétés d'activation de PPARα et PPARγ avantageuses.

Dans le cas où la composition selon l'invention est destinée au traitement ou à la prophylaxie d'une pathologie liée à la neurodégénérescence, aux dérèglements du métabolisme lipidique et/ou glucidique, à la prolifération et/ou à la différentiation cellulaire et/ou au vieillissement cutané ou du système nerveux central, les composés de formule (I) peuvent éventuellement inclure ceux de formule (I) dans laquelle X₂ représente un atome d'hydrogène et X₁ représente - G1R1 où G1 représente un atome d'oxygène et R1 représente CH2COOH. De préférence, ces composés sont toutefois exclus.

Dans le cas où la composition selon l'invention est destinée au traitement ou à la prophylaxie d'une pathologie liée à l'inflammation, à la neurodégénérescence, à la prolifération et/ou à la différentiation cellulaire et/ou au vieillissement cutané ou du système nerveux central et plus particulièrement d'une ou plusieurs allergies, de l'asthme, de l'eczéma, du psoriasis, des démangeaisons, de la maladie d'Alzheimer, de la maladie de Parkinson ou de la carcinogenèse, les composés de formule (1) peuvent éventuellement inclure ceux de formule (I) dans laquelle:
- X₁, X₂, X₃ et X₅ représentent simultanément un atome d'hydrogène, X₆ représente un atome d'oxygène et X₄ représente un groupement de formule -O-CR₈R₉-COOR₁₀, avec R₈ et R₉, identiques ou différents, représentent un radical alkyle de C₁ à C2 (comprenant un ou deux atomes de carbone), et R₁₀ représente un atome d'hydrogène ou un radical alkyle de C1 à C7, ou
- X₂, X₃ et X₅ représentent simultanément un atome d'hydrogène, X₁ représente un atome d'halogène ou un radical R1 ou -G1R1, où R1 représente un radical alkyle non substitué de C1 à C2 et G1 représente un atome d'oxygène, X₆ représente un atome d'oxygène et X₄ représente un groupement de formule -O-CR₁₁R₁₂-COOR₁₀, avec R₁₁ et R₁₂, identiques ou différents, représentent un atome d'hydrogène ou un radical alkyle de C1 à C2, et R₁₀ représente un atome d'hydrogène ou un radical alkyle de C1 à C7 (comprenant de un à sept atomes de carbone).

L'invention concerne également une méthode de traitement des pathologies liées à l'inflammation, à la neurodégénérescence, aux dérèglements du métabolisme lipidique et/ou glucidique, à la prolifération et/ou à la différentiation cellulaire et/ou au vieillissement cutané ou du système nerveux central et plus particulièrement d'une ou plusieurs allergies, de l'asthme, de l'eczéma, du psoriasis, des démangeaisons, de la maladie d'Alzheimer, de la maladie de Parkinson, du diabète, de l'athérosclérose, de l'obésité, de la carcinogenèse, comprenant l'administration à un sujet, notamment humain, d'une dose efficace d'un composé de formule générale (I) ou d'une composition pharmaceutique telle que définie ci-avant.

Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc.. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Les composés ou compositions selon l'invention peuvent être administrés de différentes manières et sous différentes formes. Ainsi, ils peuvent être administrés par voie orale ou systémique, comme par exemple par voie intraveineuse, intra-musculaire, sous-cutanée, trans-dermique, intra-artérielle, etc.. Pour les injections, les composés sont généralement conditionnés sous forme de suspensions liquides, qui peuvent être injectées au moyen de seringues ou de perfusions, par exemple. Il est entendu que le débit et/ou la dose injectée peuvent être adaptés par l'homme du métier en fonction du patient, de la pathologie, du mode d'administration, etc.. Typiquement, les composés sont administrés à des doses pouvant varier entre 1 µg et 2 g /administration, préférentiellement de 0,1 mg à 1 g/administration. Les administrations peuvent être quotidiennes ou répétées plusieurs fois par jour, le cas échéant. D'autre part, les compositions selon l'invention peuvent comprendre, en outre, d'autres agents ou principes actifs.

D'autres aspects et avantages de la présente invention apparaîtront à la lecture des exemples qui suivent, qui doivent être considérés comme illustratifs et non limitatifs.

### LEGENDES DES FIGURES

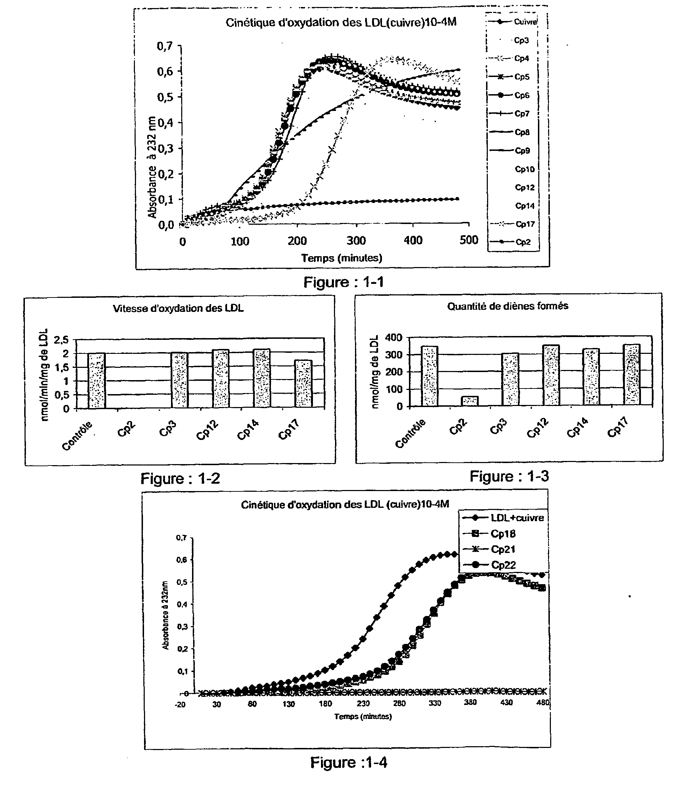

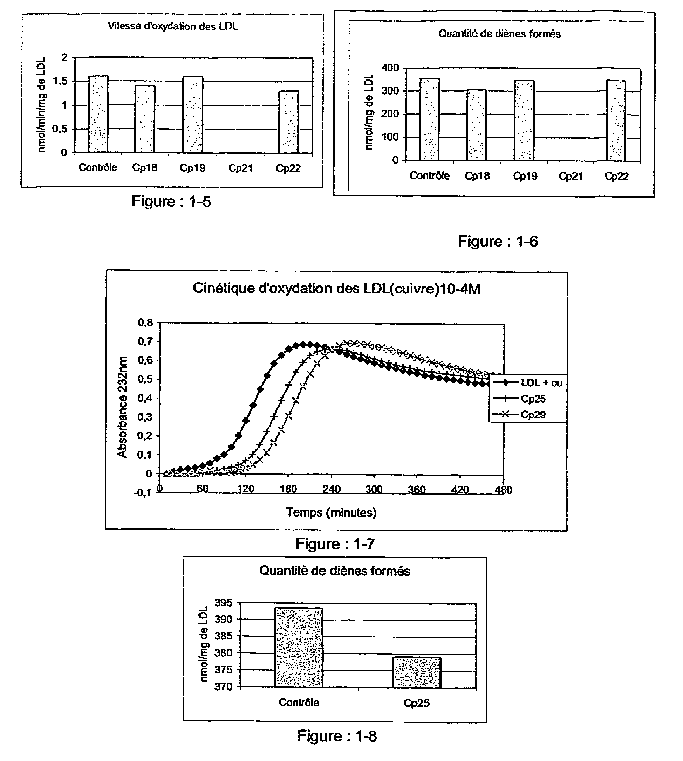

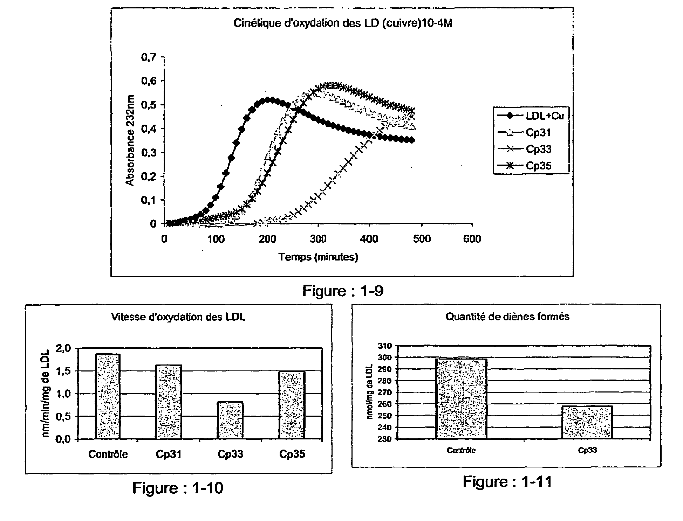

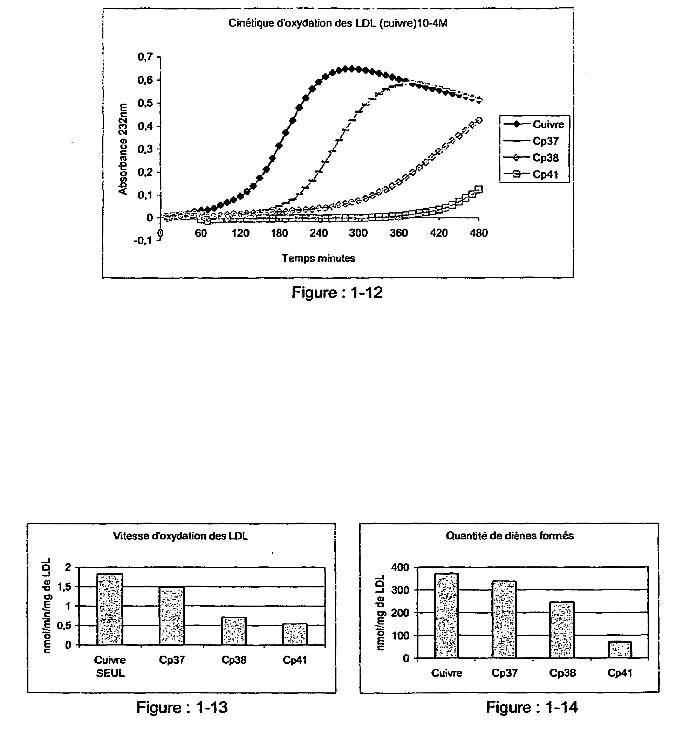

Figure 1-1, 1-2, 1-3 : Evaluation des propriétés antioxydantes des composé 2, composé 3, composé 12, composé 14 et composé 17 sur l'oxydation des LDL par le cuivre (Cu) :
Sur la figure 1-1 sont représentés les résultats de l'expérience mesurant la formation de diènes conjugués en fonction du temps. On peut observer-que l'incubation des LDL avec les composés testés à la concentration de 10⁻⁴M retarde la formation de diènes conjugués. La Lag-Phase est de 111 minutes pour le cuivre seul alors que ce délai d'apparition des diènes conjugués est respectivement de 132, 145, 134 et 203 minutes lorsque les LDL sont incubées avec les composé 3, composé 12, composé 14, composé 17. La Lag-Phase est supérieure à 480 minutes dans le cas où les LDL sont incubées avec le composé 2. Ce retard de formation de diènes conjugués est caractéristique de produits antioxydants.
La figure 1-2 représente la vitesse de formation des diènes en fonction des différents traitements. L'incubation des composés avec les LDL en présence de cuivre ralentit la vitesse de formation des diènes conjugués. La vitesse de formation des diènes conjugués est de 2 nmol/min/mg de LDL avec le cuivre seul, elle est de 1, 7 nmot/min/mg de LDL lorsque les LDL sont incubées en présence du composé 17 à 10⁻⁴M, cette vitesse est non déterminée avec le composé 2 à 10-⁴M (non mesurable car trop faible),
La Figure 1-3 représente la quantité maximum de diènes conjugués formés au cours du temps. L'incubation des LDL avec le cuivre entraîne la formation 348 nmol/mg de LDL de diènes conjugués, l'incubation avec le composé 2 à 10⁻⁴M diminue de 84% la formation de diènes conjugués (54,4 nmol/mg de LDL). Cette concentration est respectivement de 303 nmol/mg de LDL et 327 nmol/mg de LDL en présence des composés 3 et 17.

Figure 1-4, 1-5, 1-6 : Evaluation des propriétés antioxydantes des composé 18, composé 19, composé 21 et composé 22 sur l'oxydation des LDL par le cuivre (Cu):
Sur la figure 1-4, on peut observer que l'incubation des LDL avec les composés testés à la concentration de 10⁻⁴M retarde la formation de diènes conjugués. La Lag-Phase est de 178 minutes pour le cuivre seul alors que ce délai d'apparition des diènes conjugués est respectivement de 241, 182 et 241 minutes (de la mesure expérimentale) lorsque les LDL sont incubées avec les composé 18, composé 19 ou le composé 22. Elle est supérieure à 480 minutes dans le cas où les LDL sont incubées avec le composé 21. Ce retard de formation de diènes conjugués est caractéristique de produits antioxydants.
La figure 1-5 représente la vitesse de formation des diènes en fonction des différents traitements. La vitesse de formation des diènes conjugués est de 1,6 nmol/min/mg de LDL avec le cuivre seul, elle est de 1,4 nmol/min/mg de LDL lorsque les LDL sont incubées en présence des composés 18 à 10⁻⁴M et 1,3 nmol/min/mg de LDL lorsque les LDL sont incubées en présence des composés 22, cette vitesse est non déterminée avec le composé 21 à 10⁻⁴M (non mesurable car trop faible).
La Figure 1-6 représente la quantité maximum de diènes conjugués formées au cours du temps. L'incubation des LDL avec le cuivre entraîne la formation de 353 nmol/mg de LDL de diènes conjugués. L'incubation avec le composé 21 à 10⁻⁴M inhibe la formation de diènes conjugués. En présence des composés 18, 19 et 22, elle est respectivement de 305 nmol/mg de LDL, 345 nmol/mg de LDL et 345 nmol/mg de LDL.

Figure 1-7, 1-8 : Evaluation des propriétés antioxydantes des composé 25 et composé 29 sur l'oxydation des LDL par le cuivre (Cu)
Sur la figure 1-7 sont représentés les résultats de l'expérience mesurant la formation de diènes conjugués en fonction du temps. On peut observer que l'incubation des LDL avec les composés testés à la concentration de 10⁻⁴M retarde la formation de diènes conjugués. La Lag-Phase est de 82 minutes pour le cuivre seul alors que ce délai d'apparition des diènes conjugués est respectivement de 120 et 135 minutes (de la mesure expérimentale) lorsque les LDL sont incubées avec les composé 25 et avec le composé 29.
La Figure 1-8 représente la quantité maximum de diènes conjugués formée au cours du temps. L'incubation des LDL avec le cuivre entraîne la formation de 393 nmol/mg de LDL de diènes conjugués. En présence du composé 25 elle est de 378 nmol/mg de LDL.

Figures 1-9, 1-10, 1-11 : Evaluation des propriétés antioxydantes des composé 31, composé 33 et composé 35 sur l'oxydation des LDL par le cuivre (Cu) :
Sur la figure 1-9 sont représentés les résultats de l'expérience mesurant la formation de diènes conjugués en fonction du temps. On peut observer que l'incubation des LDL avec les composés testés à la concentration de 10⁻⁴M retarde la formation de diènes conjugués. La Lag-Phase est de 80 minutes pour le cuivre seul alors que ce délai d'apparition des diènes conjugués est respectivement de 139, 247 et 149 minutes (de la mesure expérimentale) lorsque les LDL sont incubées avec les composé 31, composé 33 et le composé 35. Ce retard de formation de diènes conjugués est caractéristique de produits antioxydants.
La figure 1-10 représente la vitesse de formation des diènes en fonction des différents traitements. L'incubation des composés avec les LDL en présence de cuivre ralentit la vitesse de formation des diènes conjugués. La vitesse de formation des diènes conjugués est de 1,9 nmol/min/mg de LDL avec le cuivre seul, elle est de 1,6 nmot/min/mg de LDL lorsque les LDL sont incubées en présence des composés 31 à 10⁻⁴M et 0,8 nmol/min/mg de LDL lorsque les LDL sont incubées en présence du composé 33 et 1,5 nmol/min/mg de LDL lorsque les LDL sont incubées en présence du composé 35.
La Figure 1-11 représente la quantité maximum de diènes conjugués formée au cours du temps. L'incubation des LDL avec le cuivre entraîne la formation 298 nmol/mg de LDL de diènes conjugués, en présence du composé 33 elle est de 257 nmol/mg de LDL.

Figure 1-12, 1-13, 1-14 : Evaluation des propriétés antioxydantes des composé 37, composé 38 et composé 41 sur l'oxydation des LDL par le cuivre (Cu) :
Sur la figure 1-12 sont représentés les résultats de l'expérience mesurant la formation de diènes conjugués en fonction du temps. On peut observer que l'incubation des LDL avec les composés testés à la concentration de 10⁻⁴M retarde la formation de diènes conjugués. La Lag-Phase est de 120 minutes pour le cuivre seul alors que ce délai d'apparition des diènes conjuguées est respectivement de 196, 284 et 411 minutes (de la mesure expérimentale) lorsque les LDL sont incubées avec les composé 37, composé 38 et le composé 41.
La figure 1-13 représente la vitesse de formation des diènes en fonction des différents traitements. L'incubation des composés avec les LDL en présence de cuivre ralentit la vitesse de formation des diènes conjugués. La vitesse de formation des diènes conjugués est de 1,8 nmol/min/mg de LDL avec le cuivre seul, elle est de 1,49 nmol/min/mg de LDL lorsque les LDL sont incubées en présence des composés 37 à 10⁻⁴M et 0,71 de LDL nmol/min/mg lorsque les LDL sont incubées en présence des composés 38 et 0,54 nmol/min/mg de LDL lorsque les LDL sont incubées en présence des composés 41.
La Figure 1-14 représente la quantité maximum de diènes conjugués formée au cours du temps. L'incubation des LDL avec le cuivre entraîne la formation de 372 nmol/mg de LDL de diènes conjugués. En présence des composés 37, 38 et 41, elle est respectivement de 338 nmol/mg de LDL, 244 nmol/mg de LDL et 71 nmol/mg de LDL.

Le retard de formation de diènes conjugués, la diminution de la vitesse de formation des diènes et la diminution de la quantité totale de diènes formés sont caractéristiques de produits antioxydants.

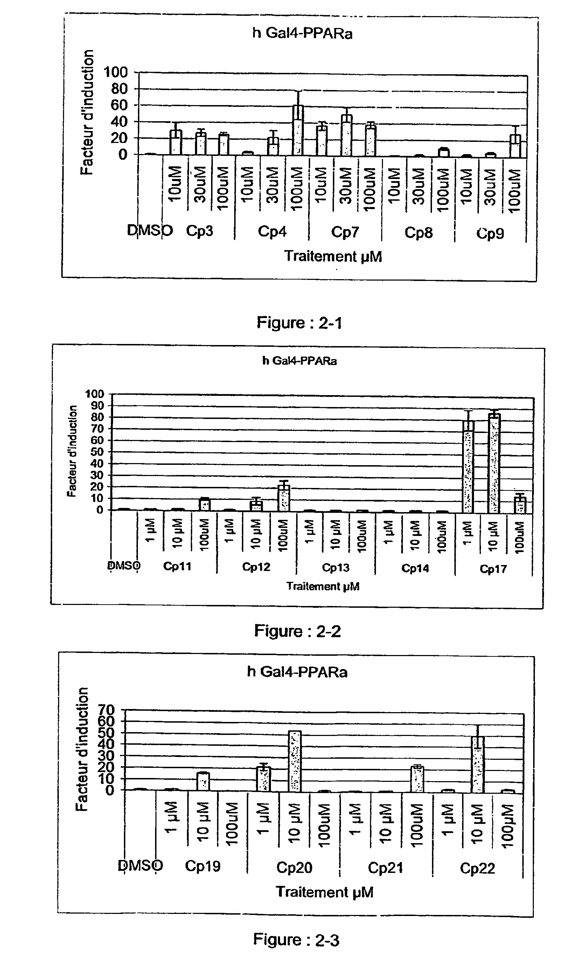

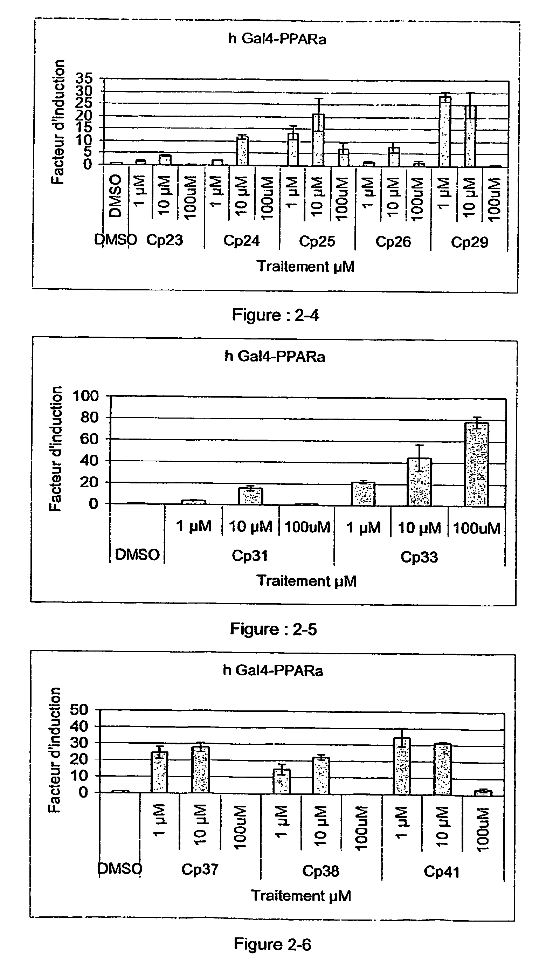

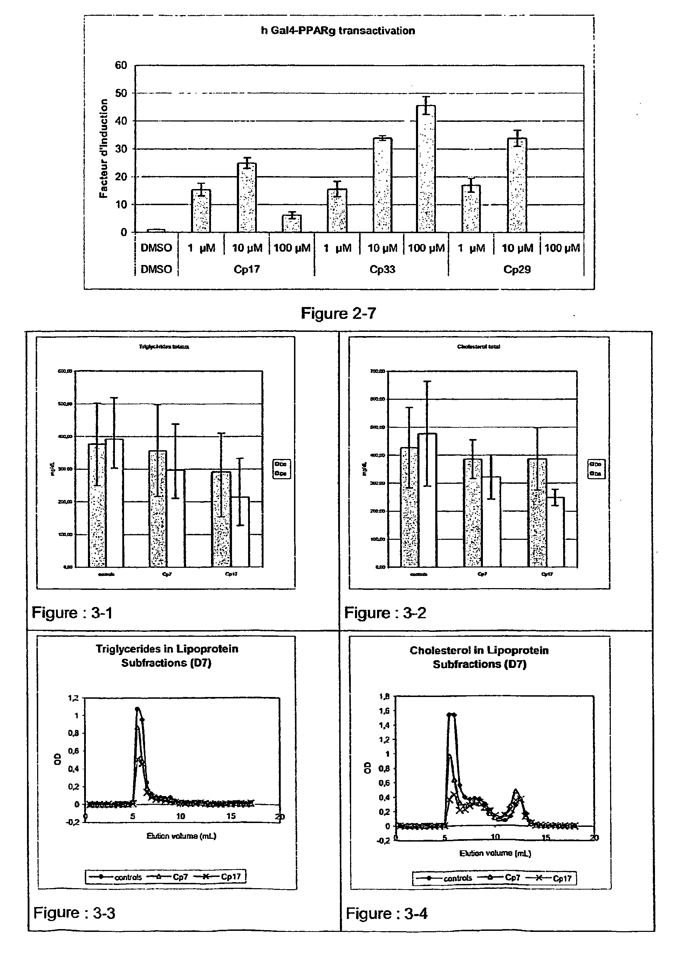

Figures 2-1, 2-2, 2-3, 2-4, 2-5, 2-6 : Evaluation des propriétés d'agoniste PPARα des composés selon l'invention avec le système de transactivation PPARα/Gal4.

Les cellules RK13 sont incubées avec différents composés aux concentrations suivantes 10, 30 et 100 µM ou 1,10 et 100 µM pendant 24h. Les résultats sont représentés par le facteur d'induction (signal luminescent par rapport aux cellules non traitées) en fonction des différents traitements. Plus le facteur d'induction est élevé meilleure est la propriété d'agoniste pour PPARα. Figure 2-1 :
Les résultats montrent les facteurs d'induction des composés composé 3, composé 4, composé 7, composé 8, composé 9. Les valeurs de ces facteurs d'induction sont notées dans le tableau 2-1.

**Tableau 2-1**

| Composé | Concentration | Facteur d'induction |
|---|---|---|
| Cp3 | 10µM | 30,12 |
| | 30µM | 27,27 |
| | 100µM | 25,84 |
| Cp4 | 10µM | 3,99 |
| | 30µM | 22,15 |
| | 100µM | 61,07 |
| Cp7 | 10µM | 36,48 |
| | 30µM | 50,37 |
| | 100µM | 37,84 |
| Cp8 | 10 µM | 0,62 |
| | 30µM | 1,27 |
| | 100µM | 9,98 |
| Cp9 | 10µM | 2,11 |
| | 30µM | 5,00 |
| | 100µM | 28,19 |

Les résultats montrent que le composé 3 permet l'induction d'un facteur maximal de 27 à la concentration de 30 µM, le composé 4 a un facteur d'induction maximal de 60 à 100 µM, de 22 à 30 µM et de 4 à 10 µM. Le composé 7 a un facteur d'induction maximal de 50 à 100µM. Le composé 8 active le système avec un facteur d'induction maximal de 10 pour la concentration de100 µM. Le composé 9 possède un facteur d'induction de 28 pour la plus forte concentration 100 µM.

Figure 2-2 :
Les résultats montrent les facteurs d'induction des composés composé 11, composé 12, composé 13, composé 14, composé 17. Les valeurs de ces facteurs d'induction sont notées dans le tableau 2-2.

**Tableau : 2-2**

| Composé | Concentration | Facteur d'induction |
|---|---|---|
| Cp11 | 1 µM | 1,20 |
| | 10 µM | 1,39 |
| | 100µM | 10,19 |
| Cp12 | 1 µM | 1,12 |
| | 10 µM | 8,45 |
| | 100µM | 22,54 |
| Cp13 | 1 µM | 1,20 |
| | 10 µM | 1,10 |
| | 100µM | 1,5 |
| Cp14 | 1 µM | 1,25 |
| | 10 µM | 1,36 |
| | 100µM | 1,38 |
| Cp17 | 1 µM | 79,76 |
| | 10 µM | 85,69 |
| | 100µM | 13,80 |

Les résultats montrent que le composé 11 permet l'induction d'un facteur maximal de 10 à 100 µM, le composé 12 a un facteur d'induction maximal de 22 à 100 µM, de 8 à 30 µM et de 1 à 10 µM. Les composés 13 et 14 ont des facteurs d'induction compris entre 1,1 et 1,5 pour les différentes concentrations testées. Le composé 17 active le système avec un facteur d'induction maximal de 85 pour la concentration de 10 µM et un facteur d'induction minimal de 13,8 pour la concentration de 100µM.

Figure : 2-3
Les résultats montrent les facteurs d'induction des composés composé 19, composé 20, composé 21, composé 22. Les valeurs de ces facteurs d'induction sont notées dans le tableau 2-3.

**Tableau: 2-3**

| Composé | Concentration | Facteur d'induction |
|---|---|---|
| Cp19 | 1 µM | 1,20 |
| | 10 µM | 15,62 |
| | 100µM | 0,07 |
| Cp20 | 1 µM | 21,50 |
| | 10 µM | 53,45 |
| | 100µM | 1,22 |
| Cp21 | 1 µM | 0,78 |
| | 10 µM | 1,10 |
| | 100µM | 22,80 |
| Cp22 | 1 µM | 2,40 |
| | 10 µM | 49,49 |
| | 100µM | 2,73 |

Les résultats montrent que le composé 19 permet l'induction d'un facteur maximal de 15,6 à 10 µM, le composé 20 a un facteur d'induction maximal de 53 à 10 µM. Le composé 21 a des facteurs d'induction compris entre 0,8 et 22 pour les différentes concentrations testées. Le composé 22 active le système avec un facteur d'induction maximal de 50 pour la concentrations de10 µM.
Figure : 2-4
Les résultats montrent les facteurs d'induction des composés 23, 24, 25, 26 et 29. Les valeurs de ces facteurs d'induction sont notées dans le tableau 2-4.

**Tableau 2-4**

| Composé | Concentration | Facteur d'induction |
|---|---|---|
| Cp23 | 1 µM | 1,55 |
| | 10 µM | 3,67 |
| | 100µM | 0,12 |
| Cp24 | 1 µM | 2,06 |
| | 10 µM | 11,62 |
| | 100uM | 0,00 |
| Cp25 | 1 µM | 13,48 |
| | 10 µM | 21,03 |
| | 100uM | 7,01 |
| Cp26 | 1 µM | 1,75 |
| | 10 µM | 7,85 |
| | 100uM | 1,08 |
| Cp29 | 1 µM | 28,36 |
| | 10 µM | 25,26 |
| | 100uM | 0,27 |

Le composé 23 a un facteur d'induction maximal de 3,6 à 10 µM, le composé 24 a un facteur d'induction maximal de 11 à 10 µM. Le composé 25 active le système avec des facteurs compris entre 7 et 21 selon les concentrations testées. Le composé 26 a un facteur d'induction maximal de 7,8 pour la concentrations de10 µM. Le composé 29 a des facteurs d'induction de 28 et 25 pour les concentrations de 1 et 10µM respectivement.

Figure 2-5 :
Les résultats montrent les facteurs d'induction des composés 31 et 33. Les valeurs de ces facteurs d'induction sont notées dans le tableau 2-5.

**Tableau : 2-5**

| Composé | Concentration | Facteur d'induction |
|---|---|---|
| Cp31 | 1 µM | 3,77 |
| | 10 µM | 15,52 |
| | 100µM | 1,21 |
| Cp33 | 1 µM | 22,05 |
| | 10 µM | 44,52 |
| | 100uM | 77,62 |

Le composé 31 active le système avec un facteur d'induction de 15,5 à la concentration de 10µM. Les facteurs d'induction observés pour le composé 33 sont de 22, 44 et 77 pour les concentration de 1,10 et 100µM respectivement.

Figure : 2-6
Les résultats montrent les facteurs d'induction des composé 37, composé 38, composé 41. Les valeurs de ces facteurs d'induction sont notées dans le tableau 2-6.

**Tableau : 2-6**

| Composé | Concentration | Facteur d'induction |
|---|---|---|
| Cp37 | 1 µM | 24,55 |
| | 10 µM | 27,83 |
| | 100µM | 0,02 |
| Cp38 | 1 µM | 14,70 |
| | 10µM | 22,22 |
| | 100uM | 0,311 |
| Cp41 | 1 µM | 34,61 |
| | 10 µM | 31,18 |
| | 100µM | 3,39 |

Les facteurs d'induction maximum pour les composés 37, 38 et 41 sont respectivement de 27, 22 et 31, ils sont observés pour la concentration de 10µM.

Ces résultats montrent que les composés selon l'invention testés possèdent la propriété de ligand vis-à-vis de PPARα et permettent aussi son activation au niveau transcriptionnel.
Figure 2-7 : Evaluation des propriétés d'agoniste PPARγ des composés selon l'invention avec le système de transactivation PPARγ/Gal4.

Les cellules RK13 sont incubées avec différents composés aux concentrations suivantes 1, 10 et 100 µM pendant 24h. Les résultats sont représentés par le facteur d'induction (signal luminescent par rapport aux cellules non traitées) en fonction des différents traitements. Plus le facteur d'induction est élevé meilleure est la propriété d'agoniste pour PPARγ.

Les résultats de la figure montrent les facteurs d'induction des composé 17, composé 33, et composé 29. Les valeurs de ces facteurs d'induction sont notées dans le tableau 2-7.

**Tableau 2-7 :**

| | Composé | Facteur d'induction |
|---|---|---|
| Cp17 | 1 µM | 15,37 |
| | 10 µM | 24,92 |
| | 100 µM | 6,13 |
| Cp33 | 1 µM | 15,65 |
| | 10 µM | 33,90 |
| | 100 µM | 45,58 |
| Cp29 | 1 µM | 17,05 |
| | 10 µM | 33,89 |
| | 100 µM | 0,01 |

Les résultats montrent que le composé 17 permet l'induction d'un facteur maximal de 25 à 10 µM. Le composé 33 a un facteur d'induction maximal de 45,6 à 100 µM et le composé 29 de 33,9 à la concentration de 10µM.

Ces résultats montrent que les composés selon l'invention testés possèdent la propriété de ligand vis-à-vis de PPARγ et permettent aussi son activation au niveau transcriptionnel.

Figures : 3-1, 3-2, 3-3, 3-4 : Evaluation de l'effet du composé 7 et du composé 17 sur le métabolisme des triglycérides et du cholestérol.

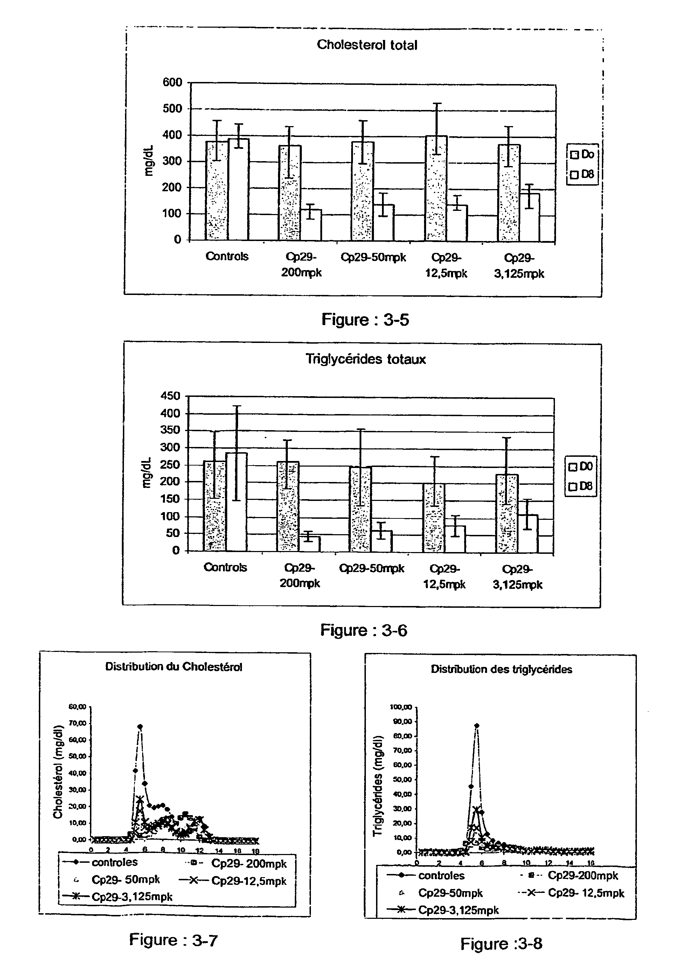

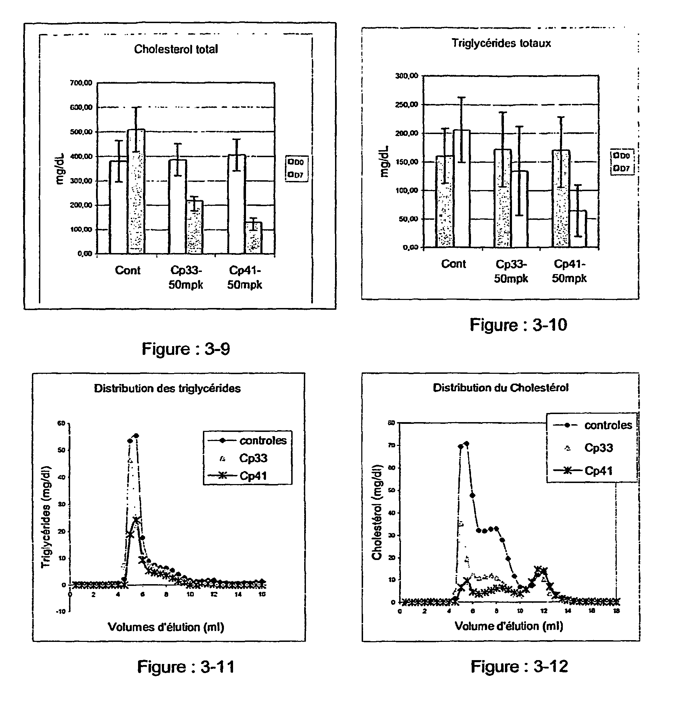

Sur les figures 3-1, 3-2, 3-3 et 3-4 sont représentés les effets du traitement avec les composés 7 et 17 sur le métabolisme des triglycérides et du cholestérol chez les souris trangéniques Apo E2/E/2. Les animaux ont été traités par gavage avec 200mg par kg de chacun des composés pendant 7 jours.
Les figures 3-1 et 3-2 montrent la diminution des taux plasmatiques de triglycérides et de cholestérol induite par les composés 7 et 17.
Les figures 3-3 et 3-4 montrent la distribution des triglycériques et du cholestérol dans les lipoparticules évaluée par chromatographie d'exclusion. On observe une distribution typique des triglycérides et du cholestérol principalement localisée dans les lipoparticules de grande taille. On observe également une diminution des triglycerides et du cholesterol dans cette classe de lipoparticules induite par le traitement avec les composés 7 et 17.

Figures 3-5, 3-6, 3-7 et 3-8 :
Les figures 3-5, 3-6, 3-7 et 3-8 illustrent l'effet du composé 29 selon l'invention sur le métabolisme des triglycérides et du cholestérol chez les souris transgéniques ApoE2/E2. Les animaux ont été traités avec le composé 29 aux doses suivantes : 200, 50, 12,5 et 3,15 mg par kg par jour pendant 8 jours.
Les figures 3-5 et 3-6 montrent la diminution dose-dépendante des taux plasmatiques de triglycérides et de cholestérol avec une diminution croissante selon la quantité de composé 29 administrée.
Les figures 3-7 et 3-8 montrent la distribution des triglycérides et du cholestérol dans les lipoparticules évaluée par chromatographie d'exclusion. On observe une distribution typique des triglycérides et du cholestérol principalement localisée dans les lipoparticules de grande taille. On observe également une diminution des triglycérides et du cholestérol dans cette classe de lipoparticules.

Figures 3-9, 3-10, 3-11 et 3-12 :
Les figures 3-9 et 3-10 illustrent l'effet du composé 33 selon l'invention et du composé 41 selon l'invention sur le métabolisme des triglycérides et du cholestérol chez les souris transgéniques Apo E2/E2. Les animaux ont été traités avec les différents composés administrés à la dose de 50 mg par kg par jour pendant 8 jours des différents composés. Les figures 5-1 et 5-2 montrent la diminution des taux plasmatiques de triglycérides et de cholestérol induite par les composés 33 et 41.
Les figures 3-11 et 3-12 montrent la distribution des triglycérides et du cholestérol dans les lipoparticules évaluée par chromatographie d'exclusion. On observe une distribution typique des triglycérides et du cholestérol principalement localisée dans les lipoparticules de grande taille et une diminution des triglycérides et du cholestérol dans cette classe de lipoparticules sous l'effet des composés 33 et 41.

D'autres aspects et avantages de la présente invention apparaîtront à la lecture des exemples qui suivent, qui doivent être considérés comme illustratifs et non limitatifs.

### EXEMPLES

Les composés de l'invention sont préparés selon les méthodes générales décrites ci-dessous.

### Description des méthodes générales de synthèse selon l'invention :

### Synthèse de 1,3-diphénylprop-2-en-1-ones :

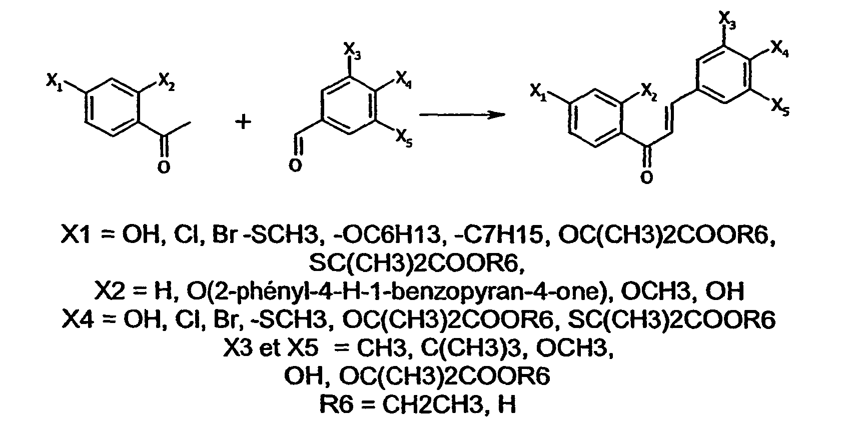

### Méthode générale 1 :

### Synthèse de 1,3-diphénylprop-2-en-1-ones en milieu acide :

La cétone (1 éq) et l'aldéhyde (1 éq) sont solubilisés dans une solution d'éthanol saturée d'acide chlorhydrique gazeux. L'ensemble est agité à température ambiante pendant 6h puis le solvant est éliminé par évaporation sous pression réduite. La 1,3-diphénylprop-2-èn-1-one est purifiée par chromatographie sur gel de silice.

### Méthode générale 2 :

### Synthèse de 1,3-diphénylprop-2-en-1-ones en milieu basique :

La cétone (1 éq) et l'aldéhyde (1 éq) sont solubilisés dans une solution hydroalcoolique d'hydroxyde de sodium (20 éq). L'ensemble est agité 18 heures à température ambiante. Le milieu est acidifié (pH = 2) avec de l'acide chlorhydrique.
La 1,3-diphénylprop-2-èn-1-one est obtenue par précipitation ou extraction solide liquide après évaporation du milieu réactionnel. Elle est purifiée par chromatographie sur gel de silice ou par recristallisation.

### Méthode générale 3 :

### Synthèse de 1,3-diphénylprop-2-en-1-ones substituées en présence d'éthylate de sodium :

Le sodium (1 éq) est dissout dans l'éthanol absolu. La cétone (1 éq) et l'aldéhyde (1 éq) sont ajoutés. L'ensemble est maintenu sous agitation à température ambiante pendant 12 heures puis une solution de soude 2N (5 éq) est ajoutée. L'ensemble est maintenu à 100°C pendant 12 heures. Le milieu réactionnel est acidifié par addition d'un solution aqueuse d'acide chlorhydrique 6N. Le solvant est éliminé par évaporation sous pression réduite. Le résidu est purifié par chromatographie sur gel de silice ou par recristallisation.

### O-Alkylation de phénols et S-alkylation de thiophénols

### Méthode générale 4 :

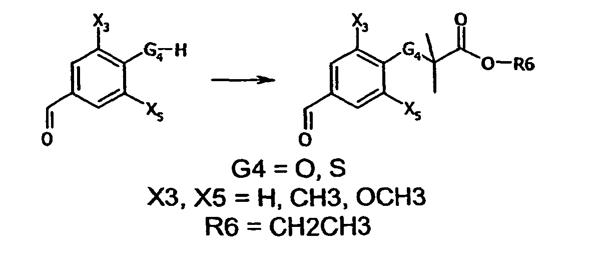

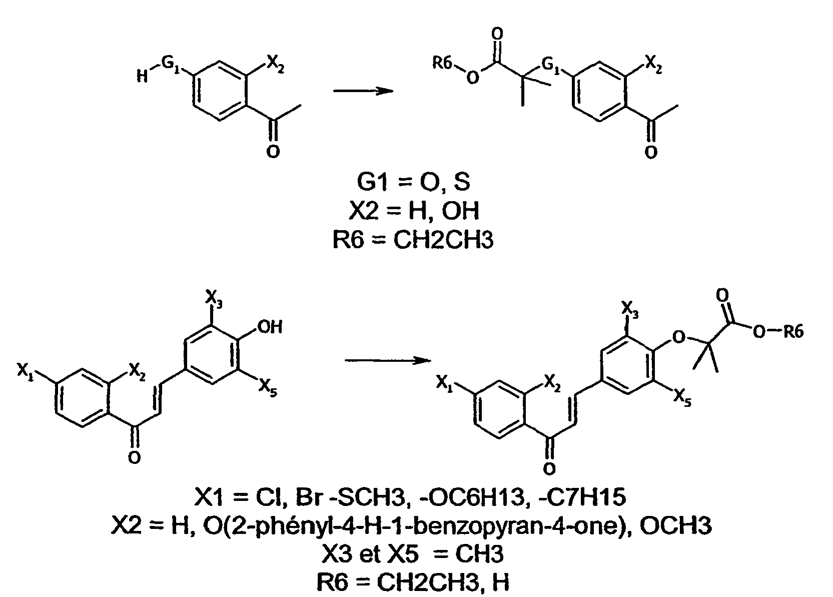

Le phénol (1 éq) est solubilisé dans l'acétonitrile, le dérivé halogéné (1 à 10 éq), puis le carbonate de potassium (5 éq) sont ajoutés. Le milieu réactionnel est maintenu environ 10 heures sous vive agitation à reflux. Les sels sont éliminés par filtration, le solvant et l'excès de réactif sont éliminés par évaporation sous pression réduite, le produit attendu est purifié par chromatographie sur gel de silice.

### Acidolyse d'esters tertiobutyliques :

### Méthode générale 5 :

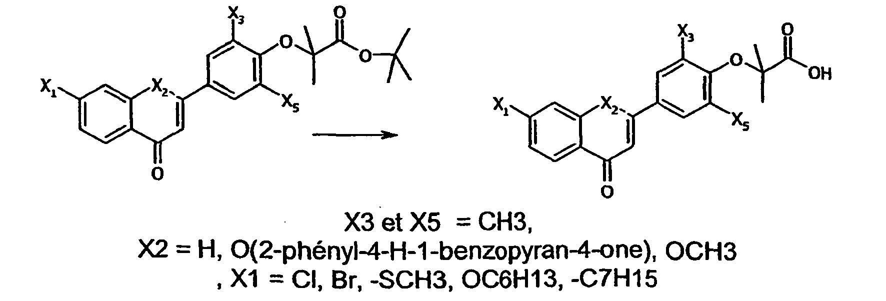

L'ester tertiobutylique (1 éq) est solubilisé dans du dichlorométhane, l'acide trifluoroacétique (10 éq) est additionné, l'ensemble est maintenu 12 heures sous agitation à température ambiante. Le produit formé est purifié par chromatographie sur gel de silice ou par recristallisation.

### Synthèse des matières premières servant à la synthèse des composés selon l'invention :

### Matière première 1 :

### 2'-Hydroxy-4'-(éthoxycarbonyldiméthylméthoxy)acétophénone :

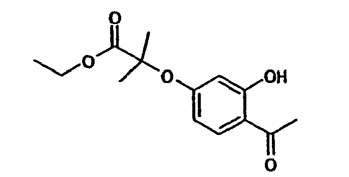

Ce composé est synthétisé à partir de 2',4'-dihydroxyacétophénone et de bromoisobutyrate d'éthyle (1 éq) selon la méthode générale 4 précédemment décrite.
Purification par chromatographie sur gel de silice (élution : cyclohexane-acétate d'éthyle : 9-1).
RMN 1H CDCl₃ δppm : 1.25 (t, J = 7.17 Hz, 3H), 1.67 (s, 6H), 2.56 (s, 3H), 4.24 (q, J = 7.17, 2H), 6.27 (d, J = 2.55 Hz, 1H), 6.37 (dd, J = 2.55Hz, J = 8.72 Hz, 1H), 7.62 (d, J = 8.72, 1H), 12,6 (signal, 1H).
Bibliographie: Brevet US n° 3,629,290 (1970), Fisons Pharmaceutical

### Matière première 2 :

### Acétate de 3-chlorophényle

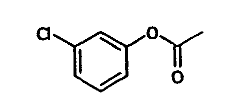

Le 3-chlorophénol est solubilisé dans le dichlorométhane. La triéthylamine (1éq) et l'anhydride acétique (2 éq) sont ajoutés. L'ensemble est agité 5 heures à température ambiante. Le solvant est éliminé par évaporation sous pression réduite. Le résidu d'évaporation est repris par du dichlorométhane, séché sur sulfate de magnésium puis le solvant est éliminé par évaporation sous pression réduite. Purification par chromatographie sur gel de silice (élution : cyclohexane-acétate d'éthyle : 95-5).
RMN 1H CDCl₃ δppm: 2.29 (s, 3H), 6.99-7.33 (m, 4H)

### Matière première 3:

### 4'-Chloro-2'-hydroxyacétophénone

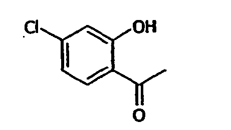

L'acétate de 3-chlorophényle (matière première 2) est mélangé au chlorure d'aluminium (3 éq). Le mélange est chauffé 1 heure à 200°C. Le milieu réactionnel est ramené à température ambiante puis versé dans la glace. La phase aqueuse est extraite par du chlorure de méthylène qui est séché sur sulfate de magnésium puis évaporé sous pression réduite.
Purification par chromatographie sur gel de silice (élution : cyclohexane-acétate d'éthyle : 95-5).
RMN 1H CDCl₃ δppm: 3.41 (s, 3H), 6.81 (dd, J = 8.82Hz, J =1.47Hz, 1H), 6.91 (d, J = 1.47Hz, 1H), 7.60 (d, 8.82Hz, 1H), 12.33 (s, 1H)
Bibliographie : Chen et al, J Chem Soc, 1958, 146-148.

### Matière première 4 :

### 4-Ethyloxycarbonyldiméthylméthyloxybenzaldéhyde

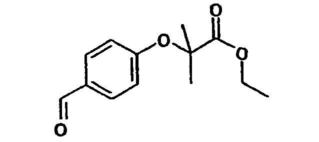

Ce composé est synthétisé à partir de 4-hydroxyabenzaldéhyde et de bromoisobutyrate d'éthyle selon la méthode générale 4 précédemment décrite.

Purification par chromatographie sur gel de silice (élution : cyclohexane-acétate d'éthyle : 9-1).
RMN 1H CDCl₃ δ ppm : 1.20(t, = 6.96Hz, 3H), 1.67(s, 6H), 4.21 (q, J = 6.96Hz, 2H), 6.89(d, J = 8.91 Hz, 2H),7.79 (d, J = 8.94Hz, 2H), 9.87(S, 1H).

### Matière première 5:

### 3,5-diméthyloxy-4-éthyloxycarbonyldiméthylméthyloxybenzaldéhyde

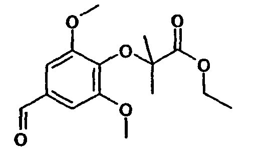

Ce composé est synthétisé à partir de 3,5-diméthyloxy-4-hydroxyabenzaldéhyde et de bromoisobutyrate d'éthyle selon la méthode générale 4 précédemment décrite.
Purification par chromatographie sur gel de silice (élution : cyclohexane-acétate d'éthyle : 8-2).
RMN 1H CDCl₃ δ ppm : 1.33(t, J = 7.29Hz, 3H), 1.50(s, 6H), 3.84(s, 6H), 4.27(q, J = 7.29Hz, 2H), 7.08(s, 2H), 9.86(s, 1H)

### Matière première 6 :

### 3,5-diméthyl-4-éthyloxycarbonyldiméthylméthyloxybenzaldéhyde

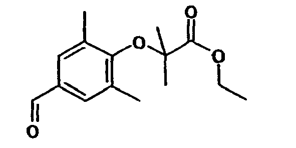

Ce composé est synthétisé à partir de 3,5-diméthyl-4-hydroxyabenzaldéhyde et de bromoisobutyrate d'éthyle selon la méthode générale 4 précédemment décrite.
Purification par chromatographie sur gel de silice (élution : cyclohexane-acétate d'éthyle : 95-5).
RMN 1H CDCl₃ δ ppm :1.37(t, J = 7.14Hz, 3H), 1.50(s, 6H), 2.29(s, 6H), 4.30(q, J = 7.14Hz, 2H), 7.54(s, 2H), 9.88 (s, 1H)

### Matière première 7 :

### 3-Ethyloxycarbonyldiméthylméthyloxybenzaldéhyde :

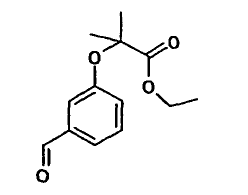

Ce composé est synthétisé à partir de 3-hydroxybenzaldéhyde et de bromoisobutyrate d'éthyle selon la méthode générale 4 précédemment décrite.
Purification par chromatographie sur gel de silice (élution : cyclohexane-acétate d'éthyle : 9-1).
RM N 1H CDCl₃ δ ppm : 1.24(t, J = 7.27Hz, 3H), 1.62(s, 6H), 4.25(q, J = 7.27Hz, 2H), 7.11(m, 1H), 7.31(m, 1H), 7.40(t, J = 8.19Hz, 1H), 7.49(m, 1H), 9.93(s, 1H).

### Matière première 8:

### 4-Ethyloxycarbonyldiméthylméthyl thiobenzaldéhyde

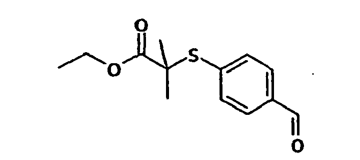

Le 4-méthylthiobenzaldéhyde (1 éq) est solubilisé dans du chlorure de méthylène, la solution est refroidie à 0°C . L'acide méta-chloro-perbenzoique (1.5 éq) est ajouté par petites fractions. L'avancement de la réaction est suivi par chromatographie sur couche mince. Un éventuel complément d'acide métachloroperbenzoique est ajouté de façon à obtenir la disparition totale du produit de départ. Le précipité formé est éliminé par filtration. De l'hydroxyde de calcium (1,5 éq) est ajouté. L'agitation est prolongée pendant 15 min. Le solide est éliminé par filtration, le filtrat est séché sur sulfate de magnésium puis le chlorure de méthylène est éliminé par évaporation sous pression réduite.
Le résidu d'évaporation est repris par de l'anhydride acétique, l'ensemble est chauffé 30 min à reflux puis évaporé à sec. Le résidu est repris par la solution méthanol/triéthylamine, agité 15 minutes à température ambiante puis les solvants sont éliminés par évaporation sous pression réduite. Le résidu huileux est repris par une solution aqueuse saturée de chlorure d'ammonium puis extrait par du chlorure de méthylène. La phase organique est séchée sur sulfate de magnésium puis évaporée sous pression réduite.
Le 4-mercaptobenzaldéhyde intermédiaire ainsi obtenu est utilisé sans autre purification. Il est alkylé selon la méthode générale 4 pour donner le 4'-éthyloxycarbonyldiméthylméthylthiobenzaldéhyde.
Purification par chromatographie sur gel de silice (élution : cyclohexane-acétate d'éthyle : 9-1).
RMN 1H CDCl₃ δ ppm : 1.22(t, J = 7.46Hz, 3H), 2.60(s, 6H), 4.15(q, J = 7.46Hz, 2H), 7.53(d, J = 8.38Hz, 2H), 7.88(d, J = 8.39, 2H), 9.99(s, 1H)
Bibliographie : Young NR, Gauthier J Y., Coombs w (1984). Tetrahedron Letters 25(17): 1753-1756

### Matière première 9 :

### 4'-Ethyloxycarbonyldiméthylméthyloxyacétophénone :

Ce composé est synthétisé à partir de 4'-hydroxyacétophénone et de bromoisobutyrate d'éthyle selon la méthode générale 4 précédemment décrite. 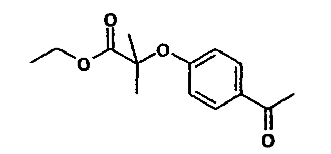 Purification par chromatographie sur gel de silice (élution : cyclohexane-acétate d'éthyle : 9-1).
RMN 1H CDCl₃ δ ppm : 1.17(t, J = 5.64Hz, 3H), 1.61(s, 6H), 2.50(s, 3H), 4.18(q, J = 5.64Hz, 2H), 6.78(d, J = 8.82Hz, 2H), 7.83(d, J = 8.81Hz, 2H).

### Matière première 10 :

### Acétate de 3-bromophényle

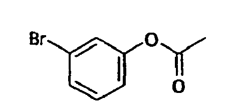

Le 3-bromophénol est solubilisé dans le dichlorométhane. La triéthylamine (1 éq) et l'anhydride acétique (2 éq) sont ajoutés. L'ensemble est agité 5 heures à température ambiante. Le solvant est éliminé par évaporation sous pression réduite. Le résidu d'évaporation est repris par du dichlorométhane puis séché sur sulfate de magnésium. Le solvant est éliminé par évaporation sous pression réduite.
Purification par chromatographie sur gel de silice (élution : cyclohexane-acétate d'éthyle : 95-5).
RMN 1H CDCl₃ δ ppm : 2.30(s, 3H), 7.0-7.4(m, 4H)

### Matière première 11 :

### 2'-hydroxy-4'-bromoacétophénone

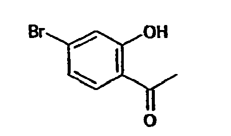

L'acétate de 3-bromophényle (matière première 10) est mélangé au chlorure d'aluminium (3 éq), le mélange est chauffé 1 heure à 200°C. Le milieu réactionnel est ramené à température ambiante puis versé dans la glace. La phase aqueuse est extraite par du chlorure de méthylène qui est séché sur sulfate de magnésium.
Purification par chromatographie sur gel de silice (élution : cyclohexane-acétate d'éthyle : 95-5).
RMN 1H CDCl₃ δ ppm : 2.59(s, 3H), 7.01(d, J = 8.5Hz, 1H), 7.13(s, 1H), 7.55(d, J = 8.5Hz, 1H), 12.33(s, 1H)

### Matière première 12 :

### 4'-Ethyloxycarbonyl diméthylméthylthiocétophénone

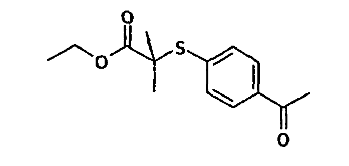

La 4'-méthylthioacétophénone est solubilisée dans du chlorure de méthylène, la solution est refroidie à 0°C . L'acide métachloroperbenzoique (1.5 éq) est ajouté par petites fractions. L'avancement de la réaction est suivi par chromatographie sur couche mince. Un éventuel complément d'acide métachloroperbenzoique est ajouté de façon à obtenir la disparition totale du produit de départ. Le précipité formé est éliminé par filtration. De l'hydroxyde de calcium (1,5 éq) est ajouté. L'agitation est prolongée pendant 15 min. Le solide est éliminé par filtration, le filtrat est séché sur sulfate de magnésium puis le chlorure de méthylène est éliminé par évaporation sous pression réduite.
Le résidu d'évaporation est repris par de l'anhydride acétique, l'ensemble est chauffé 30 min à reflux puis évaporé à sec. Le résidu est repris par la solution Méthanol/Tri éthylamine, agité 15 mn à température ambiante puis les solvants sont éliminés par évaporation sous pression réduite. Le résidu huileux est repris par une solution aqueuse saturée de chlorure d'ammonium puis extrait par du chlorure de méthylène. La phase organique est séchée sur sulfate de magnésium puis évaporée sous pression réduite.
La 4-mercaptoacétophénone intermédiaire ainsi obtenue est utilisée sans autre purification. Elle est alkylée selon la méthode générale 4 pour donner la 4-Ethyloxycarbonyldiméthylméthylthioacétophénone.
Purification par chromatographie sur gel de silice (élution : cyclohexane-acétate d'éthyle : 9-1).
Bibliographie : Young NR, Gauthier J Y., Coombs w (1984). Tetrahedron Letters 25(17): 1753-1756.
RMN 1H CDCl₃ δ ppm : 1.21(t, J = 7.32Hz, 3H), 1.51 (s, 6H), 2.59(s, 3H), 4.12(q, J = 7.32Hz, 2H), 7.51 (d, J = 8.40Hz, 2H), 7.79(d, J = 8.40Hz, 2H)

### Synthèse de composés intermédiaires servant à la synthèse des composés selon l'invention :

### Composé intermédiaire 1 :

### 1-[4-chlorophényl] -3-[3,5-diméthyl-4-hydroxyphényl]prop-2-èn-1-on

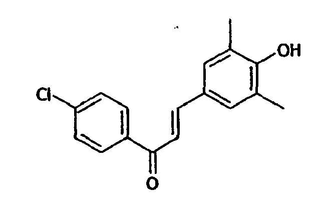

Ce composé est synthétisé à partir de 4-chloroacétophénone et de 3,5-diméthyl-4-hydroxybenzaldéhyde selon la méthode générale 1 précédemment décrite.
Purification par chromatographie sur gel de silice (élution : cyclohexane-acétate d'éthyle : 95-5).
RM N 1H CDCl₃ δ ppm : 2.30(s, 6H), 7.32(s, 2H), 7.34(d, J = 15.25Hz, 1H), 7.47(d, J = 8.86Hz, 2H), 7.75(d, J = 15.26, 1H), 7.97(d, J = 8.86Hz, 2H).

### Composé intermédiaire 2 :

### 1-[4-méthylthiophényl] -3-[3,5-diméthyl-4-hydroxyphényl]prop-2-èn-1-one

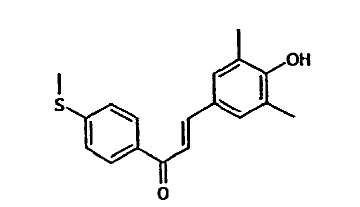

Ce composé est synthétisé à partir de 4'-méthylthioacétophénone et de 3,5-diméthyl-4-hydroxybenzaldéhyde selon la méthode générale 1 précédemment décrite.
Purification par chromatographie sur gel de silice (élution : cyclohexane-acétate d'éthyle : 8-2).
RMN 1H DMSO δ ppm : 2.22(s, 6H), 2.54(s, 3H), 7.36(d, J = 8.20Hz, 2H), 7.48(s, 2H), 7.62(d, J = 15.7Hz, 1H), 7.74(d, J = 15.7Hz, 1H), 8.10(d, J = 8.20Hz, 2H), 8.92(s, 1H)

### Composé intermédiaire 3 :

### 1-[2-méthoxyphényl] -3-[3,5-diméthyl-4-hydroxyphényl]prop-2-èn-1-one

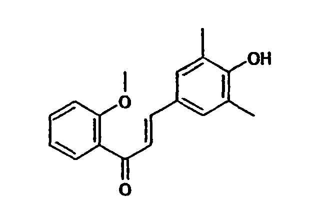

Ce composé est synthétisé à partir de 2'-méthoxyacétophénone et de 3,5-diméthyl-4 hydroxybenzaldéhyde selon la méthode générale 1 précédemment décrite.
Purification par chromatographie sur gel de silice (cyclohexane-acétate d'éthyle : 8-2).
RMN 1H DMSO δ ppm : 2.39(s, 6H), 2.22(s, 6H), 7.58(s, 2H), 7.67-7.62(m, 3H), 7.82(d, J = 15,5 Hz, 1H), 8.17(d, 1H), 12.96(s, 1H)

### Composé intermédiaire 4:

### 1-[4-hexyloxyphényl] -3-[3,5-diméthyl-4-hydroxyphényl]prop-2-èn-1-one

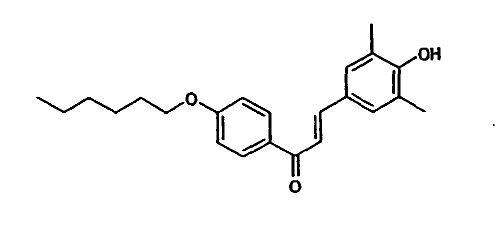

Ce composé est synthétisé à partir de 4-hexyloxyacétophénone et de 3,5-diméthyl-4-hydroxybenzaldéhyde selon la méthode générale 1 précédemment décrite.

Le composé attendu précipite dans le milieu réactionnel, il est essoré puis est utilisé sans autre purification pour la réaction suivante.
RM N 1H DMSO δ ppm : 0.88(m, 3H), 1.28-1.43(m, 6H), 1.72(m, 2H), 2.21 (s, 6H), 4.05(t, J = 6.42Hz, 2H), 7.40(d, J = 8.43Hz, 2H), 7.48(s, 2H), 7.57(d, J = 15.24Hz, 1H), 7.72(d, J = 15.24Hz, 1H), 8.12(d, J = 8.43Hz, 2H), 8.89(s, 1H)

### Composé intermédiaire 5 :

### 1-[2-hydroxy-4-chlorophényl] -3-[3,5-diméthyl-4-hydroxyphényl]prop-2-èn-1-one

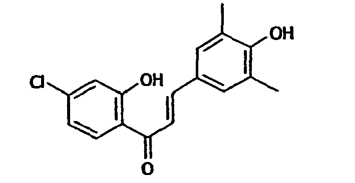

Ce composé est synthétisé à partir de 4'-chloro-2'-hydroxyacétophénone (matière première 3) et de 3,5-diméthyl-4-hydroxybenzaldéhyde selon la méthode générale 1 précédemment décrite.
Purification par chromatographie sur gel de silice (toluène : 10).
RMN 1H DMSO δppm : 2.21 (s, 6H), 7.1 (m, 2H), 7.55(s, 2H), 7.72(d, J = 15.4Hz, 1H), 7.80(d, J = 15.4Hz, 1H), 8.25(d, J=9.0Hz, 1H), 9.09(s, 1H), 13.04(s, 1H)

### Composé intermédiaire 6:

### 2-(3,5-diméthyl-4-hydroxyphényl)-7-chloro-4H-1-benzopyran-4-one

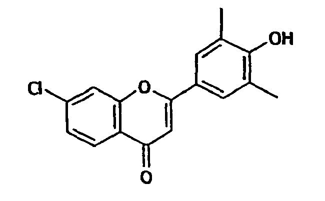

Ce composé est synthétisé à partir de 1-[2-hydroxy-4-chlorophényl]-3-[3,5-diméthyl-4-hydroxyphényl]prop-2-èn-1-one (composé intermédiaire 5) selon la méthode suivante :

La 1-[2-hydroxy-4-chlorophényl]-3-[3,5-diméthyl-4-hydroxyphényl]prop-2-èn-1-one est solubilisée dans du diméthylsulfoxyde, un cristal d'iode est ajouté, l'ensemble est maintenu 10 minutes à reflux.

Le milieu réactionnel est ramené à température ambiante, hydrolysé. Le précipité est essoré, rincé avec une solution de thiosulfate de sodium puis avec de l'eau.

Purification par dissolution dans le chlorure de méthylène et précipitation par addition d'heptane.
RMN 1H DMSO δppm : 2.25(s, 6H), 6.87(s, 1H), 7.51 (d, J = 8.55Hz, 1H), 7.73(s, 2H), 7.98(m, 2H)
Bibliographie : Doshi AG, S. P., Ghiya BJ (1986). Indian J Chem Sect B 25: 759.

### Composé intermédiaire 7 :

### 1-[2-méthyloxy-4-chlorophényl] -3-[3,5-diméthyl-4-hydroxydiméthylméthyloxyphényl]prop-2-èn-1-one:

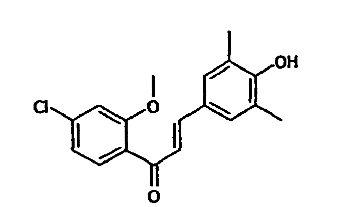

Ce composé est synthétisé à partir de 4'-chloro-2'-méthoxyacétophénone et de 3,5-diméthyl-4-hydroxybenzaldéhyde selon la méthode générale 1 précédemment décrite.
Purification par chromatographie sur gel de silice (cyclohexane-acétate d'éthyle : 85-15).
RMN 1H DMSO δ ppm: 2.21 (s, 6H), 3.90(s, 3H), 7.12(m, 1H), 7.23(d, J = 15.5Hz, 1H), 7.29(s, J = 1.80Hz, 1H), 7.38(d, J = 15.5 Hz, 1H), 7.41 (s, 2H), 7.48(d, J = 7.98Hz, 1H)

### Composé intermédiaire 8 :

### 1-[4-bromophényl]-3-[3,5-diméthyl-4-hydroxyphényl]prop-2-èn-1-one :

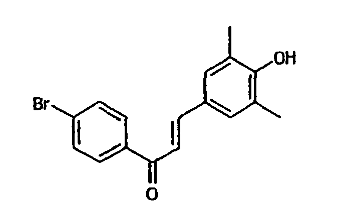

Ce composé est synthétisé à partir de 4'-bromoacétophénone et de 3,5-diméthyl-4-hydroxybenzaldéhyde selon la méthode générale 1 précédemment décrite.
Purification par chromatographie sur gel de silice (cyclohexane-acétate d'éthyle : 85-15).
RMN 1H DMSO δ ppm : 2,30(s, 6H), 7.32 (s, 2H), 7.56-7.66(m, 3H), 7.75(d, J = 15.27Hz, 1H), 7.90(d, J = 8.70Hz, 2H). 9.82(s, 1H)

### Composé intermédiaire 9 :

### 1-[4-heutylphényl] -3-[3,5-diméthyl-4-hydroxyphényl]prop-2-èn-1-one :

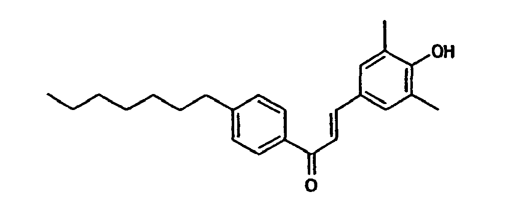

Ce composé est synthétisé à partir de 4'-heptylacétophénone et de 3,5-diméthyl-4-hydroxybenzaldéhyde selon la méthode générale 1 précédemment décrite.
Purification par chromatographie sur gel de silice (cyclohexane-acétate d'éthyle : 85-15).
RMN 1H DMSO δ ppm : 0.84 (m, 3H), 1.25(m, 8H), 1.60(m, 2H), 2.21 (s, 6H), 2.65(t, J = 7.50Hz, 2H), 7.35(d, J = 8.02Hz, 2H), 7.48(s, 2H), 7.60(d, J = 15.48Hz, 1H), 7.71 (d, J = 15.48Hz, 1H), 8.05(d, J = 8.02Hz, 2H), 8.92(s, 1H)

### Synthèse des composés selon l'invention :

### Composé 1:

### 1-[2-hydroxy-4-éthoxycarbonyldiméthylméthyloxyphényl] -3-[3,5-ditertiobutyl-4-hydroxyphényl]prop-2-en-1-one

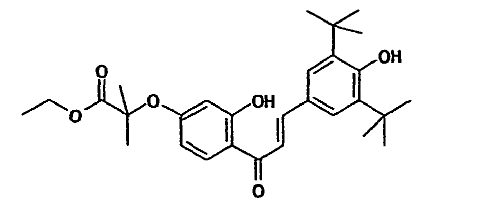

Ce composé est synthétisé à partir de 2'-Hydroxy-4'-(éthoxycarbonyldiméthylméthoxy)acétophénone (matière première 1) et de 3,5-ditertiobutyl-4-hydroxybenzaldéhyde selon la méthode générale 1 précédemment décrite.
Purification par chromatographie sur gel de silice (cyclohexane-acétate d'éthyle : 9-1).
RMN 1H CDCl₃ δ ppm : 1.25(t, J = 7.11 Hz, 3H), 1.45(s, 18H), 1.70(s, 6H), 4.26(q, J = 7.11Hz, 2H), 5.63(s, 1H), 6.33(d, J = 2.37Hz, 1H), 6.42(dd, J = 8.8Hz, J = 2.37Hz, 1H), 7.41 (d, J = 15.39Hz, 1H), 7.5(s, 2H), 7.83(d, J = 8.8Hz, 1H), 7.88(J = 15.39Hz, 1H), 13.5(s, 1H)

### Composé 2 :

### 1-[2-hydroxy-4-carboxydiméthylméthyloxyphényl] -3-[3,5-ditertiobutyl-4-hydroxyphényl]prop-2-èn-1-one :

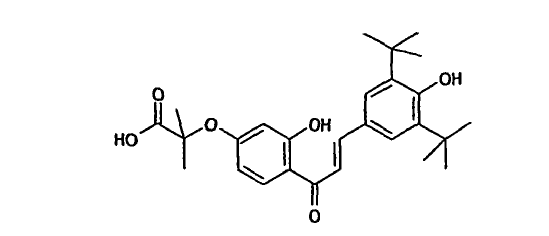

Ce composé est synthétisé à partir de 1-[2-hydroxy-4-éthoxycarbonyldiméthylméthyloxyphényil-3-[3,5-ditertiobutyl-4-hydroxyphényl] prop-2-èn-1-one (composé 1) selon la méthode suivante :

L'ester est solubilisé dans l'éthanol, une solution aqueuse de soude 1N (5 éq) est ajoutée, l'ensemble est maintenu 10 heures à reflux. Le milieu est acidifié par addition d"acide chlorhydrique (12N) puis extrait par de l'acétate d'éthyle. La phase organique est séchée sur du sulfate de magnésium puis évaporée sous pression réduite.
Purification par HPLC préparative (phase inverse RP18, licrospher 12µm, élution : eau - méthanol - acide trifluoroacétique : 22-78-0.1).
RMN 1H CDCl₃ δ ppm : 1.49(s, 18H), 1.73(s, 6H), 5.62(s, 1H), 6.44(d, J = 15.5 Hz, 1H), 7.01 (m, 2H), 7.57(t, 1H)**,** 7.81 (d, J= 15.5Hz, 1H), 7.87(d, 2H), 7.93(d, 1H), 8.26(d, 1H)
SM(ES-MS) : 453.2 (M-1)

### Composé 3 :

### 1-[2-hydroxy-4-chtorophényl] -3-[4-carboxydiméthylméthyloxyphényl]prop-2-èn-1-one :

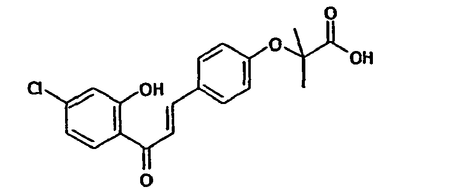

Ce composé est synthétisé à partir de 2'-hydroxy-4'-chloroacétophénone et de 4-ethyloxycarbonyldiméthylméthyloxybenzaldéhyde (matière première 9) selon la méthode générale 2 précédemment décrite.
Purification par chromatographie sur gel de silice (élution : cyclohexane-acétate d'éthyle : 9-1).
RMN 1H DMSO δ ppm : 1.58(s, 6H), 6.87(d, J = 8.54Hz, 2H), 7.05(dd, ,J = 8,55, J = 1.83 Hz, 1H), 7.09(d, J = 1.2Hz, 1H), 7.90-7.80(m, 4H), 8.25(d, J = 8.52Hz, 1H), 12.84(s, 1H), 13.26(s, 1H)
SM(ES-MS) : 359.0 (M-1)

### Composé 4 :

### 1-[2-hydroxyphényl] -3-[4-carboxydiméthylméthyloxyphényl]prop-2-èn-1-one :

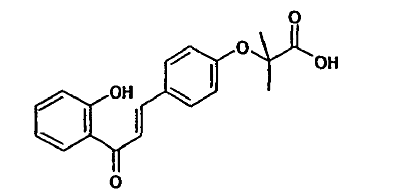

Ce composé est synthétisé à partir de 2'-hydroxyacétophénone et de 4-éthyloxycarbonyldiméthylméthyloxybenzaldéhyde (matière première 4) selon la méthode générale 2 précédemment décrite.
Purification par chromatographie sur gel de silice (élution : cyclohexane-acétate d'éthyle : 9-1).
RMN 1H DMSO δ ppm : 1.58(s, 6H), 6.88(d, 2H), 7.01 (m, 2H), 7.57(t, 1H), 7.81 (d, J =15,5 Hz, 1H), 7.87(d, 2H), 7.93(d J = 15,5 Hz, 1H), 8.26(d, 1H), 12.69(s, 1H)
SM(ES-MS) : 325.1 (M-1)

### Composé 5:

### 1-[2-hydroxyahényl] -3-[3,5-diméthoxy-4-carboxydiméthylméthyloxyphényl]prop-2-èn-1-one :

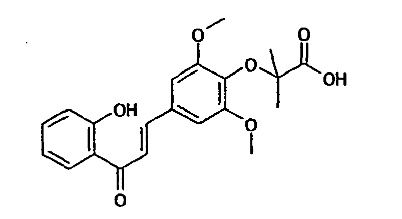

Ce composé est synthétisé à partir de 2'-hydroxyacétophénone et de 3,5-diméthyloxy-4-éthyloxycarbonyldiméthylméthyloxybenzaldéhyde (matière première 5) selon la méthode générale 2 précédemment décrite.
Purification par chromatographie sur gel de silice (élution : cyclohexane-acétate d'éthyle : 9-1).
RMN 1H DMSO δ ppm: 1.35(s, 6H), 3.80(s, 6H), 7.00-7.03(m, 2H), 7.25(s, 2H), 7.59(t, 1H,J = 8,07 Hz, 1H), 7.81 (d, J = 15,5 Hz, 1H), 8.00(d, J = 15,5 Hz, 1H), 8.31 (d, J = 8,07 Hz, 1H), 12.36(s, 1H), 12.69(s, 1H)
SM(ES-MS): 385.3 (M-1)

### Composé 6 :

### 1-[2-hydroxy-4-chlorophényl] -3-[3,5-diméthoxy-4-carboxydiméthylméthyloxyphényl]pron-2-èn-1-one :

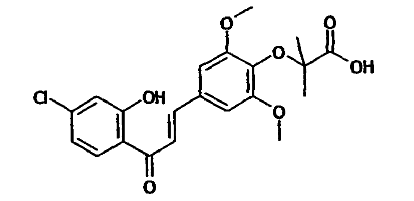

Ce composé est synthétisé à partir de 2'-hydroxy-4'-chloroacétophénone (matière première 3) et de 3,5-diméthyloxy-4-éthyloxycarbonyldiméthylméthyloxybenzaldéhyde (matière première 5) selon la méthode générale 2 précédemment décrite.
Purification par chromatographie sur gel de silice (élution : cyclohexane-acétate d'éthyle : 9-1).
RMN 1H DMSO δ ppm : 1.34(s, 6H), 3.80(s, 6H), 7.08(dd, J = 1.77Hz, 1H), 7.12(d, J = 1.77Hz,1H). 7.24(s, 2H), 7.79(d, J = 15,4 Hz, 1H), 7.93(d, J = 15,4 Hz, 1H), 8.27(d, J = 8,3 Hz, 1H), 12.36(s, 1H), 12.69(s. 1H)
SM(ES-MS):419.0(M-1)

### Composé 7 :

### 1-[2-hydroxy-4-chlorophényl]-3-[3,5-diméthyl-4-carboxydiméthyl méthyloxyphényl]prop-2-èn-1-one :

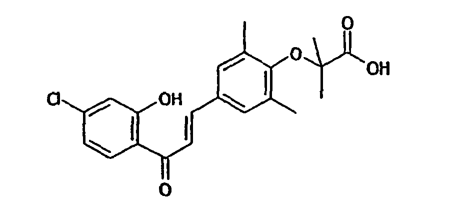

Ce composé est synthétisé à partir de 2'-hydroxy-4'-chloroacétophénone (matière première 3) et de 3,5-diméthyl-4-éthyloxycarbonyldiméthylméthyloxybenzaldéhyde selon (matière première 6) la méthode générale 2 précédemment décrite.
Purification par chromatographie sur gel de silice (élution : cyclohexane-acétate d'éthyle : 9-1).
RMN 1H DMSO δ ppm : 1.39(s, 6H), 2.22(s, 6H), 7.07(m, 1H), 7.12(d, J= 2,07 Hz, 1H), 7.61 (s, 2H), 7.74(d, J= 15,5 Hz, 1H), 7.87(d, J = 15,5 Hz, 1H), 8.26(d, 1H), 12.76(s, 1H)
SM(ES-MS): 387.1 (M-1)

### Composé 8 :

### 1-[2-hydroxy-4-carboxydiméthylméthyloxyphényl]-3-[3,5-dibromo-4-hydroxyphényl]prop-2-èn-1-one :

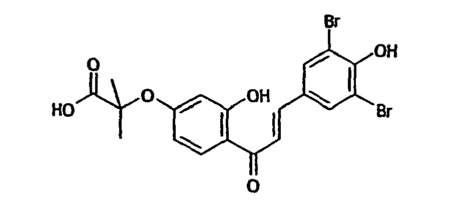

Ce composé est synthétisé à partir de 2'-hydroxy-4'-éthyl oxycarbonyldiméthylméthyloxyacétophénone (matière première 1) et de 3,5-dibromo-4-hydroxybenzaldéhyde selon la méthode générale 2 précédemment décrite.
Purification par chromatographie sur gel de silice (élution : cyclohexane-acétate d'éthyle : 9-1).
RMN 1H CDCl₃ δ ppm : 1,60(s, 6H), 6.24(d, J = 2.47 Hz, 1H), 6.43(dd, J = 2.47, J = 8.52Hz, 1H), 7.70 (d, J = 15.5 Hz, 1H), 7.96 (d, J = 15.5 Hz, 1H), 8.22(s, 2H), 8.34(d, J = 9.16 Hz, 1H), 13.34(s, 1H)
SM(ES-MS) : 498.6 (M-1)

### Composé 9 :

### 1-[2-hydroxyphényl] -3-[3-carboxydiméthylméthyloxyphényl]prop-2-èn-1-one:

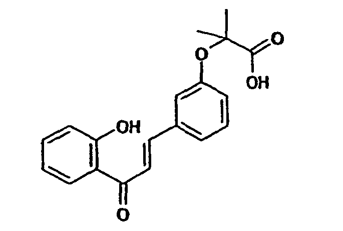

Ce composé est synthétisé à partir de 2'-hydroxyacétophénone et de 3-éthyloxycarbonyldiméthylméthyloxybenzaldéhyde (matière première 7) selon la méthode générale 2 précédemment décrite.
Purification par chromatographie sur gel de silice (élution : cyclohexane-acétate d'éthyle : 9-1).
RMN 1H DMSO δ ppm : 1.56(s, 6H), 6.91 (dd, J = 8.01 Hz, J = 2.47Hz, 1H), 7.03-6.99(m, 2H), 7.41-7.36(m, 2H), 7.60-7.52(m, 2H), 7.77(d, J = 15.5Hz, 1H), 8.00(d, J = 15.5Hz, 1H), 8.31(dd, J = 8.63Hz, J = 1.85Hz, 1H), 12.47(s, 1H), 13.17(s, 1H)
SM(ES-MS) : 325.8(M-1)

### Composé 10:

### 1-[2-hydroxy-4-carboxydiméthylméthyloxyphényl] -3-[3-hydroxyphényl]prop-2-èn-1-one:

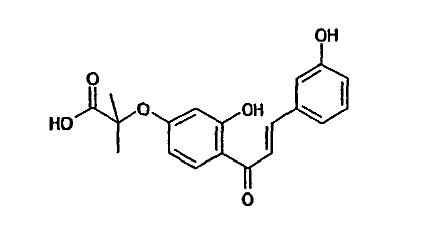

Ce composé est synthétisé à partir de 2'-hydroxy-4'-éthyloxycarbonyl diméthylméthyloxyacétophénone (matière première **1**) et de 3-hydroxybenzaldéhyde selon la méthode générale 2 précédemment décrite.
Purification par chromatographie sur gel de silice (élution : cyclohexane-acétate d'éthyle : 9-1).
RM N 1H DMSO δ ppm : 1.60(s, 6H), 6.25(d, J = 2.47 Hz, 1H), 6.43(dd, J = 2.47Hz, 9.09 Hz, 1H), 6.89(m, 1H), 7.35-7.24(m, 3H), 7.73(d, 1H), 7.92(d, J = 15,5 Hz, 1H), 8.27(d, J =15,5 Hz, 1H), 13.21 (s, 1H), 13.39 (s, 1H).
SM(ES-MS) : 341(M-1)

### Composé 11 :

### 1-[2-hydroxyphényl] -3-[3,5-diméthyl-4-carboxydiméthylméthyloxyphényl]prop-2-èn-1-one :

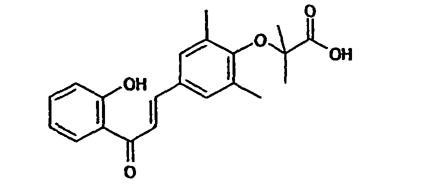

Ce composé est synthétisé à partir de 2'-hydroxyacétophénone et de 3,5-diméthyl-4-éthyloxycarbonyldiméthylméthyloxybenzaldéhyde (matière première 6) selon la méthode générale 2 précédemment décrite.
Purification par chromatographie sur gel de silice (élution : cyclohexane-acétate d'éthyle : 9-1) puis par HPLC préparative (phase inverse RP18, licrospher 12µm, élution : eau - méthanol - acide trifluoroacétique : 22-78-0.1).
RMN 1H DMSO δ ppm : 1.57 (s, 6H), 2.31 (s, 6H), 6.96(t, J= 8,17Hz, 1H), 7.04(d, J= 8,72 Hz, 1H), 7.35(s, 2H), 7.49(t, J= 8,2 Hz, 1H), 7.58(d, J= 15,8Hz,1H), 7.84(d, J= 15,8Hz, 1H), 7.94(d, J= 8,7Hz, 1H), 12.87(s, 1H)
SM(ES-MS) : 353.1 (M-1)

### Composé 12 :

### 1-[2-hydroxy-4-carboxydiméthylméthyloxyphényl] -3-[4-méthylthiophényl]prop-2-èn-1-one :

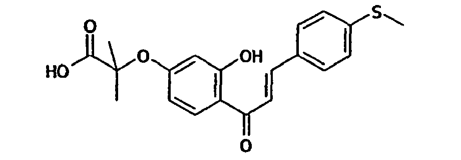

Ce composé est synthétisé à partir de 2'-hydroxy-4'-éthyloxycarbonyldiméthylméthyloxyacétophénone (matière première 1) et de 4-méthylthiobenzaldéhyde selon la méthode générale 2 précédemment décrite. Purification par chromatographie sur gel de silice (élution : cyclohexane-acétate d'éthyle : 9-1) puis HPLC préparative (phase inverse RP18, licrospher 12µm, élution : eau - méthanol - acide trifluoroacétique : 22-78-0.3).
RM N 1H DMSO δ ppm : 1.60 (s, 6H), 2.54(s, 3H), 6.25(d, 1H), 6.43(dd, J= 2.47 Hz, 1H), 7.33(d, J= 8.56 Hz, 2H), 7.8(d, 15.5Hz, 1H), 7.86(d, J = 8.56Hz, 2H), 7.98(d, J = 15.5 Hz, 1H), 8.29(d, J = 9.1 Hz, 1H), 13.34(s, 1H)
SM(ES-MS) : 373.1 (M-1)

### Composé 13 :

### 1-[2,4-dihydroxyphényl]-3-[4-carboxydiméthylméthyloxyphényl] prop-2-èn-1-one:

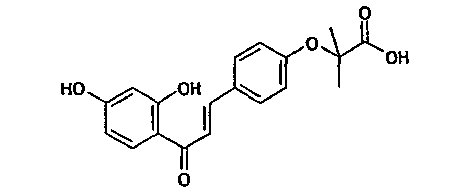

Ce composé est synthétisé à partir de 2',4'-dihydroxyacétophénone et de 4-éthoxycarbonyldiméthylméthyloxybenzaldéhyde (matière première 4) selon la méthode générale 2 précédemment décrite.
Purification par chromatographie sur gel de silice (élution : cyclohexane-acétate d'éthyle : 9-1) puis par HPLC préparative (phase inverse RP18, licrospher 12µm, élution : eau - méthanol - acide trifluoroacétique : 34-66-0.1).

RMN 1H DMSO δ ppm : 1.57(s, 6H), 6.29(d, J= 2,16 Hz, 1H), 6.41 (dd, J= 9,18, J = 2,16Hz, 1H), 6.86(d, J= 8,64Hz, 2H), 7.75(d, J= 15,67 Hz, 1H), 7.83-7.88(m, 3H), 8.19(d, J= 9,18Hz, 1H), 10.74(s, 1H), 13.53(s, 1H)
SM(maldi-Tof): 343.1(M+1)

### Composé 14 :

### 1-[2-hydroxyphényl]-3-[4-carboxydiméthylméthyl oxyphényl]prop-2-èn-1-one

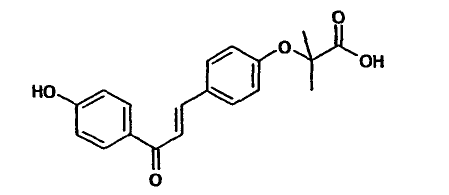

Ce composé est synthétisé à partir de 4'-hydroxyacétophénone et de 4-éthoxycarbonyldiméthylméthyloxybenzaldéhyde (matière première 4) selon la méthode générale 2 précédemment décrite.
Purification par chromatographie sur gel de silice (élution : cyclohexane-acétate d'éthyle : 9-1) puis par HPLC préparative (phase inverse RP18, licrospher 12µm, élution : eau - méthanol - acide trifluoroacétique : 34-66-0.1).
RMN 1H DMSO δ ppm : 1.56(s, 6H), 6.85(d, J= 8,63 Hz, 2H), 6.90(d, J= 9,21 Hz, 2H), 7.63(d, J= 15,54Hz, 1H), 7.78(m, 3H), 8.05(d, J= 8,61 Hz, 2H), 10.40(s, 1H), 13.22(s, 1H)
SM(maldi-Tof) : 327.1 (M+1)

### Composé 15:

### 1-[4-chloronhényl]-3-[3,5-diméthyl-4-isopropyloxycarbonyldiméthylméthyloxyhényl]prop-2-èn-1-one:

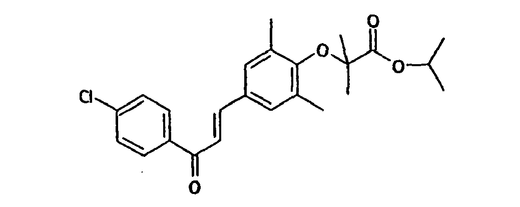

Ce composé est synthétisé à partir de 1-[4-chlorophényl]-3-[3,5-diméthyl-4-hydroxyphényl]prop-2-èn-1-one (composé intermédiaire 1) et de bromoisobutyrate d'isopropyle selon la méthode générale 4 précédemment décrite.
Purification par chromatographie sur gel de silice (élution : cyclohexane-acétate d'éthyle : 9-1).
RMN 1H DMSO δ ppm : 1.25(d, J = 6.06Hz, 6H), 1.39(s, 6H), 5.00(sept, J = 6.06Hz, 1H), 7.57(s, 2H), 7.62(d, J = 8.40Hz, 2H), 7.64(d, J = 15.8Hz, 1H), 7.81 (d, J = 15.8, 1H), 8.16(d, J = 8.40Hz, 2H).
SM(Maldi-Tof) : 415.1(M+1)

### Composé 16 :

### 1 [4-chlorophényl]-3-[3,5-diméthyl-4-tertiobutyloxycarbonyldiméthylméthyloxyphényl]prop-2-èn-1-one :

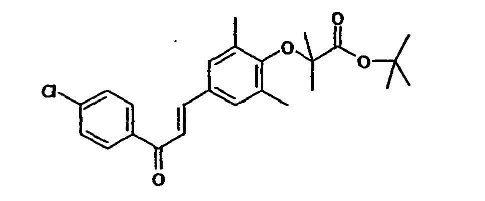

Ce composé est synthétisé à partir de 1-[4-chlorophényl]-3-[3,5-diméthyl-4-hydroxyphényl]prop-2-èn-1-one (composé intermédiaire 1) et de bromoisobutyrate de tertiobutyle selon la méthode générale 4 précédemment décrite.
Purification par chromatographie sur gel de silice (élution : cyclohexane-acétate d'éthyle : 9-1).

### Composé 17:

### 1-[4-chlorophényl] -3-[3,5-diméthyl-4-carboxydiméthylméthyloxyphényl]prop-2-èn-1-one :

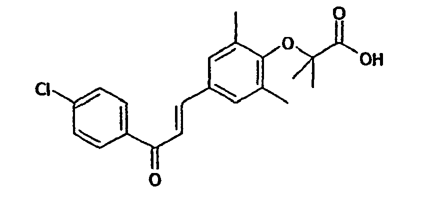

Ce composé est synthétisé à partir de 1-[4-chlorophényl]-3-[3,5-diméthyl-4 tertiobutyloxycarbonyldiméthylméthyloxyphényl]prop-2-èn-1-one (composé 16) selon la méthode générale 5 précédemment décrite.
Purification par chromatographie sur gel de silice (élution: dichlorométhane-méthanol : 98-2)
RMN 1H DMSO δ ppm : 1.39(s, 6H), 2.22(s, 6H), 7.58(s, 2H), 7.67-7.62(m, 3H), 7.82(d, J =15,5 Hz, 1H), 8.17(d, 1H), 12.96(s, 1H)
SM(Maldi-Tof): 373.3(M+1)

### Composé 18 :

### 1-[2-hydroxy-4-carboxydiméthylméthyloxyphényl]-3-[4-chlorophényl]prop-2-èn-1-one :

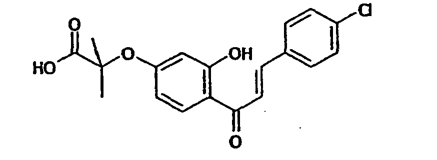

Ce composé est synthétisé à partir de 2'-hydroxy-4'-éthyloxycarbonyldiméthylméthyloxyacétophénone (matière première 1) et de 4-chlorobenzaldéhyde selon la méthode générale 2 précédemment décrite.
Purification par chromatographie sur gel de silice (élution : cyclohexane-acétate d'éthyle : 9-1) puis par HPLC préparative (phase inverse RP18, licrospher 12µm, élution : eau - méthanol - acide trifluoroacétique : 22-78-0.1).
RMN 1H DMSO δ ppm : 1.60 (s, 6H), 6.25 (d, J = 2,47 Hz, 1H), 6.45 (dd, J = 2,47, J = 9.12 Hz, 1H), 6.55 (d, J = 8,55 Hz, 2H), 7.82 (d, J = 15,54Hz, 1H), 7.97 (d, J = 8.55Hz, 2H), 8.03 (d, J = 15.54Hz, 1H), 8,29 (d, J = 9.12Hz, 1H), 13,20 (s, 1H), 13,39 (s, 1H)
SM(ES-MS) : 359.0 (M-1)

### Composé 19 :

### 1-[2-hydroxyphényl]-3-[4-carboxydiméthylméthyl thiophényl]prop-2-èn-1-one

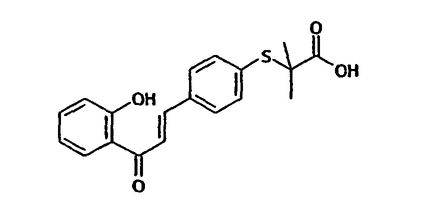

Ce composé est synthétisé à partir de 2'-hydroxyacétophénone et éthyloxycarbonyldiméthylméthylthiobenzaldéhyde (matière première 8) selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.44(s, 6H), 6.99-7.05(m, 1H), 7.52(d, J = 8.1 Hz, 2H), 7.58(m, 1H), 7.83(d, J = 15.5Hz, 1H), 7.92(d, J = 8.1 Hz, 1H), 8.09(d. J = 15.5Hz, 1H), 8.26(dd, J = 1.62, J = 8.6 Hz, 1H), 12.47(s, 1H), 12.78(s, 1H)
SM(Maldi-Tof): 242.9 (M+1)

### Composé 20 :

### 1-[4-chloro-2-hydroxyphényl] -3-[4-carboxydiméthylméthylthiophényl]prop-2-èn-1-one

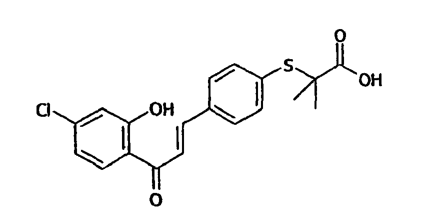

Ce composé est synthétisé à partir de 4'-chloro-2'-hydroxyacétophénone (matière première 3) et de 4-éthyloxycarbonyldiméthylméthylthiobenzaldéhyde (matière première 8) selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.43(s, 6H), 7.05(dd, J = 1,7Hz, J = 8,46Hz, 1H), 7.11 (d, J = 2,25Hz, 1H), 7.51 (d, J = 7,92Hz, 2H), 7.82(d, J = 15,8 Hz, 1H), 7.89(d, J = 7,9Hz , 2H), 8.05(d, J = 15,2Hz, 1H), 8.23(d, J = 8,46 Hz, 1H), 12.57(s, 1H), 12.78(s, 1H).
SM(Maldi-Tof) : 377.0(M-1)

### Composé 21 :

### 1-[4-carboxydiméthylméthyloxyphényl] -3-[3,5-diméthyl4-hydroxyphényl]prop-2-èn-1-one

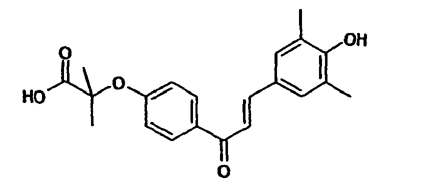

Ce composé est synthétisé à partir de 4-éthyloxycarbonyldiméthylméthyloxy acétophénone (matière première 9) et de 3,5-diméthyl-4-hydroxybenzaldéhyde selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.60(s, 6H), 2.21 (s, 6H), 6.91 (d. J = 9.09Hz, 2H), 7.48(s, 2H), 7.57(d, J = 15.12Hz, 1H), 7.70(d, J = 15.63Hz, 1H), 8.09(d, J = 9,06Hz, 2H), 8.9(s, 1H), 13.29(s, 1H)
SM(Maldi-Tof): 355.2 (M+1)

### Composé 22 :

### 1-[4-méthylthiophényl] -3-[4-carboxydiméthylméthyloxyphényl]prop-2-èn-1-one

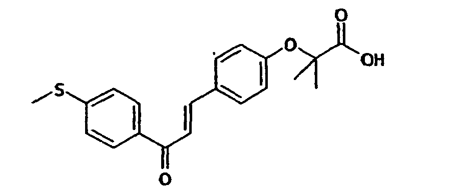

Ce composé est synthétisé à partir de 4'-méthylthioacétophénone (matière première 12) et de 4-éthyloxycarbonyldiméthylméthyloxybenzaldéhyde (matière première 9) selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.57(s, 6H), 2.57(s, 3H), 6.86(d, J = 8,94Hz, 2H), 7.41(d, J = 8,40Hz, 2H), 7.69(d, J = 15,2Hz, 1H), 7.84-7.78(m, 3H), 8.09(d, J = 8,4Hz, 2H), 13.21 (s, 1H)
SM(Maldi-Tof) : 357.2 (M-1)

### Composé 23 :

### 1-[4-carboxydiméthylméthyloxyphényl] -3-[4-chlorophényl]prop-2-èn-1-one :

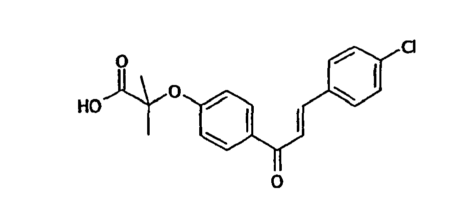

Ce composé est synthétisé à partir de 4-éthyloxycarbonyl diméthylméthyloxyacétophénone (matière première 9) et de 4-chlorobenzaldéhyde selon la méthode générale 3 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.72(s, 6H), 6.97(d, J = 8,61 Hz, 2H), 7.39(d, J = 8,25Hz, 2H), 7.50(d, J = 15,72Hz, 1H), 7.57(d, J = 8.61 Hz, 2H), 7.77(d, J = 15,72Hz, 1H), 7.99(d, J = 8,61 Hz, 2H), 13.30(s, 1H)
SM(Maldi-Tof) : 345.1 (M+1)

### Composé 24:

### 1-[4-carboxydiméthylméthylthiophényl] -3-[4-méthylthiophényl]prop-2-èn-1-one :

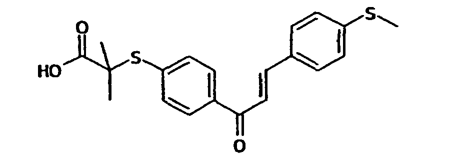

Ce composé est synthétisé à partir de 4-éthyloxycarbonyl diméthylméthylthiocétophénone (matière première 12) et de 4-méthylthiobenzaldéhyde selon la méthode générale 3 précédemment décrite. Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1,46 (s, 6H), 2.54 (s, 3H), 7.33 (d, J = 8,61 Hz, 2H), 7.59 (d, J = 8,10Hz, 2H), 7.73 (d, J = 15,66Hz, 1H), 7.85 (d, J = 8,10Hz, 2H), 7.92 (d, J = 15,66Hz, 1H), 8.13 (d , 8,10Hz, 2H), 12.85 (s, 1H)
SM(Maldi-Tof) : 373.1 (M+1)

### Composé 25 :

### 1-[2-hydroxy-4-bromophényl] -3-[3,5-diméthyl-4-carboxydiméthylméthyloxyphényl]prop-2-èn-1-one

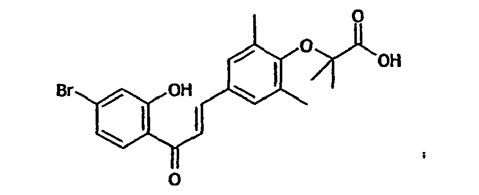

Ce composé est synthétisé à partir de 4'-bromo-2'-hydroxyacétophénone (matière première 11) et 3,5-diméthyl-4-éthyloxycarbonyldiméthyloxybenzaldéhyde (matière première 6) selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.39(s, 6H), 2.22(s, 6H), 7.20(dd, J = 2,16, J = 8,55Hz, 1H), 7.25(d, J = 1.59Hz, 1H), 7.60(s, 2H), 7.73(d, J = 15.51 Hz, 1H), 7.86(d, J = 15,51 Hz, 1H), 8.16(d, J = 8,58Hz, 1H), 12-70(s, 1H), 13.30(s, 1H)
SM(ES-MS) : 432.9 (M-1)

### Composé 26 :

### 1-[4-carboxydiméthylméthyloxyphényl] -3-[4-méthylthiophényl]prop-2-èn-1-one

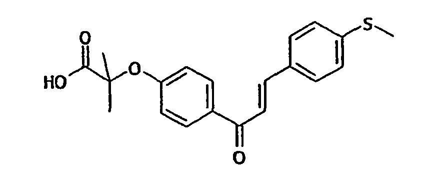

Ce composé est synthétisé à partir de 4'-éthyloxycarbonyldiméthylméthyloxyacétophénone (matière première 9) et de 4-méthylthiobenzaldéhyde selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RM N 1H DMSO δ ppm : 1.60(s, 6H), 2.53(s, 3H), 6.93(d, J = 9.00Hz, 2H), 7.32(d, J = 8.49Hz, 2H), 7.68(d, 15.51Hz, 1H), 7.82(d, J = 8.52Hz, 2H), 7.89(d, J = 15.51 Hz, 1H), 8.13(d, 9.OOHz, 2H), 13.30(s, 1H)
SM(Maldi-Tof) : 355.0(M+1)

### Composé 27 :

### 1-[4-méthylthiophényl]-3-[3,5-diméthyl-4-tertiobutyloxycarbonyldiméthylmethyloxyphényl]prop-2-èn-1-one :

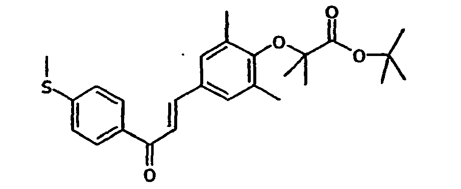

Ce composé est synthétisé à partir de 1-[4-méthylthiophényt]-3-[3,5-diméthyl-4-hydroxyphényl]prop-2-èn-1-one (composé intermédiaire 2) et de bromoisobutyrate de tertiobutyle selon la méthode générale 4 précédemment décrite.
Purification par chromatographie sur gel de silice (élution : cyclohexane-acétate d'éthyle : 8-2).

### Composé 28 :

### 1-[4-méthylthiophényl]-3-[3,5-diméthyl-4-isopropyloxycarbonyldiméthylméthyloxyphényl]prop-2-èn-1-one :

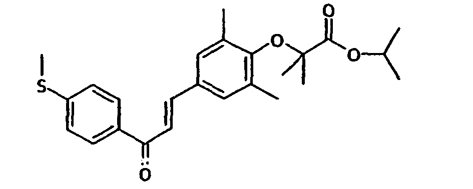

Ce composé est synthétisé à partir de 1-[4-méthylthiophényl]-3-[3,5-diméthyl-4-hydroxyphényl]prop-2-èn-1-one (composé intermédiaire 2) et de bromoisobutyrate d'isopropyle selon la méthode générale 4 précédemment décrite.
Purification par chromatographie sur gel de silice (élution : cyclohexane-acétate d'éthyle : 9-1).
RMN 1H DMSO δ ppm : 1.25(d, J = 6.18Hz, 6H), 1.39(s, 6H), 2.18(s, 6H), 2.57(s, 3H), 4.99(sept, J = 6.18Hz, 1H), 7.40(d, J = 8.28Hz, 2H), 7.58(s, 2H), 7.62(d, J = 15.5Hz, 1H), 7.82(d, J = 15.5Hz, 1H), 8.10(d, J = 8.28Hz, 2H), 12.97(s, 1H)
SM(Maldi-Tof): 427.1 (M+1)

### Composé 29 :

### 1-[4-méthylthiophényl] -3-[3,5-diméthyl-4-carboxydiméthylméthyloxyphényl]prop-2-èn-1-one :

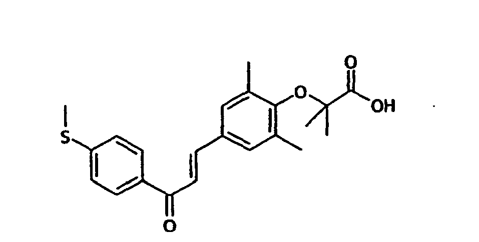

Ce composé est synthétisé à partir de 1-[4-méthylthiophényl]-3-[3,5-diméthyl-4-tertiobutyloxycarbonyldiméthylméthyloxyphényl]prop-2-èn-1-one (composé 28) selon la méthode générale 5 précédemment décrite.
Purification par chromatographie sur gel de silice (élution : dichlorométhane -méthanol : 98-2).
RMN 1H DMSO δ ppm : 1.39(s, 6H), 2.22(s, 6H), 2.57(s, 3H), 7.40(d, J = 8.55Hz, 2H), 7.57(s, 2H), 7.62(d, J = 15.5Hz, 1H), 7.83(d, J = 15.5Hz, 1H), 8.10(d, J = 8.55Hz, 2H), 12.97(s, 1H)
SM(ES-MS) : 383.3(M-1)

### Composé 30 :

### 1-[2-méthoxyphényl]-3-[3,5-diméthyl-4-tertibutyloxycarbonyldiméthylmethyloxyphényl]prop-2-èn-1-one:

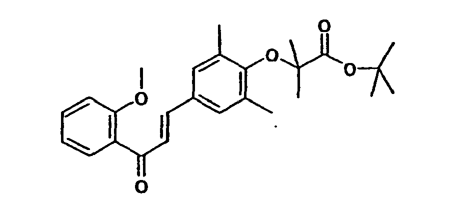

Ce composé est synthétisé à partir de 1-[2-méthoxyphényl]-3-[3,5-diméthy 1-4-hydroxyphényl]prop-2-èn-1-one (composé intermédiaire 3) et de bromoisobutyrate de tertiobutyle selon la méthode générale 4 précédemment décrite.
Purification par chromatographie sur gel de silice (élution : cyclohexane-acétate d'éthyle : 9-1).

### Composé 31 :

### 1-[2-méthoxyphényl] -3-[3,5-diméthyl-4-carboxydiméthylméthyloxyphényl]prop-2-èn-1-one :

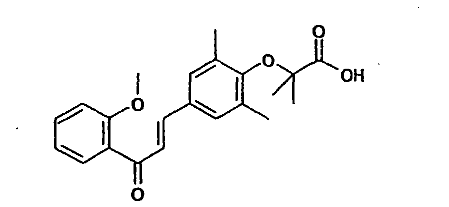

Ce composé est synthétisé à partir de 1-[2-méthoxyphényl]-3-[3,5-diméthyl-4-tertiobutyloxycarbonyldiméthylméthyloxyphényl]prop-2-èn-1-one (composé 30) selon la méthode générale 5 précédemment décrite.
Purification par chromatographie sur gel de silice (élution : dichlorométhane-méthanol : 98-2).
RMN 1H DMSO δ ppm : 1.38(s, 6H), 2.19(s, 6H), 3.93(s, 3H), 7.05(m, 1H), 7.20(d, J = 8.31 Hz, 1H), 7.25(d, J = 15.5Hz, 1H), 7.37(d, J = 15.5Hz, 1H), 7.39(s, 2H), 7.46(d, J = 7.2Hz, 1H), 7.53(m, 1H), 12.93(s, 1H)
SM(ES-MS) : 367.1(M-1)

### Composé 32 :

### 1-[4-hexyloxyphényl] -3-[3,5-diméthyl-4-tertiobutyloxycarbonyldiméthylméthyloxyphényl]prop-2-èn-1-one :

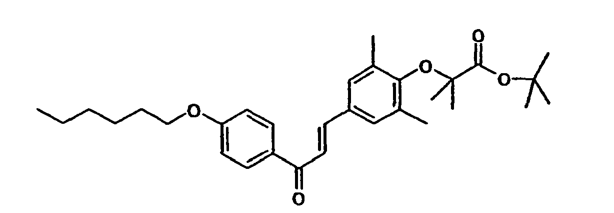

Ce composé est synthétisé à partir de 1-[4-hexyloxyphényl]-3-[3,5-diméthyl-4-hydroxyphényl]prop-2-èn-1-one (composé intermédiaire 4) de bromoisobutyrate de tertiobutyle selon la méthode générale 4 précédemment décrite:
Purification par chromatographie sur gel de silice (élution : cyclohexane-acétate d'éthyle : 95-5)

### Composé 33:

### 1-[4-hexyloxyphényl] -3-[3,5-diméthyl-4-carboxydiméthylméthyloxyphényl]prop-2-èn-1-one :

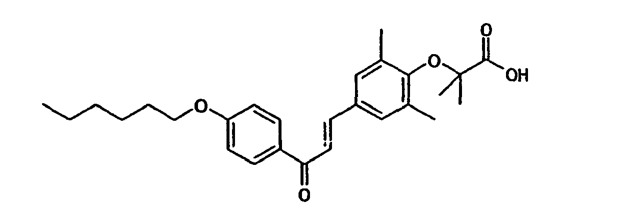

Ce composé est synthétisé à partir de 1-[4-héxyloxyphényl]-3-[3,5-diméthyl-4-tertiobutyloxycarbonyldiméthylméthyloxyphényl]prop-2-èn-1-one (composé 32) selon la méthode générale 5 précédemment décrite.
Purification par recristallisation dans le méthanol.
RM N 1H DMSO δ ppm : 0.88(t, J = 6.33Hz, 3H), 1.30(m, 4H), 1.39(s, 6H), 1.44(m, 2H), 1.73(m, 2H), 2.22(s, 6H), 4.06(t, J = 6.30Hz, 2H), 7.06(d, J = 8.61Hz, 2H), 7.56(s, 2H), 7.58(d, J = 15.5Hz, 1H), 7.82(d, J = 15.5Hz, 1H), 8.13(d, J = 6.61 Hz, 2H)
SM(ES-MS) : 437.2(M-1)

### Composé 34:

### 2-(3,5-diméthyl-4-tertiobutytoxycarbonyldiméthylméthyloxyphényl)-7-chloro-4H-1-benzopyran-4-one :

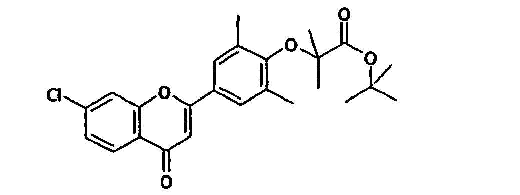

Ce composé est synthétisé à partir de 2-(3,5-diméthyl-4-hydroxyphényl)-7-chloro-4H-1-benzopyran-4-one (composé intermédiaire 6) et de bromoisobutyrate de tertiobutyle selon la méthode générale 4 précédemment décrite.
Purification par précipitation dans le mélange de solvants dichlorométhane/heptane.

### Composé 35 :

### 2-(3,5-diméthyl-4-carboxvdiméthylméthyloxyphényl)-7-chloro-4H-1-benzopyran-4-one

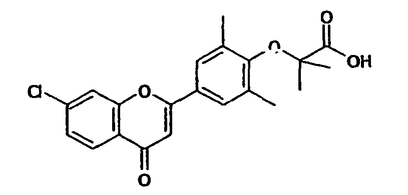

Ce composé est synthétisé à partir de 2-(3,5-diméthyl-4-tertiobutyloxycarbonyldiméthylméthyloxyphényl)-7-chloro-4H-1-benzopyran-4-one (composé 34) selon la méthode générale 5 précédemment décrite.
Purification par HPLC préparative (phase inverse RP18, licrospher 12µm, élution : eau - méthanol - acide trifluoroacétique : 22-78-0.1).
RMN 1H DMSO δ ppm : 1.24(s, 6H), 2.28(s, 6H), 7.02(s, 1H), 7.56(dd, J = 8.71 Hz, J = 1.75Hz, 1H), 7.85(s, 2H), 8.03(d, J = 1.75Hz, 1H), 8.06(d, J = 8.71Hz, 1H)
SM(MakH-Tof) : 387.1 (M+1)

### Composé 36:

### 1-[2-méthyloxy-4-chlorophényl] -3-[3,5-diméthyl-4-tertiobutyloxycarbonyldiméthylméthyloxyphényl]prop-2-èn-1-one :

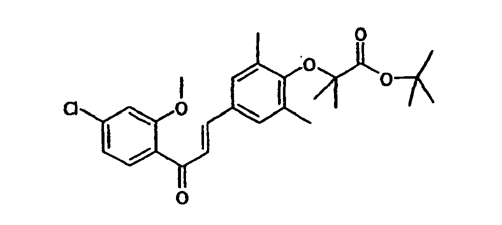

Ce composé est synthétisé à partir de 1-[2-méthyloxy-4-chlorophényl]-3-[3,5-diméthyl-4-hydroxydiméthylméthyloxyphényl]prop-2-èn-1-one (composé intermédiaire 7) et de bromoisobutyrate de tertiobutyle selon la méthode générale 4 précédemment décrite.
Purification par chromatographie sur gel de silice (élution : cyclohexane-acétate d'éthyle : 9-1)

### Composé 37 :

### 1-[2-méthyloxy-4-chlorophényl] -3-[3,5-diméthyl-4-carboxydiméthylméthyloxyphényl]prop-2-èn-1-one:

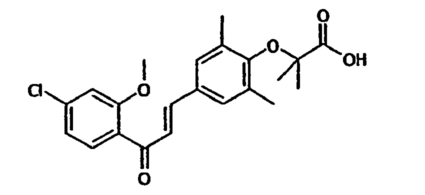

Ce composé est synthétisé à partir de 1-[2-méthoxy-4-chlorophényl]-3-[3,5-diméthyl-4-tertiobutyloxycarbonyldiméthylméthyloxyphényl]prop-2-èn-1-one (composé 36) selon la méthode générale 5 précédemment décrite.
Purification par chromatographie sur gel de silice (élution : dichlorométhane/méthanol : 98-2)
RMN 1H DMSO δ ppm : 1.38(s, 6H), 2.19(s, 6H), 3.89(s, 3H), 7.12(dd, J = 7.98, J = 1.71Hz, 1H), 7.23(d, J = 15.56Hz, 1H), 7.29(s, J = 1.71Hz, 1H), 7.38(d, J = 15.7 Hz, 1H), 7.41(s, 2H), 7.48(d, J = 7.98Hz, 1H)
SM(ES-SM) : 401.2(M-1)

### Composé 38 :

### 1-[4-heptylphényl] -3-[3,5-diméthyl-4-tertiobutyloxycarbonyldiméthylméthyloxyphényl]prop-2-èn-1-one :

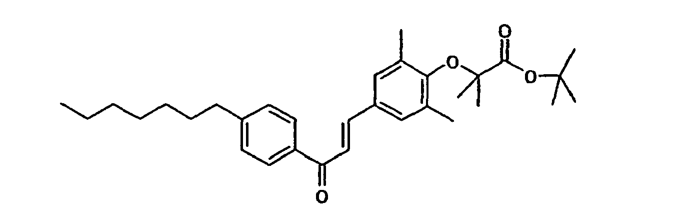

Ce composé est synthétisé à partir de 1-[4-heptylphényl]-3-[3,5-diméthyl-4-hydroxyphényl]prop-2-èn-1-one (composé intermédiaire 9) et de bromoisobutyrate de tertiobutyle selon la méthode générale 4 précédemment décrite.

Purification par chromatographie sur gel de silice (élution : cyclohexane-acétate d'éthyle : 9-1)

### Composé 39 :

### 1-[4-heptylphényl] -3-[3,5-diméthyl-4-carboxydiméthylméthyloxyphényl]prop-2èn-1-one:

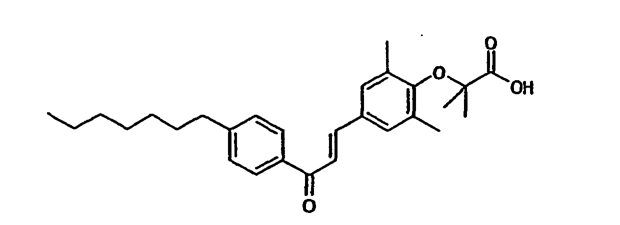

Ce composé est synthétisé à partir de 1-[4-heptylphényl]-3-[3,5-diméthyl-4-tertiobutyloxycarbonyldiméthylméthyloxyphényl]prop-2-èn-1-one (composé 38) et de bromoisobutyrate de tertiobutyle selon la méthode générale 4 précédemment décrite.
Purification par chromatographie sur gel de silice (élution : dichlorométhane/méthanol : 98-2)
RMN 1H DMSO δ ppm : 0.85(m, 3H), 1.30-1.24(m, 8H), 1.39(s, 6H), 1.60(m, 2H), 2.22(s, 6H), 2.67(t, J = 7.4Hz, 2H), 7.37(d, J = 8.04 Hz, 2H), 7.57(s, 2H), 7.62(d, J = 15.66 Hz, 1H), 7.82(d, J = 15.69 Hz, 1H), 8.07(d, J = 8.07 Hz, 2H)
SM(ES-MS) : 435.3(M-1)

### Composé 40 :

### 1-[4-bromophényl]-3-[3,5-diméthyl-4-tertiobutyloxycarbonyldiméthylméthyloxyphényl]prop-2-èn-1-one:

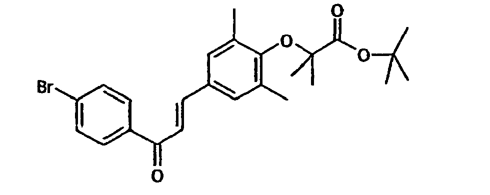

Ce composé est synthétisé à partir de 1-[4-bromophényl]-3-[3,5-diméthyl-4-hydroxyphényl]prop-2-èn-1-one (composé intermédiaire 8) et de bromoisobutyrate de tertiobutyle selon la méthode générale 4 précédemment décrite.
Purification par chromatographie sur gel de silice (élution : cyclohexane-acétate d'éthyle : 9-1)

### Composé 41 :

### 1-[4-bromophényl]-3-[3,5-diméthyl-4-carboxy diméthylméthyloxyphényl]prop-2-èn-1-one :

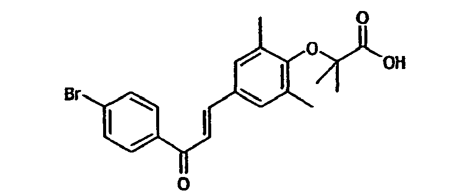

Ce composé est synthétisé à partir de 1-[4-bromophényl]-3-[3,5-diméthyl-4-tertiobutyloxycarbonyldiméthylméthyloxyphényl]prop-2-èn-1-one (composé 40) selon la méthode générale 5 précédemment décrite.
Purification par chromatographie sur gel de silice (élution : dichlorométhane-méthanol : 98-2)
RMN 1H DMSO δ ppm : 1.39 (s, 6H), 2.22 (s, 6H), 7.58 (s, 2H), 7.65 (d, J = 15.39Hz, 1H), 7.84-7.77 (m, 3H), 8.09 (d, J = 8.19Hz, 1H), 13.01 (s, 1H)
SM(ES-MS) : 417.2(M-1)

### Composé 42 :

### 1-[2-hydroxyphényl]-3-[3,5-diméthyl-4-isopropyloxycarbonyldiméthylméthvloxvphényllprop-2-èn-1-one :

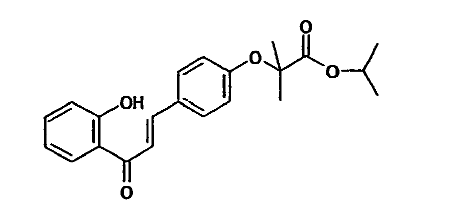

Le 1-[2-hydroxyphényl]-3-[4-carboxydiméthylméthyloxyphényl] prop-2-èn-1-one (Composé 4, 1 éq) est solubilisé dans le dichlorométhane. Le dichlorométhylméthylether (3 éq) est ajouté , L'ensemble est maintenu 8 heures à reflux. Le solvant et l'excès de réactif sont éliminés par évaporation sous pression réduite. Le résidu d'évaporation est repris par de l'isopropanol (50 éq). Après 12 heures d'agitation à température ambiante, l'isopropanol est éliminé par évaporation sous pression réduite.
Purification par chromatographie sur gel de silice (élution : toluène-acétate d'éthyle : 7-3)
RMN 1H CDCl₃ δppm : 1.21 (d, J = 6.09Hz, 6H),1.65 (s, 6H), 5.10 (sept, J = 6.10Hz, 1H), 6.86 (d, J = 8.65Hz, 2H), 6.95 (m, 1H), 7.02 (dd, J=8.65Hz, J = 1.53Hz, 1H), 7.48 (m, 1H), 7.54 (d, J=15.25Hz, 1H), 7.57 (d, J=8.65Hz, 2H), 7.87 (d, J=15.25Hz, 1H), 7.93 (d, J = 8.40 Hz, 1H), 12.94 (signal échangeable D₂O, 1H)
SM(Maldi-Tof) : 369.1(M+1)

### EXEMPLE 2: Evaluation de l'activation des PPARs in vitro

Les composés selon l'invention testés sont les composés dont la préparation est décrite dans les exemples décrits ci-dessus.

Les récepteurs nucléaires membres de la sous-famille des PPARs qui sont activés par deux classes majeures de composés pharmaceutiques, les fibrates et les glitazones, abondamment utilisées en clinique humaine pour le traitement des dyslipidémies et du diabète, jouent un rôle important dans l'homéostasie lipidique et glucidique. Les données expérimentales suivantes montrent que les composés selon l'invention activent PPARα et PPARγ *in vitro.*

L'activation des PPARs est évaluée *in vitro* dans des lignées de type fibroblastique RK13 par la mesure de l'activité transcriptionnelle de chimères constituées du domaine de liaison à l'ADN du facteur de transcription Gal4 de levure et du domaine de liaison du ligand des différents PPARs. Ces derniers résultats sont ensuite confirmés dans des lignées cellulaires selon les protocoles suivants :

L'exempte est donné pour les cellules RK13.

### a. Protocoles de culture

Les cellules RK13 proviennent de l' ECACC (Porton Down, UK) et sont cultivées dans du milieu DMEM supplémenté de 10% vol/vol sérum de veau foetal, 100 U/ml pénicilline (Gibco, Paisley, UK) et 2 mM L-Glutamine (Gibco, Paisley, UK). Le milieu de culture est changé tous les deux jours. Les cellules sont conservées à 37°C dans une atmosphère humide contenant 5% de CO₂ et 95% d'air.

### b. Description des plasmides utilisés

Les plasmides pG5TkpGL3, pRL-CMV, pGal4-hPPARα, pGal4-hPPARγ et pGal4-φ ont été décrits par Raspe, Madsen et al. (1999). Les constructions pGal4-mPPARα et pGal4-hPPARγ ont été obtenues par clonage dans le vecteur pGal4-φ de fragments d'ADN amplifiés par PCR correspondants au domaines DEF des récepteurs nucléaires PPARα et PPARγ humains.

### c. Transfection

Les cellules RK13 sont ensemencées dans des boîtes de culture de 24 puits à raison de 5x10⁴ cellules/puit et sont transfectées pendant 2 heures avec le plasmide rapporteur pG5TkpGL3 (50 ng/puit), les vecteurs d'expression pGal4-φ, pGal4-hPPARα, pGal4-hPPARγ (100 ng/puit) et le vecteur de contrôle de l'efficacité de transfection pRL-CMV (1 ng/puit) suivant le protocole décrit précédemment (Raspe, Madsen et al. 1999) et incubées pendant 36 heures avec les composés testés. A l'issue de l'expérience, les cellules sont lysées (Gibco, Paisley, UK) et les activités luciférase sont déterminées à l'aide du kit de dosage Dual-Luciferase™ Reporter Assay System (Promega, Madison, WI, USA) selon la notice du fournisseur comme décrit précédemment (Raspe, Madsen et al. 1999).

Les inventeurs mettent en évidence une augmentation de l'activité luciférase dans les cellules traitées avec les composés selon l'invention et transfectées avec le plasmide pGal4-hPPARα. Cette induction de l'activité luciférase indique que les composés selon l'invention sont des activateurs de PPARα.

Des exemples de résultats sont donnés sur les figures 2-1, 2-2, 2-3. 2-4, 2-5, 2-6 où les propriétés activatrices PPARα des composés 3, 4, 7 ,8 ,9 ,11 ,12 ,13, 14, 17, 19, 20, 21, 22, 23, 24, 25, 26, 29, 31, 33, 37, 38, 41 selon l'invention sont illustrées.

Les inventeurs mettent en évidence une augmentation de l'activité luciférase dans les cellules traitées avec les composés selon l'invention et transfectées avec le plasmide pGal4-hPPARγ. Cette induction de l'activité luciférase indique que les composés selon l'invention sont des activateurs de PPARγ.

Des exemples de résultats sont représentés par la figure 2-7 où les propriétés activatrices PPARγ des composés 17, 33 et 29 selon l'invention sont illustrées.

Un aspect de l'invention est illustré par le traitement de maladies comme l'athérosclérose et le psoriasis dont les manifestations sont respectivement d'ordre vasculaire et cutanée. Les caractéristiques de ces deux pathologies sont une inflammation systémique chronique et une prolifération cellulaire incontrôlée (cellules musculaires lisses dans le cas de l'athérosclérose et kératinocytes épidermiques dans le cas du psoriasis). Ces deux pathologies ont en commun l'expression de cytokines inflammatoires, médiée par un facteur de transcription de la réponse inflammatoire NF-kB, AP-1 et NFAT (Komuves, Hanley et al. 2000; Neve, Fruchart et al. 2000). Via une régulation négative sur la voie de signalisation NF-kB et AP-1, PPAR alpha inhibe l'expression de gènes impliqués dans la réponse inflammatoire comme celle de gènes codant pour l'interleukine-6, la cyclooxygénase-2, l'endothéline-1 et donc empêche la mobilisation des monocytes et des cellules spumeuses au niveau des lésions athéromateuses.

### EXEMPLE 3 : Evaluation des effets sur le métabolisme lipidique in vivo

Les composés selon l'invention testés sont les composés dont la préparation est décrite dans les exemples décrits ci-dessus.

Les fibrates, abondamment utilisés en clinique humaine pour le traitement des dyslipidémies impliquées dans le développement de l'athérosclérose, une des principales causes de mortalité et de morbidité dans les sociétés occidentales, sont de puissants activateurs du récepteur nucléaire PPARα. Celui-ci régule l'expression de gènes impliqués dans le transport (apolipoprotéines telles que Apo AI, Apo AII et Apo CIII, transporteurs membranaires tel que FAT) ou le catabolisme des lipides (ACO, CPT-I ou CPT-II). Un traitement par les activateurs de PPARα se traduit donc, chez l'homme et le rongeur, par une diminution des taux circulants de cholestérol et de triglycérides.

Les protocoles suivants permettent de mettre en évidence une baisse du taux de triglycérides et du taux de cholestérol circulant, ainsi que l'intérêt des composés selon l'invention dans le cadre de la prévention et/ou du traitement des maladies cardio-vasculaires.

### a) Traitement des animaux

Des souris transgéniques Apo E2/E2 sont maintenues sous un cycle lumière/obscurité de 12 heures à une température constante de 20 ± 3°C. Après une acclimatation d'une semaine, les souris sont pesées et rassemblées par groupes de 6 animaux sélectionnés de telle sorte que la distribution de leur poids corporel soit uniforme. Les composés testés sont suspendus dans la carboxyméthylcellulose et administrés par gavage intra-gastrique, à raison d'une fois par jour pendant 7 ou 8 jours, aux doses indiquées. Les animaux ont un accès libre à l'eau et à la nourriture. A l'issue de l'expérience les animaux sont pesés et sacrifiés sous anesthésie. Le sang est collecté sur EDTA. Le plasma est préparé par centrifugation à 3000 tours/minutes pendant 20 minutes. Des échantillons de foie sont prélevés et conservés congelés dans de l'azote liquide pour analyse ultérieure.

### b) Mesure des lipides et apolipoprotéines sériques

Les concentrations sériques des lipides (cholestérol total et cholestérol libre, triglycérides et phospholipides) sont mesurées par dosage colorimétrique (Boehringer, Mannheim, Allemagne) selon les indications du fournisseur. Les concentrations sériques des apolipoprotéines AI, AII et CIII sont mesurées selon les méthodes décrites précédemment (Raspé et al. J. Lipid Res. 40, 2099-2110, 1999, Asset G et al., Lipids, 34, 39-44, 1999).
Un exemple de résultats est donné sur les figures 3-1, 3-2, 3-3, 3-4, 3-5, 3-6, 3-7, 3-8, 3-9, 3-10, 3-11 et 3-12 où l'activité des composés 7, 17, 29, 33 et 41 sur le métabolisme des triglycérides et du cholestérol selon l'invention est illustrée.

### c) Analyse des ARNs

L'ARN total a été isolé des fragments de foie par extraction à l'aide du mélange thiocyanate de guanidine/phénol acide/chloroforme suivant le protocole décrit précédemment (Raspé et al. J. Lipid Res. 40, 2099-2110, 1999). Les ARN messagers ont été quantifiés par RT-PCR quantitative à l'aide du kit Light Cycler Fast Start DNA Master Sybr Green l kit (Hoffman-La Roche, Basel, Suisse) sur un appareil Light Cycler System (Hoffman-La Roche, Basel, Suisse). Des paires d'amorces spécifiques des gènes ACO, Apo CIII et Apo All ont été utilisées comme sondes. Des paires d'amorces spécifiques des gènes 36B4, β-actine et cyclophiline ont été utilisées comme sondes témoin. Alternativement, l'ARN total a été analysé par Northem Blot ou Dot Blot suivant le protocole décrit précédemment (Raspé et al. J. Lipid Res. 40, 2099-2110, 1999).

### EXEMPLE 4: Evaluation des propriétés antioxydantes des composés selon l'invention

Un aspect particulièrement avantageux de l'invention est illustré par le rôle des propriétés antioxydantes intrinsèques des composés utilisés dans les compositions selon l'invention dans le contrôle du stress oxydatif. Cette association originale entre la propriété d'agonistes de PPARα et le caractère antioxydant représente un moyen efficace pour le traitement de pathologies liées à une modification du statut redox de la cellule. Cette illustration s'applique notamment à une pathologie comme la maladie d'Alzheimer pour laquelle les radicaux libres jouent un rôle déterminant.
Chez les patients atteints de la maladie d'Alzheimer, le statut oxydatif est modifié dans les cellules du cerveau. Les radicaux libres sont ainsi responsables de la peroxydation lipidique et de l'oxydation des protéines ainsi que de celle des acides nucléiques (ADN/ARN). Ces oxydations modifient les propriétés biologiques des biomolécules et conduisent à la dégénérescence neuronale (Butterfield, Drake et al. 2001). NF-kB est connu comme un facteur de transcription sensible au statut redox des cellules. II est donc fortement impliqué dans la réponse au stress oxydatif puisqu'il permet l'activation de gènes cibles de l'inflammation (Butterfield, Drake et al. 2001). Les composés utilisés dans les compositions selon l'invention présentent donc la propriété originale d'empêcher l'activation de la voie NF-kB à deux niveaux différents, en inhibant son activation par les radicaux libres (antioxydant) mais également en prévenant son activité transcriptionnelle (agoniste PPARα).
Les composés selon l'invention constituent un moyen nouveau pour lutter contre les effets du vieillissement et plus particulièrement contre ceux du photovieillissement UV-induit où les radicaux libres participent activement à la mise en place des désordres qui vont de l'érythème cutané et la formation de rides à des pathologies plus graves comme les cancers cutanés (spino et baso-cellulaire ainsi que le mélanome).
Le métabolisme est responsable de la production de radicaux libres, mais des facteurs environnementaux comme les rayons ionisants, excitants (ultraviolets) ou les médiateurs de l'inflammation (cytokines), les agents chimiothérapeutiques, l'hyperthermie sont de puissants activateurs des espèces radicalaires et engendrent un déséquilibre de la balance redox dans la cellule. Lorsque le stress est sévère, la survie de la cellule dépend alors de sa capacité à s'adapter, à résister au stress et à dégrader les molécules endommagées. Dans le processus de vieillissement, la capacité des cellules à se défendre de manière appropriée à une attaque oxydative est capitale, aussi l'augmentation de la capacité des cellules à résister à ces attaques doit permettre d'apporter une solution pour lutter contre l'apparition des effets du vieillissement et doit favoriser une augmentation de la longévité de l'organisme.

Le rayonnement solaire peut modifier la composition de certaines molécules de l'organisme. Les UVB ont longtemps été considérés comme seuls en cause dans les effets néfastes du soleil sur l'organisme. On sait maintenant que les UVA peuvent avoir un effet néfaste direct mais surtout qu'ils potentialisent l'effet des UVB. Les principales molécules susceptibles d'être modifiées, souvent de façon néfaste mais aussi de façon bénéfique, sont :
- L'ADN, dans lequel il peut se former des dimères de thymine sous l'action des UVB. Bien que l'ADN n'absorbe pas les UVA, ces derniers peuvent causer des dommages du matériel génétique et donc être mutagènes. Une pathologie comme le *Xeroderma pigmentosum,* qui résulte de l'absence ou de l'altération des mécanismes de réparation de l'ADN, favorise l'apparition de cancers des kératinocytes basaux.
- Les protéines, qui peuvent être modifiées dans leur structure spatiale. De nombreuses protéines peuvent ainsi être inactivées : enzymes, transporteurs, canaux ioniques, protéines du cytosquelette, récepteurs. Ces modifications peuvent être induites par les UVB et les UVA.
- Les lipides, qui peuvent subir une peroxydation par les UVA, cette peroxydation étant proportionnelle au degré d'insaturation des acides gras.

Les protocoles suivants mettent en évidence les propriétés antioxydantes intrinsèques des composés utilisés dans les compositions selon l'invention pour la prévention et/ou le traitement des désordres liés au stress oxydatif.

### 1. Protection de l'oxydation des LDL par le cuivre :

Les composés selon l'invention testés sont les composés dont la préparation est décrite dans les exemples décrits ci-dessus.

L'oxydation des LDL est une modification importante et joue un rôle prépondérant dans la mise en place et le développement de l'athérosclérose (Jurgens, Hoff et al. 1987). Le protocole suivant permet la mise en évidence des propriétés antioxydantes des composés. Sauf indication contraire, les réactifs proviennent de chez Sigma (St Quentin, France).
Les LDL sont préparés suivant la méthode décrite par Lebeau et al. (Lebeau, Furman et al. 2000).
Les solutions de composés à tester sont préparées à 10⁻² M dans un tampon bicarbonate (pH = 9) et diluées dans du PBS pour avoir des concentrations finales allant de 0,1 à 100 µM pour une concentration totale d'éthanol de 1 % (v/v).

Avant l'oxydation, l'EDTA est retiré de la préparation de LDL par dialyse. L'oxydation a ensuite lieu à 30°C en ajoutant 20 µl d'une solution à 16,6 µM de CuSO₄ à 160 µL de LDL (125 µg de protéines/ml) et 20 µl d'une solution du composé à tester. La formation de diènes, l'espèce à observer, se mesure par densité optique à 234 nm dans les échantillons traités avec les composés mais avec ou sans cuivre . La mesure de la densité optique à 234 nm est réalisée toutes les 10 minutes pendant 8 heures à l'aide d'un spectrophotomètre thermostaté (Tecan Ultra 380). Les analyses sont réalisées en triplicata. Nous considérons que les composés ont une activité antioxydante lorsqu'ils induisent un retardement de la lag phase, diminuent la vitesse d'oxydation et la quantité des diènes formés par rapport à l'échantillon témoin. Les inventeurs mettent en évidence que les composés selon l'invention, présentent au moins une des propriétés antioxydantes citées ci dessus ceci indiquant que les composés selon l'invention possèdent un caractère antioxydant intrinsèque. Un exemple de résultats est donné sur les figures 1, 2 et 3 où les propriétés antioxydantes des composés 2 et 5 sont illustrées.

Des exemples de résultats sont donnés sur les figures 1-1, 1-2, 1-3,1-4,1-5, 1-6, 1-7, 1-8, 1-9, 1-10, 1-11, 1-12, 1-13 et 1-14 où les propriétés antioxydantes des composés 2, 3, 4, 5, 6, 7, 8, 9, 10, 14,17, 18, 19, 21, 22, 25, 29, 31, 33, 35, 37, 38 et 41 selon l'invention sont illustrées.

### 2 Evaluation de la protection conférée par les composés selon l'invention vis-à-vis de la peroxydation lipidique :

Les composés selon l'invention testés sont les composés dont la préparation est décrite dans les exemples décrits ci-dessus.

La mesure de l'oxydation des LDL est réalisée par la méthode des TBARS.

Selon le même principe que celui décrit précédemment, les LDL sont oxydés avec du CuSO₄ et la peroxydation lipidique est déterminée de la manière suivante :

Les TBARS sont mesurés à l'aide d'une méthode spectrophotométrique. L'hydroperoxydation lipidique est mesurée en utilisant l'oxydation péroxyde-lipide dépendante de l'iodide en iodine. Les résultats sont exprimés en nmol de malondialdehyde (MDA) ou en nmol d'hydroperoxyde/mg de protéines.

Les résultats obtenus précédemment, en mesurant l'inhibition de la formation de diènes conjugués, sont confirmés par les expériences de mesure de peroxydation lipidique des LDL. Les composés selon l'invention protègent également de manière efficace les LDL contre la peroxydation lipidique induite par le cuivre (agent oxydant).

### BIBLIOGRAPHIE

- Braissant, O. and W. Wahli (1998). "Differential expression of peroxisome proliferator-activated receptor- alpha, -beta, and -gamma during rat embryonic development." Endocrinology 139(6): 2748-54.
- Butterfield, D. A., J. Drake, et al. (2001). "Evidence of oxidative damage in Alzheimer's disease brain: central role for amyloid beta-peptide." Trends Mol Med 7(12): 548-54.
- Desvergne, B. and W. Wahli (1999). "Peroxisome proliferator-activated receptors: nuclear control of metabolism." Endocr Rev 20(5): 649-88.
- Finkel, T. and N. J. Holbrook (2000). "Oxidants, oxidative stress and the biology of ageing." Nature 408(6809): 239-47.
- Fruchart, J. C., B. Staels, et al. (2001). "PPARS, metabolic disease and atherosclerosis." Pharmacol Res 44(5): 345-52.
- Gilgun-Sherki, Y., E. Melamed, et al. (2001). "Oxidative stress induced-neurodegenerative diseases: the need for antioxidants that penetrate the blood brain barrier." Neuropharmacology 40(8): 959-75.
- Guerre-Millo, M., P. Gervois, et al. (2000). "Peroxisome proliferator-activated receptor alpha activators improve insulin sensitivity and reduce adiposity." J Biol Chem 275(22): 16638-42.
- Hourton, D., P. Delerive, et al. (2001). "Oxidized low-density lipoprotein and peroxisome-proliferator activated receptor alpha down-regulate platelet-activating-factor receptor expression in human macrophages." Biochem J 354(Pt 1): 225-32.
- Kliewer, S. A., S. S. Sundseth, et al. (1997). "Fatty acids and eicosanoids regulate gene expression through direct interactions with peroxisome proliferator-activated receptors alpha and gamma." Proc Natl Acad Sci U S A 94(9): 4318-23.
- Komuves, L. G., K. Hanley, et al. (2000). "Stimulation of PPARalpha promotes epidermal keratinocyte differentiation in vivo." J Invest Dermatol 115(3): 353-60.
- Lebeau, J., C. Furman, et al. (2000). "Antioxidant properties of di-tert-butylhydroxylated flavonoids." Free Radic Biol Med 29(9): 900-12.
- Mates, J. M., C. Perez-Gomez, et al. (1999). "Antioxidant enzymes and human diseases." Clin Biochem 32(8): 595-603.
- Morliere, P., A. Moysan, et al. (1991). "UVA-induced lipid peroxidation in cultured human fibroblasts." Biochim Biophys Acta 1084(3): 261-8.
- Neve, B. P., J. C. Fruchart, et al. (2000). "Role of the peroxisome proliferator-activated receptors (PPAR) in atherosclerosis." Biochem Pharmacol 60(8): 1245-50.
- Ram VJ (2003). "Therapeutic role of peroxisome proliferator-activated receptors in obesity, diabetes and inflammation. Prog Drug Res. 60: 93-132.Review
- Raspe, E., L. Madsen, et al. (1999). "Modulation of rat liver apolipoprotein gene expression and serum lipid levels by tetradecylthioacetic acid (TTA) via PPARalpha activation." J Lipid Res 40(11): 2099-110.
- Staels, B. and J. Auwerx (1998). "Régulation of apo A-l gene expression by fibrates." Atherosclerosis 137 Suppl: S19-23.

## Revendications

1. Composition destinée au traitement ou à la prophylaxie d'une pathologie liée à l'inflammation, à la neurodégénérescence, aux dérèglements du métabolisme lipidique et/ou glucidique, à la prolifération et/ou à la différentiation cellulaire et/ou au vieillissement cutané ou du système nerveux central, comprenant, dans un support acceptable sur le plan pharmaceutique, au moins un dérivé de 1,3-diphénylprop-2-èn-1-one substitué de formule (I) suivante : 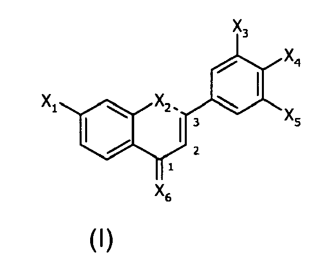 dans laquelle :
X1 représente un halogène ou un groupement -R1 ou un groupement répondant à la formule suivante : -G1-R1,
X2 représente un atome d'hydrogène ou un groupement thionitroso ou un groupement hydroxy ou un groupement alkylcarbonyloxy ou un groupement alkyloxy non substitué ou un groupement thiol ou un groupement alkylthio ou un groupement alkylcarbonylthio, X2 peut également représenter un atome d'oxygène ou de soufre lié au carbone 3 de la chaîne propène, pour former un dérivé de type 2-phényl-4H-1-benzopyran-4-one ou de type 2-phényl-4H-1-benzothiopyran-4-one,
X3 représente un groupement -R3 ou un groupement répondant à la formule suivante : -G3-R3,
X4 représente un halogène ou un groupement thionitroso ou un groupement -R4 ou un groupement répondant à la formule suivante : -G4-R4,
X5 représente un groupement -R5 ou un groupement répondant à la formule suivante : -G5-R5,
X6 est un atome d'oxygène,
R1, R3, R4, R5, identiques ou différents, représentent un atome d'hydrogène ou un groupement alkyle substitué ou non par un groupement faisant partie du groupe 1 ou du groupe 2 définis ci-dessous,
G1,G3, G4, G5, identiques ou différents, représentent un atome d' oxygène ou de soufre,
avec au moins un des groupements X1, X3, X4 ou X5 répondant à la formule -G-R, dans laquelle G représente un atome de soufre, et
avec au moins un des groupements R1, R3, R4 ou R5 présent sous la forme d'un radical alkyle portant au moins un substituant du groupe 1 ou 2, ledit radical alkyle étant lié directement au cycle ou étant associé à un groupement G selon la formule - G-R,
les substituants du groupe 1 sont choisis parmi les groupements carboxy de formule : -COOR₆ et les groupements carbamoyle de formule : -CONR₆R₇,
les substituants du groupe 2 sont choisis parmi l'acide sulfonique (SO₃H) et les groupements sulfonamide de formule : -SO₂NR₆R₇,
avec R₆ et R₇, identiques ou différents, représentant un atome d'hydrogène ou un radical alkyle éventuellement substitué par au moins un groupe de type 1 ou 2,
à l'exclusion des composés de formule (I) dans laquelle :
X₂ représente un atome d'hydrogène et X₁ représente -G1-R1 où G1 représente un atome d'oxygène et R1 représente CH2COOH,
leurs isomères optiques et géométriques, leurs racémates, leurs tautomères, leurs sels, leurs hydrates et leurs mélanges.

2. Composition destinée au traitement ou à la prophylaxie d'une pathologie liée à l'inflammation, à la neurodégénérescence, aux dérèglements du métabolisme lipidique et/ou glucidique, à la prolifération et/ou à la différentiation cellulaire et/ou au vieillissement cutané ou du système nerveux central, comprenant, dans un support acceptable sur le plan pharmaceutique, au moins un dérivé de 1,3-diphénylprop-2-èn-1-one substitué de formule (I) suivante : 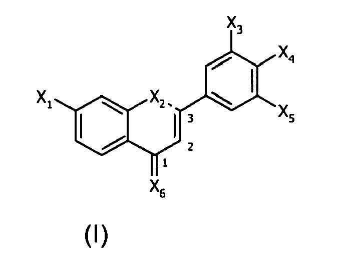 dans laquelle :
X1 représente un halogène ou un groupement -R1 ou un groupement répondant à la formule suivante : -G1-R1,
X2 représente un atome d'hydrogène ou un groupement thionitroso ou un groupement hydroxy ou un groupement alkylcarbonyloxy ou un groupement alkyloxy non substitué ou un groupement thiol ou un groupement alkylthio ou un groupement alkylcarbonylthio, X2 peut également représenter un atome de soufre lié au carbone 3 de la chaîne propène, pour former un dérivé de type 2-phényl-4H-1-benzothiopyran-4-one
X3 représente un groupement -R3 ou un groupement répondant à la formule suivante : -G3-R3,
X4 représente un halogène ou un groupement thionitroso ou un groupement -R4 ou un groupement répondant à la formule suivante : -G4-R4,
X5 représente un groupement -R5 ou un groupement répondant à la formule suivante : -G5-R5,
X6 est un atome d'oxygène,
R1, R3, R4, R5, identiques ou différents, représentent un atome d'hydrogène ou un groupement alkyle substitué ou non par un groupement faisant partie du groupe 1 ou du groupe 2 définis ci-dessous,
G1, G3, G4, G5, identiques ou différents, représentent un atome d'oxygène ou de soufre,
avec au moins un des groupements X1, X3, X4 ou X5 répondant à la formule -G-R,
avec aucun des groupements X3, X4 ou X5 ne représentant un atome d'hydrogène, et
avec au moins un des groupements R1, R3, R4 ou R5 présent sous la forme d'un radical alkyle portant au moins un substituant du groupe 1 ou 2, ledit radical alkyle étant lié directement au cycle ou étant associé à un groupement G selon la formule -G-R,
les substituants du groupe 1 sont choisis parmi les groupements carboxy de formule : -COOR₆ et les groupements carbamoyle de formule : -CONR₆R₇,
les substituants du groupe 2 sont choisis parmi l'acide sulfonique (SO₃H) et les groupements sulfonamide de formule : -SO₂NR₆R₇,
avec R₆ et R₇, identiques ou différents, représentant un atome d'hydrogène ou un radical alkyle éventuellement substitué par au moins un groupe de type 1 ou 2,
à l'exclusion des composés de formule (1) dans laquelle :
X₂ représente un atome d'hydrogène et X₁ représente -G1-R1 où G1 représente un atome d'oxygène et R1 représente CH2COOH,
leurs isomères optiques et géométriques, leurs racémates, leurs tautomères, leurs sels, leurs hydrates et leurs mélanges.

3. Composition selon la revendication 1 ou 2, **caractérisée en ce que** les dérivés peuvent correspondre à la conformation cis, trans ou leur mélange.

4. Composition selon la revendication 1, **caractérisée en ce qu'**aucun des groupements X3, X4 et X5 ne représente un atome d'hydrogène.

5. Composition selon la revendication 1, **caractérisée en ce qu'**un ou deux des groupements X3, X4 et X5 représente un atome d'hydrogène.

6. Composition selon l'une des revendications précédentes, **caractérisée en ce que** les deux groupements G1 et G4 représentent un atome de soufre.

7. Composition selon l'une des revendications précédentes, **caractérisée en ce que** X2 est un atome d'hydrogène, un groupement thionitroso ou un groupement hydroxy ou un groupement alkyloxy ou un groupement thiol ou un groupement alkylthio.

8. Composition selon l'une des revendications précédentes, **caractérisée en ce que** X4 représente un groupement thionitroso ou un groupement -R4 ou un groupement de formule -G4-R4 et X2 est un groupement thionitroso ou un groupement hydroxy, alkyloxy, thiol ou alkylthio, G4 et R4 étant tels que définis dans la revendication 1 ou 2.

9. Composition selon l'une des revendications précédentes, **caractérisée en ce que** X1 représente un groupement -R1 ou un groupement répondant à la formule -G1-R1, R1 étant un groupement alkyle substitué par un groupement faisant partie du groupe 1, G1 et le substituant du groupe 1 étant tels que définis à la revendication 1 ou 2.

10. Composition selon l'une des revendications précédentes, **caractérisée en ce que** X1 est un groupement -G1-R1, G1 et R1 étant tels que définis à la revendication 1 ou 2.

11. Composition selon l'une des revendications précédentes, **caractérisée en ce que** X1 est un groupement -G1-R1 dans lequel G1 est un atome d'oxygène, R1 étant tel que défini à la revendication 1 ou 2.

12. Composition selon l'une des revendications précédentes, **caractérisée en ce que** X1 représente un groupement -R1 ou un groupement répondant à la formule -G1-R1, R1 étant un groupement alkyle substitué par un groupement faisant partie du groupe 2, G1 et le substituant du groupe 2 étant tels que définis à la revendication 1 ou 2.

13. Composition selon l'une des revendications précédentes, **caractérisée en ce que** X3 représente un groupement -R3 ou un groupement répondant à la formule -G3-R3, R3 étant un groupement alkyle substitué par un groupement faisant partie du groupe 1, G3 et le substituant du groupe 1 étant tels que définis à la revendication 1 ou 2.

14. Composition selon l'une des revendications précédentes, **caractérisée en ce que** X3 représente un groupement -R3 ou un groupement répondant à la formule -G3-R3, R3 étant un groupement alkyle substitué par un groupement faisant partie du groupe 2, G3 et le substituant du groupe 2 étant tels que définis à la revendication 1 ou 2.

15. Composition selon l'une des revendications précédentes, **caractérisée en ce que** X4 représente un groupement -R4 ou un groupement répondant à la formule -G4-R4, R4 étant un groupement alkyle substitué par un groupement faisant partie du groupe 1, G4 et le substituant du groupe 1 étant tels que définis à la revendication 1 ou 2.

16. Composition selon l'une des revendications précédentes, **caractérisée en ce que** X4 est un groupement -G4-R4, G4 et R4 étant tels que définis à la revendication 1 ou 2.

17. Composition selon l'une des revendications précédentes, **caractérisée en ce que** X4 est un groupement -G4-R4 dans lequel G4 est un atome d'oxygène, R4 étant tel que défini à la revendication 1 ou 2.

18. Composition selon l'une des revendications précédentes, **caractérisée en ce que** X4 est un groupement -G4-R4 dans lequel G4 est un atome d'oxygène, et X3 ou X5 représente respectivement R3 ou -G3-R3, d'une part, et R5 ou -G5-R5, d'autre part, R3 ou R5 étant des groupements alkyle portant un substituant du groupe 1, R4, G3, G5 et le substituant du groupe 1 étant tels que définis à la revendication 1 ou 2.

19. Composition selon l'une des revendications précédentes, **caractérisée en ce que** X4 représente un groupement -R4 ou un groupement répondant à la formule -G4-R4, R4 étant un groupement alkyle substitué par un groupement faisant partie du groupe 2, G4 et le substituant du groupe 2 étant tels que définis à la revendication 1 ou 2.

20. Composition selon l'une des revendications précédentes, **caractérisée en ce que** X1 représente un halogène.

21. Composition selon l'une des revendications précédentes, **caractérisée en ce que** X1, X3, X4 ou X5 représente OC(CH3)2COOR6, R6 étant tel que défini à la revendication 1 ou 2.

22. Composition selon l'une des revendications précédentes, **caractérisée en ce que** X1, X3, X4 ou X5 représente SC(CH3)2COOR6, R6 étant tel que défini à la revendication 1 ou 2.

23. Composition selon l'une quelconque des revendications précédentes, **caractérisée en ce que** le dérivé est choisi parmi :
1-[2-hydroxy-4-carboxydiméthylméthyloxyphényl]-3-[3,5-di*tert*butyl-4-hydroxyphényl]prop-2-èn-1-one,
1-[2-hydroxy-4-éthoxycarbonyldiméthylméthyloxyphényl]-3-[3,5-di*tert*butyl-4-hydroxyphényl]prop-2-èn-1-one,
1-[2-hydroxy-4-isopropyloxycarbonyldiméthylméthyloxyphényl]-3-[3,5-di*tert*butyl-4-hydroxyphényl]prop-2-èn-1-one
1-[2-hydroxyphényl]-3-[3-carboxydiméthylméthyloxy-4-hydroxy-5-*tert*butylphényl]prop-2-èn-1-one,
1-[2-hydroxyphényl]-3-[3-*iso*propyloxycarbonyldiméthylméthyloxy-4-hydroxy-5-*tert*butylphényl]prop-2-èn-1-one,
1-[2-hydroxy-4-chlorophényl]-3-[3-carboxydiméthylméthyloxy-4-hydroxy-5-*tert*butylphényl]prop-2-èn-1-one,
1-[2-hydroxy-4-chlorophényl]-3-[3-*iso*propyloxycarbonyldiméthylméthyloxy-4-hydroxy-5-*tert*butylphényl]prop-2-èn-1-one,
1-[2-hydroxyphényl]-3-[3-carboxydiméthylméthyl-4-hydroxy-5-*tert*butylphényl]prop-2-èn-1-one,
1-[2-hydroxyphényl]-3-[3-*iso*propyloxycarbonyldiméthylméthyl-4-hydroxy-5-*tert*butylphényl]prop-2-èn-1-one,
1-[2-hydroxy-4-chlorophényl]-3-[3-carboxydiméthylméthyl-4-hydroxy-5-*tert*butylphényl]prop-2-èn-1-one,
1-[2-hydroxy-4-chlorophényl]-3-[3-*iso*propyloxycarbonyldiméthylméthyl-4-hydroxy-5-*tert*butylphényl]prop-2-èn-1-one,
1-[2-hydroxy-4-chlorophényl]-3-[3,5-diméthoxy-4-carboxydiméthylméthyloxyphényl]prop-2-èn-1-one,
1-[2-hydroxy-4-chlorophényl]-3-[3,5-diméthoxy-4-*iso*propyloxycarbonyldiméthylméthyloxyphényl]prop-2-èn-1-one,
1-[2-hydroxyphényl]-3-[3,5-diméthoxy-4-carboxydiméthylméthyloxyphényl]prop-2-èn-1-one,
1-[2-hydroxyphényl]-3-[3,5-d iméthoxy-4-*iso*propyloxycarbonyl diméthylméthyloxyphényl]prop-2-èn-1-one,
1-[2-hydroxy-4-carboxydiméthylméthyloxyphényl]-3-[3,5-diméthoxy-4-hydroxyphényl]prop-2-èn-1-one,
1-[2-hydroxy-4-*iso*propyloxycarbonyldiméthylméthyloxyphényl]-3-[3,5-diméthoxy-4-hydroxyphényl]prop-2-èn-1-one,
1-[2-hydroxy-4-chlorophényl]-3-[3,4-dihydroxy-5-carboxydiméthylméthyloxyphényl]prop-2-èn-1-one,
1-[2-hydroxy-4-chlorophényl]-3-[3,4-dihydroxy-5-*iso*propyloxycarbonyldiméthylméthyloxyphényl]prop-2-èn-1-one,
1-[2-hydroxy-4-carboxydiméthylméthyloxyphényl]-3-[3,5-diméthyl-4-hydroxyphényl]prop-2-èn-1-one,
1-[2-hydroxy-4-*iso*propyloxycarbonyldiméthylméthyloxyphényl]-3-[3,5-diméthyl-4-hydroxyphényl]prop-2-èn-1-one,
1-[2-hydroxy-4-chlorophényl]-3-[3,5-diméthyl-4-carboxydiméthylméthyloxyphényl]prop-2-èn-1-one,
1-[2-hydroxy-4-chlorophényl]-3-[3,5-diméthyl-4-*iso*propyloxycarbonyldiméthylméthyloxyphényl]prop-2-èn-1-one,
1-[2-hydroxyphényl]-3-[3,5-diméthyl-4-carboxydiméthylméthyloxyphényl]prop-2-èn-1-one,
1-[2-hydroxyphényl]-3-[3,5-diméthyl-4-*iso*propyloxycarbonyldiméthylméthyloxyphényl]prop-2-èn-1-one,
1-[2-hydroxyphényl]-3-[4-carboxydiméthylméthylthiophényl]prop-2-èn-1-one,
1-[2-hydroxyphényl]-3-[4-*iso*propyloxycarbonyldiméthylméthylthiophényl]prop-2-èn-1-one,
1-[2-hydroxy-4-carboxydiméthylméthyloxyphényl]-3-[4-méthylthiophényl]prop-2-èn-1-one,
1-[4-chlorophényl]-3-[3,5-diméthyl-4-tertiobutyloxycarbonyldiméthylméthyloxyphényl]prop-2-èn-1-one,
1-[4-chlorophényl]-3-[3,5-diméthyl-4-isopropyloxycarbonyldiméthylméthyloxyphényl]prop-2-èn-1-one,
1-[4-chlorophényl]-3-[3,5-diméthyl-4-carboxydiméthylméthyloxyphényl]prop-2-èn-1-one,
1-[4-chloro-2-hydroxyphényl]-3-[4-carboxydiméthylméthylthiophényl]prop-2-èn-1-one,
1-[4-carboxydiméthylméthyloxyphényl]-3-[3,5-diméthyl-4-hydroxyphényl]prop-2-èn-1-one,
1-[4-méthylthiophényl]-3-[4-carboxydiméthylméthyloxyphényl]prop-2-èn-1-one,
1-[4-carboxydiméthylméthylthiophényl]-3-[4-méthylthiophényl]prop-2-èn-1-one,
1-[2-hydroxy-4-bromophényl]-3-[3,5-diméthyl-4-carboxydiméthylméthyloxyphényl]prop-2-èn-1-one,
1-[4-carboxydiméthylméthyloxyphényl]-3-[4-méthylthiophényl]prop-2-èn-1-one,
1-[4-méthylthiophényl]-3-[3,5-diméthyl-4-tertiobutyloxycarbonyldiméthylméthyloxyphényl]prop-2-èn-1-one,
1-[4-méthylthiophényl]-3-[3,5-diméthyl-4-isopropyloxycarbonyldiméthylméthyloxyphényl]prop-2-èn-1-one,
1-[4-méthylthiophényl]-3-[3,5-diméthyl-4-carboxydiméthylméthyloxyphényl]prop-2-èn-1-one,
1-[2-méthoxyphényl]-3-[3,5-diméthyl-4-tertiobutyloxycarbonyldiméthylméthyloxyphényl]prop-2-èn-1-one,
1-[2-méthoxyphényl]-3-[3,5-diméthyl-4-carboxydiméthylméthyloxyphényl]prop-2-èn-1-one,
1-[4-hexyloxyphényl]-3-[3,5-diméthyl-4-tertiobutyloxycarbonyldiméthylméthyloxyphényl]prop-2-èn-1-one,
1-[4-hexyloxyphényl]-3-[3,5-diméthyl-4-carboxydiméthylméthyloxyphényl]prop-2-èn-1-one,
1-[2-méthyloxy-4-chlorophényl]-3-[3,5-diméthyl-4-tertiobutyloxycarbonyldiméthylméthyloxyphényl]prop-2-èn-1-one,
1-[2-méthyloxy-4-chlorophényl]-3-[3,5-diméthyl-4-carboxydiméthylméthyloxyphényl]prop-2-èn-1-one,
1-[4-heptylphényl]-3-[3,5-diméthyl-4-tertiobutyloxycarbonyldiméthylméthyloxyphényl]prop-2-èn-1-one,
1-[4-heptylphényl]-3-[3,5-diméthyl-4-carboxydiméthylméthyloxyphényl]prop-2-èn-1-one,
1-[4-bromophényl]-3-[3,5-diméthyl-4-tertiobutyloxycarbonyldiméthylméthyloxyphényl]prop-2-èn-1-one,
1-[4-bromophényl]-3-[3,5-diméthyl-4-carboxydiméthylméthyloxyphényl]prop-2-èn-1-one.

24. Composition selon l'une quelconque des revendications précédentes, **caractérisée en ce que** le dérivé est choisi parmi :
1-[4-méthylthiophényl]-3-[3,5-diméthyl-4-carboxydiméthylméthyloxyphényl]prop-2-èn-1-one,
1-[4-hexyloxyphényl]-3-[3,5-diméthyl-4-carboxydiméthylméthyloxyphényl]prop-2-èn-1-one, et
1-[4-bromophényl]-3-[3,5-diméthyl-4-carboxydiméthylméthyloxyphényl]prop-2-èn-1-one.

25. Composition selon l'une quelconque des revendications précédentes **caractérisée en ce que** le dérivé est la 1-[4-méthylthiophényl]-3-[3,5-diméthyl-4-carboxydiméthylméthyloxyphényl]prop-2-èn-1-one.

26. Composition selon l'une quelconque des revendications 1 à 25, **caractérisée en ce que** la pathologie liée à l'inflammation est choisie parmi l'athérosclérose, une allergie, l'asthme, l'eczéma, le psoriasis et les démangeaisons.

27. Composition selon l'une des revendications 1 à 25, **caractérisée en ce que** la pathologie liée à la neurodégénérescence est la maladie d'Alzheimer ou la maladie de Parkinson.

28. Composition selon l'une des revendications 1 à 25, **caractérisée en ce que** la pathologie liée aux dérèglements du métabolisme lipidique et/ou glucidique est choisie parmi le diabète, l'athérosclérose et l'obésité.

29. Composition selon l'une des revendications 1 à 25, **caractérisée en ce que** la pathologie liée à la prolifération et/ou à la différentiation cellulaire est choisie parmi la carcinogenèse, le psoriasis et l'athérosclérose.

## Claims

1. Composition for the treatment or prophylaxis of a pathology related to inflammation, neurodegeneration, deregulations of lipid and/or glucose metabolism, cell proliferation and/or differentiation and/or skin or central nervous system ageing, comprising, in a pharmaceutically acceptable support, at least one substituted 1,3-diphenylprop-2-en-1-one derivative represented by formula (I) below : 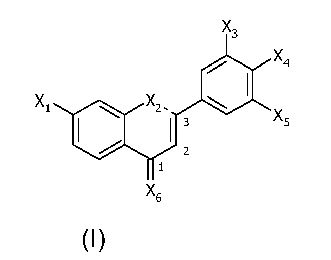 wherein :
X1 represents a halogen or a -R1 group or a group corresponding to the following formula : -G1-R1,
X2 represents a hydrogen atom or a thionitroso group or a hydroxy group or an alkylcarbonyloxy group or an unsubstituted alkyloxy group or a thiol group or an alkylthio group or an alkylcarbonylthio group, X2 can also represent an oxygen or sulphur atom bound to carbon 3 of the propene chain, so as to form a derivative of the type 2-phenyl-4H-1-benzopyran-4-one or of the type 2-phenyl-4H-1-benzothiopyran-4-one,
X3 represents a -R3 group or a group corresponding to the following formula : -G3-R3,
X4 represents a halogen or a thionitroso group or a -R4 group or a group corresponding to the following formula : -G4-R4,
X5 represents a -R5 group or a group corresponding to the following formula : -G5-R5,
X6 is an oxygen atom,
R1, R3, R4, R5, which are the same or different, represent a hydrogen atom or an alkyl group substituted or not by a substituent which is part of group 1 or group 2 defined hereinbelow,
G1, G3, G4, G5, which are the same or different, represent an oxygen or sulfur atom,
with at least one of the groups X1, X3, X4 or X5 corresponding to the formula -G-R, in which G is a sulphur atom, and
with at least one of the groups R1, R3, R4 or R5 present in the form of an alkyl group containing at least one substituent from group 1 or 2, said alkyl group being bound directly to the ring or being associated with a group G according to the formula -G-R,
the substituents from group 1 are selected in the group consisting of carboxy groups having the formula : -COOR₆ and carbamoyl groups having the formula : -CONR₆R₇,
the substituents from group 2 are selected in the group consisting of sulfonic acid (SO₃H) and sulfonamide groups having the formula : -SO₂NR₆R₇,
with R₆ and R₇, which are the same or different, representing a hydrogen atom or an alkyl group possibly substituted by at least one group of type 1 or 2,
with the exception of compounds represented by formula (I) in which X₂ represents a hydrogen atom and X₁ represents -G1-R1 where G1 represents an oxygen atom and R1 represents CH2COOH,
the optical and geometrical isomers, racemates, tautomers, salts, hydrates and mixtures thereof.

2. Composition for the treatment or prophylaxis of a pathology related to inflammation, neurodegeneration, deregulations of lipid and/or glucose metabolism, cell proliferation and/or differentiation and/or skin or central nervous system ageing, comprising, in a pharmaceutically acceptable support, at least one substituted 1,3-diphenylprop-2-en-1-one derivative represented by formula (I) below : 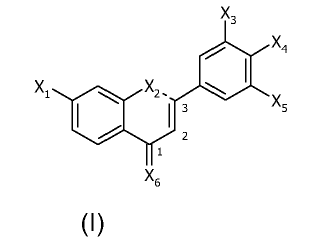 wherein :
X1 represents a halogen or a -R1 group or a group corresponding to the following formula : -G1-R1,
X2 represents a hydrogen atom or a thionitroso group or a hydroxy group or an alkylcarbonyloxy group or an unsubstituted alkyloxy group or a thiol group or an alkylthio group or an alkylcarbonylthio group, X2 can also represent a sulphur atom bound to carbon 3 of the propene chain, so as to form a derivative of the type 2-phenyl-4H-1-benzothiopyran-4-one,
X3 represents a -R3 group or a group corresponding to the following formula : -G3-R3,
X4 represents a halogen or a thionitroso group or a -R4 group or a group corresponding to the following formula : -G4-R4,
X5 represents a -R5 group or a group corresponding to the following formula : -G5-R5,
X6 is an oxygen atom,
R1, R3, R4, R5, which are the same or different, represent a hydrogen atom or an alkyl group substituted or not by a substituent which is part of group 1 or group 2 defined hereinbelow,
G1, G3, G4, G5, which are the same or different, represent an oxygen or sulfur atom,
with at least one of the groups X1, X3, X4 or X5 corresponding to the formula -G-R,
with none of the groups X3, X4 or X5 representing a hydrogen atom,
with at least one of the groups R1, R3, R4 or R5 present in the form of an alkyl group containing at least one substituent from group 1 or 2, said alkyl group being bound directly to the ring or being associated with a group G according to the formula -G-R,
the substituents from group 1 are selected in the group consisting of carboxy groups having the formula : -COOR₆ and carbamoyl groups having the formula : -CONR₆R₇,
the substituents from group 2 are selected in the group consisting of sulfonic acid (SO₃H) and sulfonamide groups having the formula : -SO₂NR₆R₇
with R₆ and R₇, which are the same or different, representing a hydrogen atom or an alkyl group possibly substituted by at least one group of type 1 or 2,
with the exception of compounds represented by formula (I) in which X₂ represents a hydrogen atom and X₁ represents -G1-R1 where G1 represents an oxygen atom and R1 represents CH2COOH,
the optical and geometrical isomers, racemates, tautomers, salts, hydrates and mixtures thereof.

3. Composition according to claim 1 or 2, **characterized in that** the derivatives can correspond to the cis or trans conformation or a mixture thereof.

4. Composition according to claim 1, **characterized in that** none of the groups X3, X4 and X5 represents a hydrogen atom.

5. Composition according to claim 1, **characterized in that** one or two of the groups X3, X4 and X5 represents a hydrogen atom.

6. Composition according to any one of the preceding claims, **characterized in that** both G1 and G4 groups represent a sulphur atom.

7. Composition according to any one of the preceding claims, **characterized in that** X2 represent a hydrogen atom, a thionitroso group or a hydroxy group or an alkyloxy group or a thiol group or an alkylthio group.

8. Composition according to any one of the preceding claims, **characterized in that** X4 represents a thionitroso group or a -R4 group or a group corresponding to the formula -G4-R4 and X2 is a thionitroso group or a hydroxy, alkyloxy, thiol or alkylthio group, G4 and R4 being such as defined in claim 1 or 2.

9. Composition according to any one of the preceding claims, **characterized in that** X1 represents a -R1 group or a group corresponding to the formula -G1-R1, R1 being an alkyl group substituted by a substituent which is part of group 1, G1 and the substituent from group 1 being such as defined in claim 1 or 2.

10. Composition according to any one of the preceding claims, **characterized in that** X1 is a -G1-R1 group, G1 and R1 being such as defined in claim 1 or 2.

11. Composition according to any one of the preceding claims, **characterized in that** X1 is a -G1-R1 group in which G1 is an oxygen atom, R1 being such as defined in claim 1 or 2.

12. Composition according to any one of the preceding claims, **characterized in that** X1 represents a -R1 group or a group corresponding to the formula -G1-R1, R1 being an alkyl group substituted by a substituent which is part of group 2, G1 and the substituent from group 2 being such as defined in claim 1 or 2.

13. Composition according to any one of the preceding claims, **characterized in that** X3 represents a -R3 group or a group corresponding to the formula -G3-R3, R3 being an alkyl group substituted by a substituent which is part of group 1, G3 and the substituent from group 1 being such as defined in claim 1 or 2.

14. Composition according to any one of the preceding claims, **characterized in that** X3 represents a -R3 group or a group corresponding to the formula -G3-R3, R3 being an alkyl group substituted by a substituent which is part of group 2, G3 and the substituent from group 2 being such as defined in claim 1 or 2.

15. Composition according to any one of the preceding claims, **characterized in that** X4 represents a -R4 group or a group corresponding to the formula -G4-R4, R4 being an alkyl group substituted by a substituent which is part of group 1, G4 and the substituent from group 1 being such as defined in claim 1 or 2.

16. Composition according to any one of the preceding claims, **characterized in that** X4 is a -G4-R4 group, G4 and R4 being such as defined in claim 1 or 2.

17. Composition according to any one of the preceding claims, **characterized in that** X4 is a -G4-R4 group in which G4 is an oxygen atom, R4 being such as defined in claim 1 or 2.

18. Composition according to any one of the preceding claims, **characterized in that** X4 is a -G4-R4 group in which G4 is an oxygen atom, and X3 or X5 respectively represent R3 or -G3-R3, on the one hand, and R5 or -G5-R5, on the other hand, R3 and R5 being alkyl groups having a substituent from group 1, R4, G3, G5 and the substituent from group 1 being such as defined in claim 1 or 2.

19. Composition according to any one of the preceding claims, **characterized in that** X4 represents a -R4 group or a group corresponding to the formula -G4-R4, R4 being an alkyl group substituted by a substituent which is part of group 2, G4 and the substituent from group 2 being such as defined in claim 1 or 2.

20. Composition according to any one of the preceding claims, **characterized in that** X1 represents a halogen.

21. Composition according to any one of the preceding claims, **characterized in that** X1, X3, X4 or X5 represents OC(CH3)2COOR6, R6 being such as defined in claim 1 or 2.

22. Composition according to any one of the preceding claims, **characterized in that** X1, X3, X4 or X5 represents SC(CH3)2COOR6, R6 being such as defined in claim 1 or 2.

23. Composition according to any one of the previous claims, **characterized in that** the derivative is selected in the group consisting of
1-[2-hydroxy-4-carboxydimethylmethyloxyphenyl]-3-[3,5-di*tert*butyl-4-hydroxyphenyl]prop-2-en-1-one,
1-[2-hydroxy-4-ethoxycarbonyldimethylmethyloxyphenyl]-3-[3,5-di*tert*butyl-4-hydroxyphenyl]prop-2-en-1-one,
1-[2-hydroxy-4-isopropyloxycarbonyldimethylmethyloxyphenyl]-3-[3,5-di*tert*butyl-4-hydroxyphenyl]prop-2-en-1-one,
1-[2-hydroxyphenyl]-3-[3-carboxydimethylmethyloxy-4-hydroxy-5-*tert*butylphenyl]prop-2-en-1-one,
1-[2-hydroxyphenyl]-3-[3-*iso*propyloxycarbonyldimethylmethyloxy-4-hydroxy-5-*tert*butylphenyl]prop-2-en-1-one,
1-[2-hydroxy-4-chlorophenyl]-3-[3-carboxydimethylmethyloxy-4-hydroxy-5-*tert*butylphenyl]prop-2-en-1-one,
1-[2-hydroxy-4-chlorophenyl]-3-[3-*iso*propyloxycarbonyldimethylmethyloxy-4-hydroxy-5-*tert*butylphenyl]prop-2-en-1-one,
1-[2-hydroxyphenyl]-3-[3-carboxydimethylmethyl-4-hydroxy-5-*tert*butylphenyl]prop-2-en-1-one,
1-[2-hydroxyphenyl]-3-[3-*iso*propyloxycarbonyldimethylmethyl-4-hydroxy-5-*tert*butylphenyl]prop-2-en-1-one,
1-[2-hydroxy-4-chlorophenyl]-3-[3-carboxydimethylmethyl-4-hydroxy-5-*tert*butylphenyl]prop-2-en-1-one,
1-[2-hydroxy-4-chlorophenyl]-3-[3-*iso*propyloxycarbonyldimethylmethyl-4-hydroxy-5-*tert*butylphenyl]prop-2-en-1-one,
1-[2-hydroxy-4-chlorophenyl]-3-[3,5-dimethoxy-4-carboxydimethylmethyloxyphenyl]prop-2-en-1-one,
1-[2-hydroxy-4-chlorophenyl]-3-[3,5-dimethoxy-4-*iso*propyloxycarbonyldimethylmethyloxyphenyl]prop-2-en-1-one,
1-[2-hydroxyphenyl]-3-[3,5-dimethoxy-4-carboxydimethylmethyloxyphenyl]prop-2-en-1-one,
1-[2-hydroxyphenyl]-3-[3,5-dimethoxy-4-*iso*propyloxycarbonyl dimethylmethyloxyphenyl]prop-2-en-1-one,
1-[2-hydroxy-4-carboxydimethylmethyloxyphenyl]-3-[3,5-dimethoxy-4-hydroxyphenyl]prop-2-en-1-one,
1-[2-hydroxy-4-*iso*propyloxycarbonyldimethylmethyloxyphenyl]-3-[3,5-dimethoxy-4-hydroxyphenyl]prop-2-en-1-one,
1-[2-hydroxy-4-chlorophenyl]-3-[3,4-dihydroxy-5-carboxydimethylmethyloxyphenyl]-2-prop-2-en-1-one,
1-[2-hydroxy-4-chlorophenyl]-3-[3,4-dihydroxy-5-*iso*propyloxycarbonyldimethylmethyloxyphenyl]prop-2-en-1-one,
1-[2-hydroxy-4-carboxydimethylmethyloxyphenyl]-3-[3,5-dimethyl-4-hydroxyphenyl]prop-2-en-1-one,
1-[2-hydroxy-4-*iso*propyloxycarbonyldimethylmethyloxyphenyl]-3-[3,5-dimethyl-4-hydroxyphenyl]prop-2-en-1-one,
1-[2-hydroxy-4-chlorophenyl]-3-[3,5-dimethyl-4-carboxydimethylmethyloxyphenyl]prop-2-en-1-one,
1-[2-hydroxy-4-chlorophenyl]-3-[3,5-dimethyl-4-*iso*propyloxycarbonyldimethylmethyloxyphenyl]prop-2-en-1-one,
1-[2-hydroxyphenyl]-3-[3,5-dimethyl-4-carboxydimethylmethyloxyphenyl]prop-2-en-1-one,
1-[2-hydroxyphenyl]-3-[3,5-dimethyl-4-*iso*propyloxycarbonyldimethylmethyloxyphenyl]prop-2-en-1-one,
1-[2-hydroxyphenyl]-3-[4-carboxydimethylmethylthiophenyl]prop-2-en-1-one,
1-[2-hydroxyphenyl]-3-[4-*iso*propyloxycarbonyldimethylmethylthiophenyl]prop-2-en-1-one,
1-[2-hydroxy-4-carboxydimethylmethyloxyphenyl]-3-[4-methylthiophenyl]prop-2-en-1-one,
1-[4-chlorophenyl]-3-[3,5-dimethyl-4-tertiobutyloxycarbonyldimethylmethyloxyphenyl]prop-2-en-1-one,
1-[4-chlorophenyl]-3-[3,5-dimethyl-4-isopropyloxycarbonyldimethylmethyloxyphenyl]prop-2-en-1-one,
1-[4-chlorophenyl]-3-[3,5-dimethyl-4-carboxydimethylmethyloxyphenyl]prop-2-en-1-one,
1-[4-chloro-2-hydroxyphenyl]-3-[4-carboxydimethylmethylthiophenyl]prop-2-en-1-one,
1-[4-carboxydimethylmethyloxyphenyl]-3-[3,5-dimethyl-4-hydroxyphenyl]prop-2-en-1-one,
1-[4-methylthiophenyl]-3-[4-carboxydimethylmethyloxyphenyl]prop-2-en-1-one,
1-[4-carboxydimethylmethylthiophenyl]-3-[4-methylthiophenyl]prop-2-en-1-one,
1-[2-hydroxy-4-bromophenyl]-3-[3,5-dimethyl-4-carboxydimethylmethyloxyphenyl]prop-2-en-1-one,
1-[4-carboxydimethylmethyloxyphenyl]-3-[4-methylthiophenyl]prop-2-en-1-one,
1-[4-methylthiophenyl]-3-[3,5-dimethyl-4-tertiobutyloxycarbonyldimethylmethyloxyphenyl]prop-2-en-1-one,
1-[4-methylthiophenyl]-3-[3,5-dimethyl-4-isopropyloxycarbonyldimethylmethyloxyphenyl]prop-2-en-1-one,
1-[4-methylthiophenyl]-3-[3,5-dimethyl-4-carboxydimethylmethyloxyphenyl]prop-2-en-1-one,
1-[2-methoxyphenyl]-3-[3,5-dimethyl-4-tertiobutyloxycarbonyldimethylmethyloxyphenyl]prop-2-en-1-one,
1-[2-methoxyphenyl]-3-[3,5-dimethyl-4-carboxydimethylmethyloxyphenyl]prop-2-en-1-one,
1-[4-hexyloxyphenyl]-3-[3,5-dimethyl-4-tertiobutyloxycarbonyldimethylmethyloxyphenyl]prop-2-en-1-one,
1-[4-hexyloxyphenyl]-3-[3,5-dimethyl-4-carboxydimethylmethyloxyphenyl]prop-2-en-1-one,
1-[2-methyloxy-4-chlorophenyl]-3-[3,5-dimethyl-4-tertiobutyloxycarbonyldimethylmethyloxyphenyl]prop-2-en-1-one,
1-[2-methyloxy-4-chlorophenyl]-3-[3,5-dimethyl-4-carboxydimethylmethyloxyphenyl]prop-2-en-1-one,
1-[4-heptylphenyl]-3-[3,5-dimethyl-4-tertiobutyloxycarbonyldimethylmethyloxyphenyl]prop-2-en-1-one,
1-[4-heptylphenyl]-3-[3,5-dimethyl-4-carboxydimethylmethyloxyphenyl]prop-2-en-1-one,
1-[4-bromophenyl]-3-[3,5-dimethyl-4-tertiobutyloxycarbonyldimethylmethyloxyphenyl]prop-2-en-1-one,
1-[4-bromophenyl]-3-[3,5-dimethyl-4-carboxydimethylmethyloxyphenyl]prop-2-en-1-one.

24. Composition according to any one of the previous claims, **characterized in that** the derivative is selected in the group consisting of
1-[4-methylthiophenyl]-3-[3,5-dimethyl-4-carboxydimethylmethyloxyphenyl]prop-2-en-1-one,
1-[4-hexyloxyphenyl]-3-[3,5-dimethyl-4-carboxydimethylmethyloxyphenyl]prop-2-en-1-one, et
1-[4-bromophenyl]-3-[3,5-dimethyl-4-carboxydimethylmethyloxyphenyl]prop-2-en-1-one.

25. Composition according to any one of the previous claims, **characterized in that** the derivative is 1-[4-methylthiophenyl]-3-[3,5-dimethyl-4-carboxydimethylmethyloxyphenyl]prop-2-en-1-one.

26. Composition according to any one of claims 1 to 25, **characterized in that** the pathology related to inflammation is selected in the group consisting of atherosclerosis, allergy, asthma, eczema, psoriasis and pruritus.

27. Composition according to any one of claims 1 to 25, **characterized in that** the pathology related to neurodegeneration is Alzheimer's disease or Parkinson's disease.

28. Composition according to any one of claims 1 to 25, **characterized in that** the pathology related to deregulations of lipid and/or glucose metabolism is selected in the group consisting of diabetes, atherosclerosis and obesity.

29. Composition according to any one of claims 1 to 25, **characterized in that** the pathology related to cell proliferation and/or differentiation is selected in the group consisting of carcinogenesis, psoriasis and atherosclerosis.

## Patentansprüche

1. Zusammensetzung zur Behandlung oder Prophylaxe einer Erkrankung, verbunden mit Entzündung, mit Neurodegeneration, mit Lipid- und/oder Glucose-Stoffwechselstörungen, mit Zellwachstum und/oder -teilung und/oder mit der Hautalterung oder des Zentralnervensystems, umfassend, in einem pharmazeutisch verträglichen Träger, mindestens ein substituiertes 1,3-Diphenylprop-2-en-1-on-Derivat der folgenden Formel (I): 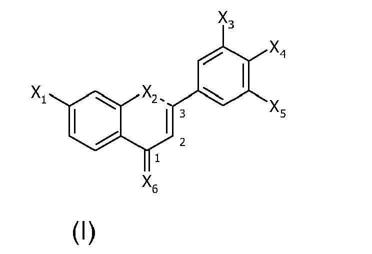 in der:
X1 ein Halogenatom oder einen Rest -R1 oder einen Rest der folgenden Formel:
-G1-R1 bedeutet;
X2 ein Wasserstoffatom oder einen Thionitrosorest oder einen Hydroxyrest oder einen Alkylcarbonyloxyrest oder einen unsubstituierten Alkyloxyrest oder einen Thiolrest oder einen Alkylthiorest oder einen Alkylcarbonylthiorest bedeutet, X2 auch ein Sauerstoffatom oder Schwefelatom bedeuten kann, das an das Kohlenstoffatom 3 der Propenkette gebunden ist, um ein Derivat vom Typ 2-Phenyl-4H-1-benzopyran-4-on oder vom Typ 2-Phenyl-4H-1-benzothiopyran-4-on zu bilden;
X3 einen Rest -R3 oder einen Rest der folgenden Formel: -G3-R3 bedeutet;
X4 ein Halogenatom oder einen Thionitrosorest oder einen Rest -R4 oder einen Rest der folgenden Formel: -G4-R4 bedeutet;
X5 einen Rest -R5 oder einen Rest der folgenden Formel: -G5-R5 bedeutet;
X6 ein Sauerstoffatom ist;
R1, R3, R4, R5, gleich oder voneinander verschieden, ein Wasserstoffatom oder einen Akylrest bedeuten, der mit einem Rest aus der nachstehend definierten Gruppe 1 oder Gruppe 2 substituiert ist oder nicht;
G1, G3, G4, G5, gleich oder voneinander verschieden, ein Sauerstoffatom oder Schwefelatom bedeuten;
wobei mindestens einer der Reste X1, X3, X4 oder X5 die Formel-G-R aufweist, in der G ein Schwefelatom bedeutet, und
wobei mindestens einer der Reste R1, R3, R4 oder R5 in Form eines Alkylrests vorliegt, der mindestens einen Substituenten der Gruppe 1 oder 2 trägt, wobei der Alkylrest direkt an den Ring gebunden ist oder mit einem Rest G gemäß der Formel -G-R assoziiert ist;
wobei die Substituenten der Gruppe 1 ausgewählt sind aus Carboxyresten der Formel: -COOR₆ und Carbamoylresten der Formel: -CONR₆R₇;
wobei die Substituenten der Gruppe 2 ausgewählt sind aus Sulfonsäure (SO₃H) und Sulfonamidresten der Formel: -SO₂NR₆R₇;
wobei R₆ und R₇, gleich oder voneinander verschieden, ein Wasserstoffatom oder einen Alkylrest bedeuten, der gegebenenfalls mit mindestens einem Rest vom Typ 1 oder 2 substituiert ist;
ausgenommen Verbindungen der Formel (I), in denen:
X₂ ein Wasserstoffatom bedeutet und X₁ -G1-R1 bedeutet, wobei G1 ein Sauerstoffatom bedeutet und R1 CH2COOH bedeutet;
deren optische und geometrische Isomere, deren Racemate, deren Tautomere, deren Salze, deren Hydrate und deren Gemische.

2. Zusammensetzung zur Behandlung oder Prophylaxe einer Erkrankung, verbunden mit Entzündung, mit Neurodegeneration, Lipid- und/oder Glucose-Stoffwechselstörungen, mit Zellwachstum und/oder -teilung und/oder mit Hautalterung oder des Zentralnervensystems, umfassend, in einem pharmazeutisch verträglichen Träger, mindestens ein substituiertes 1,3-Diphenylprop-2-en-1-on-Derivat der folgenden Formel (I): 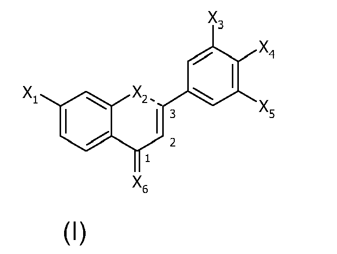 in der:
X1 ein Halogenatom oder einen Rest -R1 oder einen Rest der folgenden Formel: -G1-R1 bedeutet;
X2 ein Wasserstoffatom oder einen Thionitrosorest oder einen Hydroxyrest oder einen Alkylcarbonyloxyrest oder einen unsubstituierten Alkyloxyrest oder einen Thiolrest oder einen Alkylthiorest oder einen Alkylcarbonylthiorest bedeutet; X2 auch ein Schwefelatom bedeuten kann, das an das Kohlenstoffatom 3 der Propenkette gebunden ist, um ein Derivat vom Typ 2-Phenyl-4H-1-benzothiopyran-4-on zu bilden;
X3 einen Rest -R3 oder einen Rest der folgenden Formel: -G3-R3 bedeutet;
X4 ein Halogenatom oder einen Thionitrosorest oder einen Rest -R4 oder einen Rest der folgenden Formel: -G4-R4 bedeutet;
X5 einen Rest -R5 oder einen Rest der folgenden Formel: -G5-R5 bedeutet;
X6 ein Sauerstoffatom ist;
R1, R3, R4, R5, gleich oder voneinander verschieden, ein Wasserstoffatom oder einen Alkylrest bedeuten, der mit einem Rest aus der nachstehend definierten Gruppe 1 oder Gruppe 2 substituiert ist oder nicht;
G1, G3, G4, G5, gleich oder voneinander verschieden, ein Sauerstoffatom oder Schwefelatom bedeuten;
wobei mindestens einer der Reste X1, X3, X4 oder X5 die Formel -G-R aufweist;
wobei keiner der Reste X3, X4 oder X5 ein Wasserstoffatom bedeutet; und
wobei mindestens einer der Reste R1, R3, R4 oder R5 in Form eines Alkylrests vorliegt, der mindestens einen Substituenten der Gruppe 1 oder 2 trägt, wobei der Alkylrest direkt an den Ring gebunden ist oder mit einem Rest G gemäß der Formel -G-R assoziiert ist;
die Substituenten der Gruppe 1 aus Carboxyresten der Formel: -COOR₆ und Carbamoylresten der Formel: -CONR₆R₇ ausgewählt sind;
die Substituenten der Gruppe 2 aus Sulfonsäure (SO₃H) und Sulfonamidresten der Formel: -SO₂NR₆R₇ ausgewählt sind;
wobei R₆ und R₇, gleich oder voneinander verschieden, ein Wasserstoffatom oder einen Alkylrest bedeuten, der gegebenenfalls mit mindestens einem Rest vom Typ 1 oder 2 substituiert ist;
ausgenommen Verbindungen der Formel (I), in denen:
X₂ ein Wasserstoffatom bedeutet und X₁ -G1-R1 bedeutet, wobei G1 ein Sauerstoffatom bedeutet und R1 CH2COOH bedeutet;
deren optische und geometrische Isomere, deren Racemate, deren Tautomere, deren Salze, deren Hydrate und deren Gemische.

3. Zusammensetzung nach Anspruch 1 oder 2, **dadurch gekennzeichnet, dass** die Derivate in cis-Konformation, trans-Konformation oder einer Mischung daraus vorliegen können.

4. Zusammensetzung nach Anspruch 1, **dadurch gekennzeichnet, dass** keiner der Reste X3, X4 und X5 ein Wasserstoffatom bedeutet.

5. Zusammensetzung nach Anspruch 1, **dadurch gekennzeichnet, dass** einer oder zwei der Reste X3, X4 und X5 ein Wasserstoffatom bedeuten.

6. Zusammensetzung nach einem der vorhergehenden Ansprüche, **dadurch gekennzeichnet, dass** die beiden Reste G1 und G4 ein Schwefelatom bedeuten.

7. Zusammensetzung nach einem der vorhergehenden Ansprüche, **dadurch gekennzeichnet, dass** X2 ein Wasserstoffatom, ein Thionitrosorest oder ein Hydroxyrest oder ein Alkyloxyrest oder ein Thiolrest oder ein Alkylthiorest ist.

8. Zusammensetzung nach einem der vorhergehenden Ansprüche, **dadurch gekennzeichnet, dass** X4 einen Thionitrosorest oder einen Rest -R4 oder einen Rest der Formel -G4-R4 bedeutet und X2 ein Thionitrosorest oder ein Hydroxy-, Alkyloxy-, Thiol- oder Alkylthiorest ist, wobei G4 und R4 wie in Anspruch 1 oder 2 definiert sind.

9. Zusammensetzung nach einem der vorhergehenden Ansprüche, **dadurch gekennzeichnet, dass** X1 einen Rest -R1 oder einen Rest der Formel -G1-R1 bedeutet, wobei R1 ein Alkylrest ist, der mit einem Rest aus der Gruppe 1 substituiert ist, wobei G1 und der Substituent der Gruppe 1 wie in Anspruch 1 oder 2 definiert sind.

10. Zusammensetzung nach einem der vorhergehenden Ansprüche, **dadurch gekennzeichnet, dass** X1 ein Rest -G1-R1 ist, wobei G1 und R1 wie in Anspruch 1 oder 2 definiert sind.

11. Zusammensetzung nach einem der vorhergehenden Ansprüche, **dadurch gekennzeichnet, dass** X1 ein Rest -G1-R1 ist, wobei G1 ein Sauerstoffatom ist, wobei R1 wie in Anspruch 1 oder 2 definiert ist.

12. Zusammensetzung nach einem der vorhergehenden Ansprüche, **dadurch gekennzeichnet, dass** X1 einen Rest -R1 oder einen Rest der Formel -G1-R1 bedeutet, wobei R1 ein Alkylrest ist, der mit einem Rest aus der Gruppe 2 substituiert ist, wobei G1 und der Substituent der Gruppe 2 wie in Anspruch 1 oder 2 definiert sind.

13. Zusammensetzung nach einem der vorhergehenden Ansprüche, **dadurch gekennzeichnet, dass** X3 einen Rest -R3 oder einen Rest der Formel -G3-R3 bedeutet, wobei R3 ein Alkylrest ist, der mit einem Rest aus der Gruppe 1 substituiert ist, wobei G3 und der Substituent der Gruppe 1 wie in Anspruch 1 oder 2 definiert sind.

14. Zusammensetzung nach einem der vorhergehenden Ansprüche, **dadurch gekennzeichnet, dass** X3 einen Rest -R3 oder einen Rest der Formel -G3-R3 bedeutet, wobei R3 ein Alkylrest ist, der mit einem Rest aus der Gruppe 2 substituiert ist, wobei G3 und der Substituent der Gruppe 2 wie in Anspruch 1 oder 2 definiert sind.

15. Zusammensetzung nach einem der vorhergehenden Ansprüche, **dadurch gekennzeichnet, dass** X4 einen Rest -R4 oder einen Rest der Formel -G4-R4 bedeutet, wobei R4 ein Alkylrest ist, der mit einem Rest aus der Gruppe 1 substituiert ist, wobei G4 und der Substituent der Gruppe 1 wie in Anspruch 1 oder 2 definiert sind.

16. Zusammensetzung nach einem der vorhergehenden Ansprüche, **dadurch gekennzeichnet, dass** X4 ein Rest -G4-R4 ist, wobei G4 und R4 wie in Anspruch 1 oder 2 definiert sind.

17. Zusammensetzung nach einem der vorhergehenden Ansprüche, **dadurch gekennzeichnet, dass** X4 ein Rest -G4-R4 ist, wobei G4 ein Sauerstoffatom ist, wobei R4 wie in Anspruch 1 oder 2 definiert ist.

18. Zusammensetzung nach einem der vorhergehenden Ansprüche, **dadurch gekennzeichnet, dass** X4 ein Rest -G4-R4 ist, wobei G4 ein Sauerstoffatom ist und X3 oder X5 jeweils R3 oder -G3-R3 einerseits bzw. R5 oder -G5-R5 andererseits bedeuten, wobei R3 oder R5 Alkylreste sind, die einen Substituenten der Gruppe 1 tragen, wobei R4, G3, G5 und der Substituent der Gruppe 1 wie in Anspruch 1 oder 2 definiert sind.

19. Zusammensetzung nach einem der vorhergehenden Ansprüche, **dadurch gekennzeichnet, dass** X4 einen Rest -R4 oder einen Rest der Formel -G4-R4 bedeutet, wobei R4 ein Alkylrest ist, der mit einem Rest aus der Gruppe 2 substituiert ist, wobei G4 und der Substituent der Gruppe 2 wie in Anspruch 1 oder 2 definiert sind.

20. Zusammensetzung nach einem der vorhergehenden Ansprüche, **dadurch gekennzeichnet, dass** X1 ein Halogenatom bedeutet.

21. Zusammensetzung nach einem der vorhergehenden Ansprüche, **dadurch gekennzeichnet, dass** X1, X3, X4 oder X5 OC(CH3)2COOR6 bedeuten, wobei R6 wie in Anspruch 1 oder 2 definiert ist.

22. Zusammensetzung nach einem der vorhergehenden Ansprüche, **dadurch gekennzeichnet, dass** X1, X3, X4 oder X5 SC(CH3)2COOR6 bedeuten, wobei R6 wie in Anspruch 1 oder 2 definiert ist.

23. Zusammensetzung nach einem der vorhergehenden Ansprüche, **dadurch gekennzeichnet, dass** das Derivat ausgewählt ist aus:
1-[2-Hydroxy-4-carboxydimethylmethyloxyphenyl]-3-[3,5-di*tert*butyl-4-hydroxyphenyl]-prop-2-en-1-on,
1-[2-Hydroxy-4-ethoxycarbonyldimethylmethyloxyphenyl]-3-[3,5-di*tert*butyl-4-hydroxyphenyl]prop-2-en-1-on,
1-[2-Hydroxy-4-isopropyloxycarbonyldimethylmethyloxyphenyl]-3-[3,5-di*tert*butyl-4-hydroxyphenyl]prop-2-en-1-on,
1-[2-Hydroxyphenyl]-3-[3-carboxydimethylmethyloxy-4-hydroxy-5*-tert*butylphenyl]-prop-2-en-1-on,
1-[2-Hydroxyphenyl]-3-[3-*iso*propyloxycarbonyldimethylmethyloxy-4-hydroxy-5-*tert*butylphenyl]prop-2-en-1-on,
1-[2-Hydroxy-4-chlorphenyl]-3-[3-carboxydimethylmethyloxy-4-hydroxy-5-*tert*butylphenyl]prop-2-en-1-on,
1-[2-Hydroxy-4-chlorphenyl]-3-[3-*iso*propyloxycarbonyldimethylmethyloxy-4-hydroxy-5-*tert*butylphenyl]prop-2-en-1-on,
1-[2-Hydroxyphenyl]-3-[3-carboxydimethylmethyl-4-hydroxy-5-*tert*butylphenyl]prop-2-en-1-on,
1-[2-Hydroxyphenyl]-3-[3-*iso*propyloxycarbonyldimethylmethyl-4-hydroxy-5-*tert*butylphenyl]prop-2-en-1-on,
1-[2-Hydroxy-4-chlorphenyl]-3-[3-carboxydimethylmethyl-4-hydroxy-5-*tert*butylphenyl]prop-2-en-1-on,
1-[2-Hydroxy-4-chlorphenyl]-3-[3-*iso*propyloxycarbonyldimethylmethyl-4-hydroxy-5-*tert*butylphenyl]prop-2-en-1-on,
1-[2-Hydroxy-4-chlorphenyl]-3-[3,5-dimethoxy-4-carboxydimethylmethyloxyphenyl]-prop-2-en-1-on,
1-[2-Hydroxy-4-chlorphenyl]-3-[3,5-dimethoxy-4-*iso*propyloxycarbonyldimethylmethyloxyphenyl]prop-2-en-1-on,
1-[2-Hydroxyphenyl]-3-[3,5-dimethoxy-4-carboxydimethylmethyloxyphenyl]prop-2-en-1-on,
1-[2-Hydroxyphenyl]-3-[3,5-dimethoxy-4-*iso*propyloxycarbonyldimethylmethyloxyphenyl]prop-2-en-1-on,
1-[2-Hydroxy-4-carboxydimethylmethyloxyphenyl]-3-[3,5-dimethoxy-4-hydroxyphenyl]prop-2-en-1-on,
1-[2-Hydroxy-4-*iso*propyloxycarbonyldimethylmethyloxyphenyl]-3-[3,5-dimethoxy-4-hydroxyphenyl]prop-2-en-1-on,
1-[2-Hydroxy-4-chlorphenyl]-3-[3,4-dihydroxy-5-carboxydimethylmethyloxyphenyl]-prop-2-en-1-on,
1-[2-Hydroxy-4-chlorphenyl]-3-[3,4-dihydroxy-5-*iso*propyloxycarbonyldimethylmethyloxyphenyl]prop-2-en-1-on,
1-[2-Hydroxy-4-carboxydimethylmethyloxyphenyl]-3-[3,5-dimethyl-4-hydroxyphenyl]-prop-2-en-1-on,
1-[2-Hydroxy-4-*iso*propyloxycarbonyldimethylmethyloxyphenyl]-3-[3,5-dimethyl-4-hydroxyphenyl]prop-2-en-1-on,
1-[2-Hydroxy-4-chlorphenyl]-3-[3,5-dimethyl-4-carboxydimethylmethyloxyphenyl]prop-2-en-1-on,
1-[2-Hydroxy-4-chlorphenyl]-3-[3,5-dimethyl-4-*iso*propyloxycarbonyldimethylmethyloxyphenyl]prop-2-en-1-on,
1-[2-Hydroxyphenyl]-3-[3,5-dimethyl-4-carboxydimethylmethyloxyphenyl]prop-2-en-1-on,
1-[2-Hydroxyphenyl]-3-[3,5-dimethyl-4-*iso*propyloxycarbonyldimethylmethyloxyphenyl]prop-2-en-1-on,
1-[2-Hydroxyphenyl]-3-[4-carboxydimethylmethylthiophenyl]prop-2-en-1-on,
1-[2-Hydroxyphenyl]-3-[4-*iso*propyloxycarbonyldimethylmethylthiophenyl]prop-2-en-1-on,
1-[2-Hydroxy-4-carboxydimethylmethyloxyphenyl]-3-[4-methylthiophenyl]prop-2-en-1-on,
1-[4-Chlorphenyl]-3-[3,5-dimethyl-4-tert-butyloxycarbonyldimethylmethyloxyphenyl]-prop-2-en-1-on,
1-[4-Chlorphenyl]-3-[3,5-dimethyl-4-isopropyloxycarbonyldimethylmethyloxyphenyl]-prop-2-en-1-on,
1-[4-Chlorphenyl]-3-[3,5-dimethyl-4-carboxydimethylmethyloxyphenyl]prop-2-en-1-on,
1-[4-Chlor-2-hydroxyphenyl]-3-[4-carboxydimethylmethylthiophenyl]prop-2-en-1-on,
1-[4-Carboxydimethylmethyloxyphenyl]-3-[3,5-dimethyl-4-hydroxyphenyl]prop-2-en-1-on,
1-[4-Methylthiophenyl]-3-[4-carboxydimethylmethyloxyphenyl]prop-2-en-1-on,
1-[4-Carboxydimethylmethylthiophenyl]-3-[4-methylthiophenyl]prop-2-en-1-on,
1-[2-Hydroxy-4-bromphenyl]-3-[3,5-dimethyl-4-carboxydimethylmethyloxyphenyl]-prop-2-en-1-on,
1-[4-Carboxydimethylmethyloxyphenyl]-3-[4-methylthiophenyl]prop-2-en-1-on,
1-[4-Methylthiophenyl]-3-[3,5-dimethyl-4-tert-butyloxycarbonyldimethylmethyloxyphenyl]prop-2-en-1-on,
1-[4-Methylthiophenyl]-3-[3,5-dimethyl-4-isopropyloxycarbonyldimethylmethyloxyphenyl]prop-2-en-1-on,
1- [4-Methylthiophenyl] -3 - [3,5 -dimethyl-4-carboxydimethylmethyloxyphenyl]prop-2-en-1 - on,
1-[2-Methoxyphenyl]-3-[3,5-dimethyl-4-tert-butyloxycarbonyldimethylmethyloxyphenyl]prop-2-en-1-on,
1-[2-Methoxyphenyl] -3 - [3,5 -dimethyl-4-carboxydimethylmethyloxyphenyl]prop-2-en-1 - on,
1-[4-Hexyloxyphenyl]-3-[3,5-dimethyl-4-tert-butyloxycarbonyldimethylmethyloxyphenyl]prop-2-en-1-on,
1-[2-Methyloxy-4-chlorphenyl]-3-[3,5-dimethyl-4-tert-butyloxycarbonyldimethylmethyloxyphenyl]prop-2-en-1-on,
1-[4-Hexyloxyphenyl]-3-[3,5 -dimethyl-4-carboxydimethylmethyloxyphenyl]prop-2-en-1-on,
1-[2-Methyloxy-4-chlorphenyl]-3-[3,5-dimethyl-4-carboxydimethylmethyloxyphenyl]-prop-2-en-1-on,
1-[4-Heptylphenyl]-3-[3,5-dimethyl-4-tert-butyloxycarbonyldimethylmethyloxyphenyl]-prop-2-en-1-on,
1-[4-Heptylphenyl] -3 - [3,5 -dimethyl-4-carboxydimethylmethyloxyphenyl]prop-2-en-1-on,
1-[4-Bromphenyl]-3-[3,5-dimethyl-4-tert-butyloxycarbonyldimethylmethyloxyphenyl]-prop-2-en-1-on,
1-[4-Bromphenyl] -3 - [3,5 -dimethyl-4-carboxydimethylmethyloxyphenyl]prop-2-en-1-on.

24. Zusammensetzung nach einem der vorhergehenden Ansprüche, **dadurch gekennzeichnet, dass** das Derivat ausgewählt ist aus:
1-[4-Methylthiophenyl]-3-[3,5-dimethyl-4-carboxydimethylmethyloxyphenyl]prop-2-en-1-on,
1- [4-Hexyloxyphenyl] -3 - [3,5 -dimethyl-4-carboxydimethylmethyloxyphenyl]prop-2-en-1 - on und
1-[4-Bromphenyl]-3-[3,5-dimethyl-4-carboxydimethylmethyloxyphenyl]prop-2-en- 1 -on.

25. Zusammensetzung nach einem der vorhergehenden Ansprüche, **dadurch gekennzeichnet, dass** das Derivat 1-[4-Methylthiophenyl]-3-[3,5-dimethyl-4-carboxydimethylmethyloxyphenyl]prop-2-en-1-on ist.

26. Zusammensetzung nach einem der Ansprüche 1 bis 25, **dadurch gekennzeichnet, dass** die mit einer Entzündung verbundene Erkrankung aus Atherosklerose, einer Allergie, Asthma, Ekzem, Schuppenflechte und Juckreiz ausgewählt ist.

27. Zusammensetzung nach einem der Ansprüche 1 bis 25, **dadurch gekennzeichnet, dass** die mit Neurodegeneration verbundene Erkrankung Alzheimer oder Parkinson ist.

28. Zusammensetzung nach einem der Ansprüche 1 bis 25, **dadurch gekennzeichnet, dass** die mit Lipid- und/oder Glucose-Stoffwechselstörungen verbundene Erkrankung aus Diabetes, Atherosklerose und Fettsucht ausgewählt ist.

29. Zusammensetzung nach einem der Ansprüche 1 bis 25, **dadurch gekennzeichnet, dass** die mit dem Zellwachstum und/oder der Zellteilung verbundene Erkrankung aus Karzinogenese, Schuppenflechte und Atherosklerose ausgewählt ist.
